# EUROPEAN PATENT SPECIFICATION

(11) **EP 2 081 893 B1**
(45) Date of publication and mention of the grant of the patent: **23.03.2011**
(21) Application number: 07844472.6
(22) Date of filing: 19.10.2007
(51) Int. Cl.: C07D 209/14, C07D 209/16, C07D 403/12

(54) **SUBSTITUTED INDOLES**
SUBSTITUIERTE INDOLE
INDOLES SUBSTITUÉS

(30) Priority: 19.10.2006 US 853243 P
(43) Date of publication of application: 29.07.2009
(73) Proprietor: Auspex Pharmaceuticals, Inc., Vista, CA 92081-8356 (US)
(72) Inventor: GANT, Thomas G., Vista, CA 92081-8356 (US); SARSHAR, Sepehr, Vista, CA 92081-8356 (US)
(74) Representative: Smaggasgale, Gillian Helen
(86) International application number: PCT/US2007/081977
(87) International publication number: WO 2008/049116

(56) References cited:
- DE-A1- 3 320 521
- DE-A1-102005 008 312
- US-B1- 6 376 531
- IAN FELLOWS, RICHARD M. CARR, NIGEL DE BOECK, STEPHEN MONTGOMERY, IAN WATERHOUSE, DEREK R. SUTHERLAND: "Simple methods for the labelling of N-methyl amines using isotopically labelled methyl iodide" JOURNAL OF LABELLED COMPOUNDS AND RADIOPHARMACEUTICALS, vol. 41, no. 12, 1998, pages 1127-1143, XP002474309

## Description

This application claims the benefit of priority of United States provisional application No. 60/853,243, filed October 19, 2006.

### FIELD

Disclosed herein are new substituted indoles, processes of preparation, and pharmaceutical compositions thereof. Also disclosed are methods of their use for treating, preventing, or ameliorating headaches, movement disorders, depression, and anxiety and/or any other disease, disorder, or condition ameliorated by modulating 5-hydroxytryptamine 1B and/or 1D receptors.

### BACKGROUND

Sumatriptan (Imitrex®, disclosed in U.S. Patents No. 4,816,470 (issued Mar. 28, 1989), 5,037,845 (issued Aug. 6, 1991), 5,863,559 (issued Jan. 26, 1999), 6,020,001 (issued Feb. 1, 2000), and 6,368,627 (issued Apr. 9, 2002)) is purported to alleviate the acute symptoms and causes of migraine headaches through agonism of the 5-hydroxytryptaininel B (5-HT_{1B}) and 5-hydroxytryptamine 1D (5-HT_{1D}) receptors. This receptor modulation causes a temporary vasoconstriction in the central nervous system, particularly the vasculature of the dura mater and basilar artery. This general class of therapeutics is usually referred to as the "triptans" and includes zolmitriptan, rizatriptan, naratriptan, frovatriptan, almotriptan (disclosed in U.S. Patents No. 5,565,447 (issued Oct. 15, 1996)), avitriptan (phase III), and eletriptan, as well as the first-in-class sumatriptan.

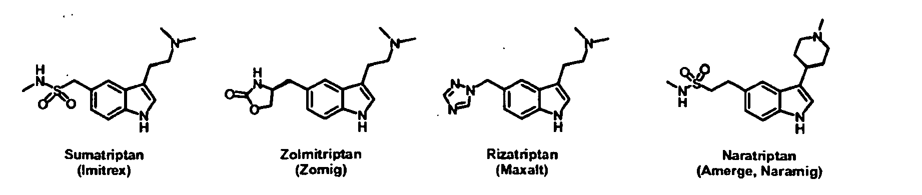

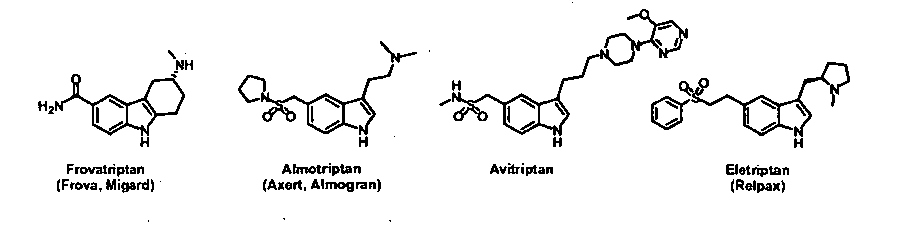

The benefits and shortcomings of this drug have been extensively reviewed. Some of these shortcomings may be traced to metabolism-related phenomena. Sumatriptan is converted in vivo by oxidative and conjugative degradation to multiple metabolites. Oxidative metabolism of the N,N-dimethylaminoethyl group leads to the primary metabolite, the substituted indoleacetic acid, which is inactive. This oxidative transformation (or an analogous variant) is common in this class. Moreover because sumatriptan is metabolize primarily by monoamine oxidase A (MAOA), an enzyme responsible for transformations of endogenous neurotransmitters as well as several centrally acting drugs, polyphannacy is necessarily complex and has potential for adverse events. This phenomenon increases inter-patient variability in response to polypharmacy. Other members of this class are metabolized by MAOs and P450s (CYPs) in a manner that could also be manipulated to therapeutic benefit by the approach disclosed herein. Some of the specific drugs and their biological oxidizing agents are: almotriptan (MAOA, CYP3A4, CYP2D6), eletriptan (CYP3A4), frovatriptan (CYP1A2), naratriptan (as-yet-unidentified CYPs, MAOA), rizatriptan (MAOA), and zolmitriptan (CYP1A2, MAOA).

Aspects of the present invention are not limited to specific synthetic methods, specific pharmaceutical carriers, or to particular phannaceutical formulations or administration regimens, as such may, of course, vary. The terminology used herein is for the purpose of describing particular embodiments only and is not intended to be limiting.

In certain embodiments of the present invention, compounds have structural Formula I 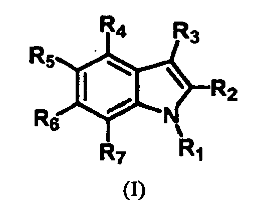 or a pharmaceutically acceptable salt or solvate thereof, wherein: R₃ is selected from the group consisting of: 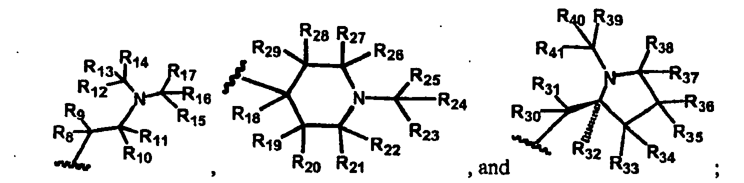 R₅ is selected from the group consisting of: 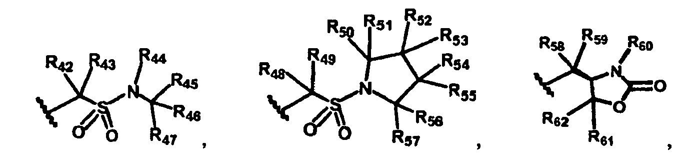 and 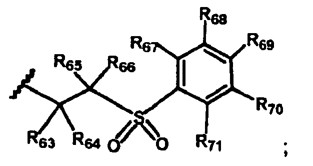 R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R_{40,} R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₁, and R₇, are independently selected from the group consisting of hydrogen, and deuterium;
provided that at least one of R₁, R₂, R₄, R₆, R₇, R₈, R₉, R₁₀, R₁₁, R₁₂, R₁₃, R₁₄, R₁₅, R₁₆, R₁₇, R₁₈, R₁₉, R_{20,} R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R_{43,} R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, and R₇₁ is deuterium; and
with the proviso that compounds having structural Fonnula I cannot be:

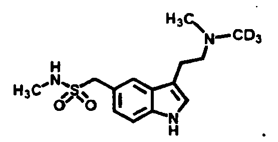

Certain compounds disclosed herein may possess useful 5-HT_{1B} and/or 5-HT_{1D} receptor modulating activity, and may be used in the treatment or prophylaxis of a disease or condition in which 5-HT_{1B} and/or 5-HT_{1D} receptors plays an active role. Thus, in broad aspect, the certain embodiments also provide pharmaceutical compositions comprising one or more compounds disclosed herein together with a pharmaceutically acceptable carrier, as well as methods of making and using the compounds and compositions. Certain embodiments provide methods for modulating 5-HT_{1B} and/or 5-HT_{1D} receptors. Other embodiments provide methods for treating a 5-HT_{1B} and/or 5-HT_{1D} receptor-mediated disorder in a patient in need of such treatment, comprising administering to said patient a therapeutically effective amount of a compound or composition according to the present invention. Also provided is the use of certain compounds disclosed herein for use in the manufacture of a medicament for the treatment of a disease or condition ameliorated by the modulation of 5-HT_{1B} and/or 5-HT_{1D} receptors.

In further embodiments, a compound having structural Formula I is substantially a single enantiomer, a mixture of about 90% or more by weight of the (-)-enantiomer and about 10% or less by weight of the (+)-enantiomer, a mixture of about 90% or more by weight of the (+)-enantiomer and about 10% or less by weight of the (-)-enantiomer, substantially an individual diastereomer, a mixture of about 90% or more by weight of an individual diastereomer and about 10% or less by weight of any other diastereomer, or a mixture of about 90% or more by weight of an individual diastereomer and about 10% or less by weight of any other diastereomer.

In yet further embodiments, R₃ is 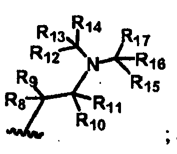 and R₅ is selected from the group consisting of:

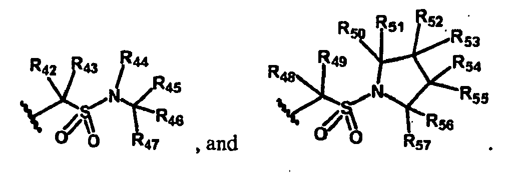

In certain embodiments, compounds of the present invention have structural Formula II 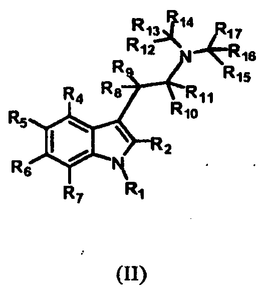 or a pharmaceutically acceptable salt or solvate thereof, wherein: R₅ is selected from the group consisting of: 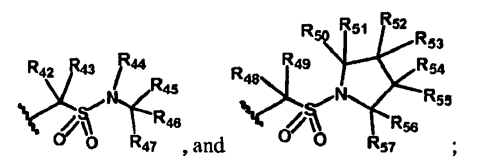 R₁, R₂, R₄, R₆, R₇, R₈, R₉, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, and R₅₇ are independently selected from the group consisting of hydrogen, and deuterium; and
R₁₀, R₁₁, R₁₂, R₁₃, R₁₄, R₁₅, R₁₆, and R₁₇ are each deuterium, R₁₀ and R₁₁ are each hydrogen and R₁₂, R₁₃, R₁₄, R₁₅, R₁₆, and R₁₇ are each deuterium, or R₁₀ and R₁₁ are each deuterium and R₁₂, R₁₃, R₁₄, R₁₅, R₁₆, and R₁₇ are each hydrogen.

In further embodiments a compound having structural Formula II is substantially a single enantiomer, a mixture of about 90% or more by weight of the (-)-enantiomer and about 10% or less by weight of the (+)-enantiomer, a mixture of about 90% or more by weight of the (+)-enantiomer and about 10% or less by weight of the (-)-enantiomer, substantially an individual diastereomer, a mixture of about 90% or more by weight of an individual diastereomer and about 10% or less by weight of any other diastereomer, or a mixture of about 90% or more by weight of an individual diastereomer and about 10% or less by weight of any other diastereomer.

In further embodiments R₁₀, R₁₁, R₁₂, R₁₃, R₁₄, R₁₅, R₁₆, and R₁₇ each have deuterium enrichment of at least 50%, R₁₀ and R₁₁ are each hydrogen and R₁₂, R₁₃, R₁₄, . R_{15,} R₁₆, and R₁₇ each have deuterium enrichment of at least 50%, or R₁₀ and R₁₁ each have deuterium enrichment of at least 50% and R₁₂, R₁₃, R₁₄, R₁₅, R₁₆, and R₁₇ are each hydrogen.

In yet further embodiments R₁, R₂, R₄, R₆, R₇, R₄₂, R₄₃, R₄₄, R₄₅, R₄₆, R₄₇, R₄₈, R₄₉, R₅₀, R₅₁, R₅₂, R_{53,} R₅₄, R₅₅, R₅₆, and R₅₇ are hydrogen.

In yet further embodiments R₁₀, R₁₁, R₁₂, R₁₃, R₁₄, R₁₅, R₁₆, and R₁₇ each have deuterium enrichment of at least 50%, R₁₀ and R₁₁ are each hydrogen and R₁₂, R₁₃, R₁₄, R₁₅, R₁₆, and R₁₇ each have deuterium enrichment of at least 50%, or R₁₀ and R₁₁ each have deuterium enrichment of at least 50% and R₁₂, R₁₃, R₁₄, R₁₅, R₁₆, and R₁₇ are each hydrogen.

In certain embodiments of the present invention, compounds have a structural formula selected from the group consisting of: 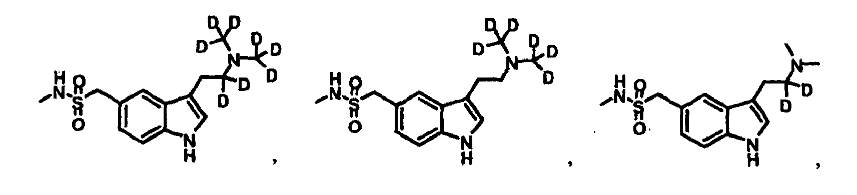 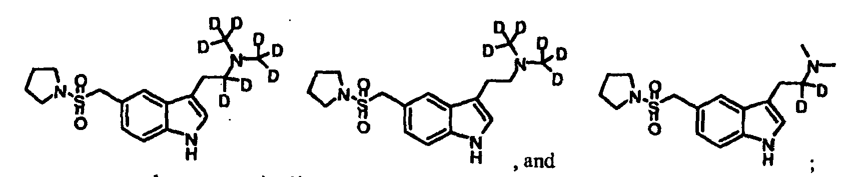 or a pharmaceutically acceptable salt or solvate thereof, wherein:

In further embodiments a compound of the present invention is substantially a single enantiomer, a mixture of about 90% or more by weight of the (-)-enantiomer and about 10% or less by weight of the (+)-enantiomer, a mixture of about 90% or more by weight of the (+)-enantiomer and about 10% or less by weight of the (-)-enantiomer, substantially an individual diastereomer, a mixture of about 90% or more by weight of an individual diastereomer and about 10% or less by weight of any other diastereomer, or a mixture of about 90% or more by weight of an individual diastereomer and about 10% or less by weight of any other diastereomer.

In further embodiments each of said deuteriums have deuterium enrichment of at least 50%.

In certain embodiments of the present invention compounds have structural Formula III 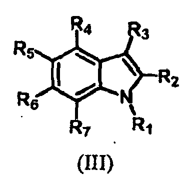 or a pharmaceutically acceptable salt or solvate thereof, wherein: R₃ is selected from the group consisting of: 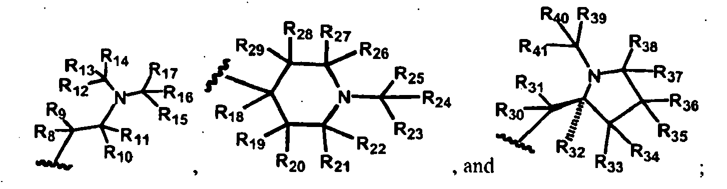 R₅ is selected from the group consisting of: 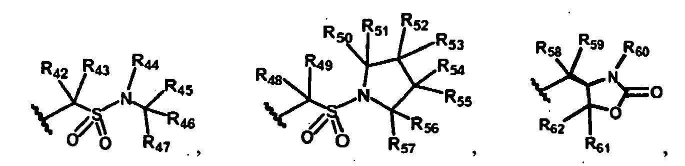 and 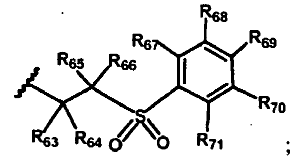 R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R_{22,} R₂₃, R₂₄, R₂₅, R₂₆, R₂₇, R₂₈, R₂₉, R₃₀, R₃₁, R₃₂, R₃₃, R₃₄, R₃₃, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R_{69,} R₇₀, and R₇₁ are independently selected from the group consisting of hydrogen, and deuterium; and
provided that at least one of R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R_{22,} R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, _{R40}, R₄₁, R₄₂, R₄₃, R_{44,} R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R_{68,} R₆₉, R₇₀, and R₇₁ is deuterium.

In certain embodiments of the present invention a pharmaceutical compositions is comprised of one or more compounds disclosed herein together with a pharmaceutically acceptable carrier

In further embodiments the pharmaceutical composition is suitable for oral, parenteral, intranasal, or intravenous infusion administration.

In yet further embodiments the pharmaceutical composition comprises a tablet or a capsule.

In yet further embodiments the compounds as disclosed herein are administered in a dose of 0.5 to 300 milligrams.

In certain embodiments of the present invention the compounds are for use in a method of treating a subject suffering from a disorder selected from the group consisting of headaches, movement disorders, depression, and anxiety

In further embodiments said disorder is a migraine headache with aura.

In yet further embodiments said disorder is a migraine headache without aura.

In yet further embodiments said compound has at least one of the following properties:
a) decreased inter-individual variation in plasma levels of said compound or a metabolite thereof as compared to the non-isotopically enriched compound;
b) increased average plasma levels of said compound per dosage unit thereof as compared to the non-isotopically enriched compound;
c) decreased average plasma levels of at least one metabolite of said compound per dosage unit thereof as compared to the non-isotopically enriched compound; and
d) an improved clinical effect during the treatment of said subject per dosage unit thereof as compared to the non-isotopically enriched compound.

In yet further embodiments said compound has at least two of the following properties:
a) decreased inter-individual variation in plasma levels of said compound or a metabolite thereof as compared to the non-isotopically enriched compound;
b) increased average plasma levels of said compound per dosage unit thereof as compared to the non-isotopically enriched compound;
c) decreased average plasma levels of at least one metabolite of said compound per dosage unit thereof as compared to the non-isotopically enriched compound; and
d) an improved clinical effect during the treatment of said subject per dosage unit thereof as compared to the non-isotopically enriched compound.

In yet further embodiments said compound has a decreased metabolism by at least one polymorphically-expressed cytochrome P₄₅₀ isoform in said subject per dosage unit thereof as compared to the non-isotopically enriched compound.

In yet further embodiments said cytochrome P₄₅₀ isoform is selected from the group consisting of CYP2C8, CYP2C9, CYP2C 19, and CYP2D6.

In yet further embodiments said compound is characterized by decreased inhibition of at least one cytochrome P₄₅₀ or monoamine oxidase isoform in said subject per dosage unit thereof as compared to the non-isotopically enriched compound.

In yet further embodiments said cytochrome P₄₅₀ or monoamine oxidase isoform is selected from the group consisting of CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B 1, CYP39, CYP46, CYP51, MAO_{A}, and MAO_{B}.

### DETAILED DESCRIPTION

To facilitate understanding of the disclosure set forth herein, a number of terms are defined below. Generally, the nomenclature used herein and the laboratory procedures in organic chemistry, medicinal chemistry, and pharmacology described herein are those well known and commonly employed in the art. Unless defined otherwise, all technical and scientific terms used herein generally have the same meaning as commonly understood in the art to which this disclosure belongs. In the event that there is a plurality of definitions for a term used herein, those in this section prevail unless stated otherwise.

As used herein, the singular forms "a," "an," and "the' may refer to plural articles unless specifically stated otherwise.

The term "subject" refers to an animal, including, but not limited to, a primate (e.g., human monkey, chimpanzee, gorilla, and the like), rodents (e.g., rats, mice, gerbils, hamsters, ferrets, and the like), lagomorphs, swine (e.g., pig, miniature pig), equine, canine, feline, and the like. The terms "subject" and "patient" are used interchangeably herein in reference, for example, to a mammalian subject, such as a human subject.

The terms "treat," "treating," and "treatment" are meant to include alleviating or abrogating a disease, disorder, or condition; or one or more of the symptoms associated with the disorder, disease, or condition; or alleviating or eradicating the cause(s) of the disease, disorder, or condition itself.

The terms "prevent," "preventing," and "prevention" refer to a method of delaying or precluding the onset of a disease, disorder, or condition; and/or its attendant symptoms, barring a subject from acquiring a disease disorder, or condition or reducing a subject's risk of acquiring a disease, disorder, or condition.

The term "therapeutically effective amount" refers to the amount of a compound that, when administered, is sufficient to prevent development of, or alleviate to some extent, one or more of the symptoms of the disease, disorder, or condition being treated. The term "therapeutically effective amount" also refers to the amount of a compound that is sufficient to elicit the biological or medical response of a cell, tissue, system, animal, or human that is being sought by a researcher, veterinarian, medical doctor, or clinician.

The term "pharmaceutically acceptable carrier," "pharmaceutically acceptable excipients," "physiologically acceptable carrier," or "physiologically acceptable excipient" refers to a pharmaceutically-acceptabe material, composition, or vehicle, such as a liquid or solid filler, diluent, excipient, solvent, or encapsulating material. Each component must be "phannaceutically acceptable" in the sense of being compatible with the other ingredients of a pharmaceutical fonnulation. It must also be suitable for use in contact with the tissue or organ of humans and animals without excessive toxicity, irritation, allergic response, immunogenecity, or other problems or complications, commensurate with a reasonable benefit/risk ratio. *See.* Remington: The Science and Practice of Pharmacy, 21st Edition; Lippincott Williams & Wilkins: Philadelphia, PA, 2005; Handbook of Pharmaceutical Excipients, 5th Edition; Rowe et al., Eds., The Pharmaceutical Press and the American Pharmaceutical Association: 2005; and Handbook of Pharmaceutical Additives, 3rd Edition; Ash and Ash Eds., Gower Publishing Company: 2007; Pharmaceutical Preformulation and Formulation, Gibson Ed., CRC Press LLC: Boca Raton, FL, 2004).

The term "pharmaceutically acceptable salts" includes hydrochloric salt, hydrobromic salt, hydroiodic salt, hydrofluoric salt, sulfuric salt, citric salt, maleic salt, acetic salt, lactic salt, nicotinic salt, succinic salt, oxalic salt, phosphoric salt, malonic salt, salicylic salt, phenylacetic salt, stearic salt, pyridine salt, ammonium salt, piperazine salt, diethylamine salt, nicotinamide salt, formic salt, urea salt, sodium salt, potassium salt, calcium salt, magnesium salt, zinc salt, lithium salt, cinnamic salt, methylamino salt, methanesulfonic salt, picric salt, tartaric salt, triethylamino salt, dimethylamino salt, tris(hydroxymethyl)aminumethane salt and the like. Additional pharmaceutically acceptable salts are known to those of skill in the art.

The tenn "pharmaceutically acceptable" refers to a compound, additive or composition that is not biologically or otherwise undesirable. For example, the additive or composition may be administered to a subject along with a compound of the invention without causing any undesirable biological effects or interacting in an undesirable manner with any of the other components of the pharmaceutical composition in which it is contained.

The term "deuterium enrichment" refers to the percentage of incorporation of deuterium at a given position in a molecule in the place of hydrogen. For example, deuterium enrichment of 1% at a given position means that I % of molecules in a given sample contain deuterium at the specified position. Because the naturally occurring distribution of deuterium is about 0.0156%, deuterium enrichment at any position in a compound synthesized using non-enriched starting materials is about 0.0156%. The deuterium enrichment can be detennined using conventional analytical methods known to one of ordinary skill in the art, including mass spectrometry and nuclear magnetic resonance spectroscopy.

The term "is/are deuterium," when used to describe a given position in a molecule such as R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, N₆₆, R₆₇, R₆₈, R₆₉, R₇₀, and R₇₁, means that the specified position is enriched with deuterium above the naturally occurring distribution of deuterium. In an embodiment deuterium enrichment is of no less than about 1%, in another no less than about 5%, in another no less than about 10%, in another no less than about 20%, in another no less than about 50%, in another no less than about 70%, in another no less than about 80%, in another no less than about 90%, or in another no less than about 95% of deuterium at the specified position.

The tenn "isotopic enrichment" refers to the percentage of incorporation of a less prevalent isotope of an element at a given position in a molecule in the place of the more prevalent isotope of the element.

The term "non-isotopically enriched" refers to a molecule in which the percentages of the various isotopes are substantially the same as the naturally occurring percentages.

The tenns "substantially pure" and "substantially homogeneous" mean sufficiently homogeneous to appear free of readily detectable impurities as determined by standard analytical methods used by one of ordinary skill in the art, including, but not limited to, thin layer chromatography (TLC), gel electrophoresis, high performance liquid chromatography (HPLC), infrared spectroscopy (IR), gas chromatography (GC), Ultraviolet Spectroscopy (UV), nuclear magnetic resonance (NMR), and mass spectroscopy (MS); or sufficiently pure such that further purification would not detectably alter the physical and chemical properties, or biological and pharmacological properties, such as enzymatic and biological activities, of the substance. In certain embodiments, "substantially pure" or "substantially homogeneous" refers to a collection of molecules, wherein at least about 50%, at least about 70%, at least about 80%, at least about 90%, at least about 95%, at least about 98%, at least about 99%, or at least about 99.5% of the molecules are a single compound, including a racemic mixture or single stereoisomer thereof, as detennined by standard analytical methods.

The terms "optically pure compound" or "optically pure isomer" refers to a single stereoisomer of a chiral compound regardless of the configuration of the said compound.

The term "about" or "approximately" means an acceptable error for a particular value, which depends in part on how the value is measured or determined. In certain embodiments, "about" can mean 1 or more standard deviations.

The terms "active ingredient" and "active substance" refer to a compound, which is administered, alone or in combination with one or more pharmaceutically acceptable excipients or carriers, to a subject for treating, preventing, or ameliorating one or more symptoms of a disorder or disease.

The term "pharmaceutical composition" refers to a mixture of a compound disclosed herein with other chemical components, such as diluents or carriers. The pharmaceutical composition facilitates administration of the compound to an organism. Multiple techniques of administering a compound exist in the art including, but not limited to, oral, injection, aerosol, parenteral, and topical administration. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like.

The term "carrier" defines a chemical compound that facilitates the incorporation of a compound into cells or tissues. For example dimethyl sulfoxide (DMSO) is a commonly utilized carrier as it facilitates the uptake of many organic compounds into the cells or tissues of an organism.

The term "diluent" defines a solution, typically one that is aqueous or partially aqueous, that dissolves chemical compounds of interest and may stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound.

The tenns "drug," "therapeutic agent," and "chemotherapeutic agent" refer to a compound, or a pharmaceutical composition thereof, which is administered to a subject for treating, preventing, or ameliorating one or more symptoms of a disorder or disease.

The term "disease" as used herein is intended to be generally synonymous, and is used interchangeably with, the tenns "disorder" and "condition" (as in medical condition), in that all reflect an abnonnal condition of the body or of one of its parts that impairs normal functioning and is typically manifested by distinguishing signs and symptoms.

The term "release controlling excipient" refers to an excipient whose primary function is to modify the duration or place of release of the active substance from a dosage form as compared with a conventional immediate release dosage form.

The term "nonrelease controlling excipient" refers to an excipient whose primary function do not include modifying the duration or place of release of the active substance from a dosage form as compared with a conventional immediate release dosage form.

The tenn "5-hydroxytryptamine 1B and/or 1D receptors" or "5-HT 1B and 5-HT 1D receptors" refers to two subtypes of a larger 5-Hydroxytryptamine (5-HT) receptor family. 5-HT receptors are located on the cell membrane of nerve cells and other cell types in animals. 5-HT receptors mediate the effects of serotonin, the endogenous ligand, and a broad range of phannaceutical and hallucinogenic drugs. 5-HT 1B and 5-HT 1D receptors are G protein coupled seven transmembrane (or *heptahelical*) receptors that activate an intracellular second messenger cascade. Although 5-HT 1B and 5-HT 1D receptors differ widely at the amino acid level, they both bind various ligands with similar potencies. Both receptor subtypes are involved with vascular constriction.

When used in conjunction with a compound of this invention, the tenns "elicit", "eliciting," "modulator", "modulate", "modulating", "regulator", "regulate" or "regulating" the activity refer to a compound that can act as an agonist, a partial agonist, an inverse agonist, an inhibitor, or an antagonist of a particular enzyme or receptor, such as for example a 5-HT_{1B} receptor or 5-HT_{1D} receptor.

The tenn "Lewis acid" refers to a molecule that can accept an unshared pair of electrons and as such would be obvious to one of ordinary skill and knowledge in the art. The definition of "Lewis acid" includes but is not limited to: boron trifluoride, boron trifluoride etherate, boron trifluoride tetrahydrofuran complex, boron trifluoride tert-butyl-methyl ether complex, boron trifluoride dibutyl ether complex, boron trifluoride dihydrate, boron trifluoride di-acetic acid complex, boron trifluoride dimethyl sulfide complex, boron trichloride, boron trichloride dimethyl sulfide complex, boron tribromide, boron tribromide dimethyl sulfide complex, boron triiodide, triimethoxyborane, triethoxyborane, trimethylaluminum, triethylaluminum, aluminum trichloride, aluminum trichloride tetrahydrofuran complex, aluminum tribromide, titanium tetrachloride, titanium tetrabromide, titanium iodide, titanium tetraethoxide, titanium tetraisopropoxide, scandium (III) trifluoromethanesulfonate, yttrium (III) trifluoromethanesulfonate, ytterbium (III) trifluoromethanesulfonate, lanthanum (III) trifluoromethanesulfonate, zinc (II) chloride, zinc (II) bromide, zinc (II) iodide, zinc (II) trifluoromethanesulfonate, zinc (II) sulfate, magnesium sulfate, Lithium perchlorate, copper (II) trifluoromethanesulfonate, copper (II) tetrafluoroborate and the like. Certain Lewis acids may have optically pure ligands attached to the electron acceptor atom, as set forth in Corey, E. J. Angewandte Chemie, International Edition (2002), 41(10), 1650-1667; Aspinall, H. C. Chemical Reviews (Washington, DC, United States) (2002), 102(6), 1807-1850; Groger, H. Chemistry-A European Journal (2001), 7(24), 5246-5251; Davies, H. M. L. Chemtracts (2001), 14(11), 642-645; Wan, Y. Chemtracts (2001), 14(11), 610-615; Kim, Y. H. Accounts of Chemical Research (2001), 34(12), 955-962; Seebach, D. Angewandte Chemie, International Edition (2001), 40(1), 92-138; Blaser, H. U. Applied Catalysis, A: General (2001), 221(1-2), 119-143; Yet, L. Angewandte Chemie, International Edition (2001), 40(5), 875-877; Jorgensen, K. A. Angewandte Chemie, International Edition (2000), 39(20), 3558-3588; Dias, L. C. Current Organic Chemistry (2000), 4(3), 305-342; Spindler, F. Enantiomer (1999), 4(6), 557-568; Fodor, K. Enantiomer (1999), 4(6), 497-511; Shimizu, K. D.; Comprehensive Asymmetric Catalysis I-III (1999), 3, 1389-1399; Kagan, H. B. Comprehensive Asymmetric Catalysis I-III (1999), 1, 9-30; Mikami, K. Lewis Acid Reagents (1999), 93-136 and all references cited therein. Such Lewis acids may be used by one of ordinary skill and knowledge in the art to produce optically pure compounds from achiral starting materials.

The term "acylating agent" refers to a molecule that can transfer an alkylcarbonyl, substituted alkylcarbonyl or aryl carbonyl group to another molecule. The definition of "acylating agent" includes but is not limited to ethyl acetate, vinyl acetate, vinyl propionate, vinyl butyrate, isopropenyl acetate, 1-ethoxyvinyl acetate, trichloroethyl butyrate, trifluoroethyl butyrate, trifluoroethyl laureate, S-ethyl thiooctanoate, biacetyl monooxime acetate, acetic anhydride, acetyl chloride, succinic anhydride, diketene, diallyl carbonate, carbonic acid but-3-enyl ester cyanomethyl ester, amino acid and the like.

The tenn "nucleophile" or "nucleophilic reagent" refers to a negatively charged or neutral molecule that has an unshared pair of electrons and as such would be obvious to one of ordinary skill and knowledge in the art. The definition of "nucleophile" includes but is not limited to: water, alkylhydroxy, alkoxy anion, arylhydroxy, aryloxy anion, alkylthiol, alkylthio anion, arylthiol, arylthio anion, ammonia, alkylamine, arylamine, alkylamine anion, arylamine anion, hydrazine, alkyl hydrazine, arylhydrazine, alkylcarbonyl hydrazine, arylcarbonyl hydrazine, hydrazine anion, alkyl hydrazine anion, arylhydrazine anion, alkylcarbonyl hydrazine anion, arylcarbonyl hydrazine anion, cyanide, azide, hydride, alkyl anion, aryl anion and the like.

The tenn "electrophile" or "electrophilic reagent" refers to a positively charged or neutral molecule that has an open valence shell or an attraction for an electron-rich reactant and as such would be obvious to one of ordinary skill and knowledge in the art. The definition of "electrophile" includes but is not limited to: hydronium, acylium, Lewis acids, such as for example, boron trifluoride and the like, halogens, such as for example Br₂ and the like, carbocations, such as for example tert-butyl cation and the like, diazomethane, trimethylsilyldiazomethane, alkyl halides, such as for example methyl iodide, trideuteromethyl iodide (CD₃I), benzyl bromide and the like, alkyl triflates, such as for example methyl triflate and the like, alkylsulfonates, such as for example ethyl toluenesulfonate, butyl methanesulfonate, dimethylsulfate, hexadeuterodimethylsulfate ((CD₃)₂SO₄) and the like, acyl halides, such as for example acetyl chloride, benzoyl bromide and the like, acid anhydrides, such as for example acetic anhydride, succinic anhydride, maleic anhydride and the like, isocyanates, such as for example methyl isocyanate, phenylisocyanate and the like, chloroformates, such as for example methyl chloroformate, ethyl chloroformate, benzyl chloroformate and the like, sulfonyl halides, such as for example methanesulfonyl chloride, p-toluenesulfonyl chloride and the like, silyl halides, such as for example trimethylsilyl chloride, tert-butyldimethylsilyl chloride and the like, phosphoryl halide such as for example dimethyl chlorophosphate and the like, alpha-beta-unsaturated carbonyl compounds such as for example acrolein, methyl vinyl ketone, cinnamaldehyde and the like.

The term "oxidant" refers to any reagent that will increase the oxidation state of an atom, such as for example, hydrogen, carbon, nitrogen, sulfur, phosphorus and the like in the starting material by either adding an oxygen to this atom or removing an electron from this atom and as such would be obvious to one of ordinary skill and knowledge in the art. The definition of "oxidant" includes but is not limited to: osmium tetroxide, ruthenium tetroxide, ruthenium trichloride, potassium permanganate, metachloroperbenzoic acid, hydrogen peroxide, dimethyl dioxirane and the like.

The term "metal ligand" refers to a molecule that has an unshared pair of electrons and can coordinate to a metal atom and as such would be obvious to one of ordinary skill and knowledge in the art. The definition of "metal ligand" includes but is not limited to: water, alkoxy anion, alkylthio anion, ammonia, trialkylamine, triarylamine, trialkylphosphine, triarylphosphine, cyanide, azide and the like.

The term "reducing reagent" refers to any reagent that will decrease the oxidation state of an atom in the starting material by either adding a hydrogen to this atom, or adding an electron to this atom, or by removing an oxygen from this atom and as such would be obvious to one of ordinary skill and knowledge in the art. The definition of "reducing reagent" includes but is not limited to: borane-dimethyl sulfide complex, 9-borabicyclo[3.3.1.]nonane (9-BBN), catechol borane, lithium borohydride, lithium borodeuteride, sodium borohydride, sodium borodeuteride, sodium borohydride-methanol complex, potassium borohydride, sodium hydroxyborohydride, lithium triethylborohydride, lithium n-butylborohydride, sodium cyanoborohydride, sodium cyanoborodeuteride, calcium (II) borohydride, lithium aluminum hydride, lithium aluminum deuteride, diisobutylaluminum hydride, n-butyl-diisobutylaluminum hydride, sodium bis-methoxyethoxyaluminum hydride, triethoxysilane, diethoxymethylsilane, lithium hydride, lithium, sodium, hydrogen Ni/B, and the like. Certain acidic and Lewis acidic reagents enhance the activity of reducing reagents. Examples of such acidic reagents include: acetic acid, methanesulfonic acid, hydrochloric acid, and the like. Examples of such Lewis acidic reagents include: trimethoxyborane, triethoxyborane, aluminum trichloride, lithium chloride, vanadium trichloride, dicyclopentadienyl titanium dichloride, cesium fluoride, potassium fluoride, zinc (II) chloride, zinc (II) bromide, zinc (II) iodide, and the like.

The tenn "coupling reagent" refers to any reagent that will activate the carbonyl of a carboxylic acid and facilitate the formation of an ester or amide bond. The definition of "coupling reagent" includes but is not limited to: acetyl chloride, ethyl chloroformate, dicyclohexylcarbodiimide (DCC), diisopropyl carbodiiimide (DIC), 1-ethyl-3-(3-dimethylaminopropyl) carbodiimide (EDCI), N-hydroxybenzotriazole (HOBT), N-hydroxysuccinimide (HOSu), 4-nitrophenol, pentafluorophenol, 2-(1H-benzotriazole-1-yl)-1,1,3,3-tetramethyluronium tetrafluoroborate (TBTU), O-benzotriazole-N,N,N'N'-tetramethyluronium hexafluorophosphate (HBTU), benzotriazole-1-yl-oxy-tris-(dimethylamino)-phosphonium hexafluorophosphate (BOP), benzotriazole-1-yl-oxy-tris-pyrrolidinophosphonium hexafluorophosphate, bromo-trispyrrolidino- phosphonium hexafluorophosphate, 2-(5-norbornene-2,3-dicarboximido)-1,1,3,3-tetramethyluronium tetrafluoroborate (TNTU), O-(N-succinimidyl)-1,1,3,3-tetramethyluronium tetrafluoroborate (TSTU), tetramethylfluoroformamidinium hexafluorophosphate and the like.

The term "protecting group" or "removable protecting group" refers to a group which, when bound to a functionality, such as the oxygen atom of a hydroxyl or carboxyl group, or the nitrogen atom of an amino group, prevents reactions from occurring at that functional group, and which can be removed by a conventional chemical or enzymatic step to reestablish the functional group (Greene and Wuts, Protective Groups in Organic Synthesis, 3rd Ed., John Wiley & Sons, New York, NY, 1999).

The definition of "hydroxyl protecting group" includes but is not limited to:
a) Methyl, tert-butyl, allyl, propargyl, p-chlorophenyl, p-methoxyphenyl, p-nitrophenyl, 2,4-dinitrophenyl, 2,3,5,6-tetrafluoro-4-(trifluoromethyl)phenyl, methoxymethyl, methylthiomethyl, (phenyldimethylsilyl)methoxymethyl, benzyloxymethyl, p-methoxy-benzyloxymethyl, p-nitrobenzyloxymethyl, o-nitrobenzyloxymethyl, (4-methoxyphenoxy)methyl, guaiacolmethyl, tert-butoxymethyl, 4-pentenyloxymethyl, tert-butyldimethylsiloxymethyl, thexyldimethylsiloxymethyl, tert-butyldiphenylsiloxymethyl, 2-methoxyethoxymethyl, 2,2,2-trichloroethoxymethyl, bis(2-chloroethoxy)methyl, 2-(trimethylsilyl)ethoxymethyl, menthoxymethyl, 1-ethoxyethyl, 1-(2-chloroethoxy)ethyl, 1-[2-(trimethylsilyl)ethoxy]ethyl, 1-methyl-1-ethoxyethyl, 1-methyl-1-benzyloxyethyl, 1-methyl-1-benzyloxy-2-fluoroethyl, 1-methyl-1-phenoxyethyl, 2,2,2-trichloroethyl, 1-dianisyl-2,2,2-trichloroethyl, 1,1,1,3,3,3-hexafluoro-2-phenylisopropyl, 2-trimethylsilylethyl, 2-(benzylthio)ethyl, 2-(phenylselenyl)ethyl, tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydropyranyl S,S-dioxide, 1-[(2-chloro-4-methyl)phenyl]-4-methoxypiperidin-4-yl, 1-(2-fluorophenyl)-4-methoxypiperidin-4-yl, 1,4-dioxan-2-yl, tetrahydrofuranyl, tetrahydrothiofuranyl and the like;
b) Benzyl, 2-nitrobenzyl, 2-trifluoromethylbenzyl, 4-methoxybenzyl, 4-nitrobenzyl, 4-chlorobenzyl, 4-bromobenzyl, 4-cyanobenzyl, 4-phenylbenzyl, 4-acylaminobenzyl, 4-azidobenzyl, 4-(methylsulfinyl)benzyl, 2,4-dimethoxybenzyl, 4-azido-3-chlorobenzyl, 3,4-dimethoxybenzyl, 2,6-dichlorobenzyl, 2,6-difluorobenzyl, 1-pyrenylmethyl, diphenylmethyl, 4,4'-dinitrobenzhydryl, 5-benzosuberyl, triphenylmethyl (trityl), α-naphthyldiphenylmethyl, (4-methoxyphenyl)-diphenyl-methyl, di-(p-methoxyphenyl)-phenylmethyl, tri-(p-methoxyphenyl)methyl, 4-(4'-bromophenacyloxy)-phenyldiphenylmethyl, 4,4',4"-tris(4,5-dichlorophthalimidophenyl)methyl, 4,4',4"-tris(levulinoyloxyphenyl)methyl, 4,4'-dimethoxy-3"-[N-(imidazolylmethyl)]trityl, 4,4'-dimethoxy-3"-[N-(imidazolylethyl)carbamoyl]trityl, 1,1-bis(4-methoxyphenyl)-1'-pyrenylmethyl, 4-(17-tetrabenzo[a,c,g,i]fluorenylmethyl)-4,4'-dimethoxytrityl, 9-anthryl, 9-(9-phenyl)xanthenyl, 9-(9-phenyl-10-oxo)anthryl and the like;
c) Trimethylsilyl, triethylsilyl, triisopropylsilyl, dimethylisopropylsilyl, diethylisopropylsilyl, dimethylhexylsilyl, tert-butyldimethylsilyl, tert-butyldiphenylsilyl, tribenzylsilyl, tri-p-xylylsilyl, triphenylsilyl, diphenylmethylsilyl, di-tert-butylmethylsilyl, tris(trimethylsilyl)silyl, (2-hydroxystyryl)dimethylsilyl, (2-hydroxystyryl)diisopropylsilyl, tert-butylmethoxyphenylsilyl, tert-butoxydiphenylsilyl and the like;
d) -C(O)R₈₀, where R₈₀ is selected from the group consisting of alkyl, substituted alkyl, aryl and more specifically R₈₀ = hydrogen, methyl, ethyl, tert-butyl, adamantyl, crotyl, chloromethyl, dichloromethyl, trichloromethyl, trifluoromethyl, methoxymethyl, triphenylmethoxymethyl, phenoxymethyl, 4-chlorophenoxymethyl, phenylmethyl, diphenylmethyl, 4-methoxycrotyl, 3-phenylpropyl, 4-pentenyl, 4-oxopentyl, 4,4-(ethylenedithio)pentyl, 5-[3-bis(4-methoxyphenyl)hydroxymethylphenoxy]- 4-oxopentyl, phenyl, 4-methylphenyl, 4-nitrophenyl, 4-fluorophenyl, 4-chlorophenyl, 4-methoxyphenyl, 4-phenylphenyl, 2,4,6-trimethylphenyl, α-naphthyl, benzoyl and the like;
e) -C(O)OR₈₀, where R₈₀ is selected from the group consisting of alkyl, substituted alkyl, aryl and more specifically R₈₀ = methyl, methoxymethyl, 9-fluorenylmethyl, ethyl, 2,2,2-trichloromethyl, 1,1-dimethyl-2,2,2-trichloroethyl, 2-(trimethylsilyl)ethyl, 2-(phenylsulfonyl)ethyl, isobutyl, tert-butyl, vinyl, allyl, 4-nitrophenyl, benzyl, 2-nitrobenzyl, 4-nitrobenzyl, 4-methoxybenzyl, 2,4-dimethoxybenzyl, 3,4-dimethoxybenzyl, 2-(methylthiomethoxy)ethyl, 2-dansenylethyl, 2-(4-nitrophenyl)ethyl, 2-(2,4-dinitrophenyl)ethyl, 2-cyano-1-phenylethyl, thiobenzyl, 4-ethoxy-1-naphthyl and the like. Other examples of hydroxyl protecting groups are given in Greene and Wutts, above.

The definition of "amino protecting group" includes but is not limited to:
2-methylthioethyl, 2-methylsulfonylethyl, 2-(p-toluenesulfonyl)ethyl, [2-(1,3-dithianyl)]methyl, 4-methylthiophenyl, 2,4-dimethylthiophenyl, 2-phosphonioethyl, 1-methyl-1-(triphenylphosphonio)ethyl, 1,1-dimethyl-2-cyanoethyl, 2-dansylethyl, 2-(4-nitrophenyl)ethyl, 4-phenylacetoxybenzyl, 4-azidobenzyl, 4-azidomethoxybenzyl, m-chloro-p-acyloxybenzyl, p-(dihydroxyboryl)benzyl, 5-benzisoxazolylmethyl, 2-(trifluoromethyl)-6-chromonylmethyl, m-nitrophenyl, 3.5-dimethoxybenzyl, 1-methyl-1-(3,5-dimethoxyphenyl)ethyl, o-nitrobenzyl, α-methylnitropiperonyl, 3,4-dimethoxy-6-nitrobenzyl, N-benzenesulfenyl, N-o-nitrobenzenesulfenyl, N-2,4-dinitrobenzenesulfenyl, N-pentachlorobenzenesulfenyl. N-2-nitro-4-methoxybenzenesulfenyl, N-triphenylmethylsulfenyl, N-1-(2,2,2-trifluoro-1,1-diphenyl)ethylsulfenyl, N-3-nitro-2-pyridinesulfenyl, N-p-toluenesulfonyl, N-benzenesulfonyl, N-2,3,6-trimethyl-4-methoxybenzenesulfonyl, N-2,4,6-trimethoxybenzene-sulfonyl, N-2,6-dimethyl-4-methoxybenzenesulfonyl, N-pentamethylbenzenesulfonyl, N-2,3,5.6-tetramethyl-4-methoxybenzenesulfonyl and the like;
-C(O)OR₈₀, where R₈₀ is selected from the group consisting of alkyl, substituted alkyl, aryl and more specifically R₈₀ = methyl, ethyl, 9-fluorenylmethyl, 9-(2-sulfo)fluorenylmethyl. 9-(2,7-dibromo)fluorenylmethyl, 17-tetrabenzo[a,c,g,i]fluorenylmethyl. 2-chloro-3-indenylmethyl, benz[f]inden-3-ylmethyl, 2,7-di-t-butyl-[9-(10,10-dioxo-10,10,10,10-tetrahydrothloxanthyl)]methyl, 1,1-dioxobenzo[b]thiophene-2-ylmethyl, 2,2,2-trichloroethyl, 2-trimethylsilylethyl, 2-phenylethyl, 1-(1-adamantyl)-1-methylethyl, 2-chloroethyl, 1,1-dimethyl-2-haloethyl, 1,1-dimethyl-2,2-dibromoethyl, 1,1-dimethyl-2,2,2-trichloroethyl, 1-methyl-1-(4-biphenylyl)ethyl, 1-(3,5-di-tert-butylphenyl)-1-methylethyl, 2-(2'-pyridyl)ethyl, 2-(4'-pyridyl)ethyl, 2,2-bis(4'-nitrophenyl)ethyl, N-(2-pivaloylamino)-1,1-dimethylethyl, 2-[(2-nitrophenyl)dithio]-1-phenylethyl, tert-butyl, 1-adamantyl, 2-adamantyl, Vinyl, allyl, 1-Isopropylallyl, cinnamyl. 4-nitrocinnamyl, 3-(3-pyridyl)prop-2-enyl, 8-quinolyl, N-Hydroxypiperidinyl, alkyldithio, benzyl, p-methoxybenzyl, p-nitrobenzyl, p-bromobenzyl. p-chlorobenzyl, 2,4-dichlorobenzyl, 4-methylsulfinylbenzyl, 9-anthrylmethyl, diphenylmethyl, tert-amyl, S-benzyl thiocarbamate, butynyl, p-cyanobenzyl, cyclobutyl, cyclohexyl, cyclopentyl, cyclopropylmethyl, p-decyloxybenzyl, diisopropylmethyl, 2,2-dimethoxycarbonylvinyl, o-(N,N'-dimethylcarboxamido)benzyl, 1,1-dimethyl-3-(N,N'-dimethylcarboxamido)propyl, 1,1-dimethylpropynyl, di(2-pyridyl)methyl, 2-furanylmethyl, 2-lodoethyl, isobornyl, isobutyl, isonicotinyl, p-(p'-methoxyphenylazo)benzyl, 1-methylcyclobutyl, 1-methylcyclohexyl, 1-methyl-1-cyclopropylmethyl, 1-methyl-1-(p-phenylazophenyl)ethyl, 1-methyl-1-phenylethyl, 1-methyl-1-4'-pyridylethyl, phenyl, p-(phenylazo)benzyl, 2,4,6-trimethylphenyl, 4-(trimethylammonium)benzyl, 2,4,6-trimethylbenzyl and the like. Other examples of amino protecting groups are given in Greene and Wutts, above.

The definition of "carboxyl protecting group" includes but is not limited to: 2-N-(morpholino)ethyl, choline, methyl, methoxyethyl, 9-fluorenylmethyl, methoxymethyl, methylthiomethyl, tetrahydropyranyl, tetrahydrofuranyl, methoxyethoxymethyl, 2-(trimethylsilyl)ethoxymethyl, benzyloxymethyl, pivaloyloxymethyl, phenylacetoxymethyl, triisopropylsilylmethyl, cyanomethyl, acetol, p-bromophenacyl. α-methylphenacyl, p-methoxyphenacyl, desyl, carboxamidomethyl, p-azobenzenecarboxamido-methyl, N-phthalimidomethyl, (methoxyethoxy)ethyl, 2,2,2-trichloroethyl, 2-fluoroethyl, 2-chloroethyl, 2-bromoethyl, 2-iodoethyl, 4-chlorobutyl, 5-chloropentyl, 2-(trimethylsilyl)ethyl, 2-methylthioethyl, 1,3-dithianyl-2-methyl, 2-(p-nitrophenylsulfenyl)ethyl, 2-(p-toluenesulfonyl)ethyl, 2-(2-pyridyl)ethyl, 2-(p-methoxyphenyl)ethyl, 2-(diphenylphosphino)ethyl, 1-methyl-1-phenylethyl, 2-(4-acetyl-2-nitrophenyl)ethyl, 2-cyanoethyl, heptyl, tert-butyl, 3-methyl-3-pentyl, dicyclopropylmethyl, 2,4-dimethyl-3-pentyl, cyclopentyl, cyclohexyl, allyl, methallyl, 2-methylbut-3-en-2-yl, 3-methylbut-2-(prenyl), 3-buten-1-yl, 4-(trimethylsilyl)-2-buten-1-yl, cinnamyl, α-methylcinnamyl, propargyl, phenyl, 2,6-dimethylphenyl, 2,6-diisopropylphenyl, 2,6-di-tert-butyl-4-methylphenyl, 2,6-di-tert-butyl-4-methoxyphenyl, p-(methylthio)phenyl, pentafluorophenyl, benzyl, triphenylmethyl, diphenylmethyl, bis(o-nitrophenyl)methyl, 9-anthrylmethyl, 2-(9,10-dioxo)anthrylmethyl. 5-dibenzosuberyl, 1-pyrenylmethyl, 2-(trifluoromethyl)-6-chromonylmethyl, 2,4,6-trimethylbenzyl, p-bromobenzyl, o-nitrobenzyl, p-nitrobenzyl, p-methoxybenzyl, 2.6-dimethoxybenzyl, 4-(methylsulfinyl)benzyl, 4-Sulfobenzyl, 4-azidomethoxybenzyl, 4-{N-[1-(4,4-dimethyl-2,6-dioxocyclohexylidene)-3-methylbutyl]amino}benzyl, piperonyl, 4-picolyl, trimethylsilyl, triethylsilyl, tert-butyldimethylsilyl, isopropyldimethylsilyl, phenyldimethylsilyl, di-tert-butylmethylsilyl, triisopropylsilyl and the like. Other examples of carboxyl protecting groups are given in Greene and Wutts, above.

The definition of "thiol protecting group" includes but is not limited to:
Alkyl, benzyl, 4-methoxybenzyl, 2-hydroxybenzyl, 4-hydroxybenzyl, 2-acetoxybenzyl, 4-acetoxybenzyl, 4-nitrobenzyl, 2,4,6-trimethylbenzyl, 2,4,6-trimethoxybenzyl, 4-picolyl, 2-quinolinylmethyl, 2-picolyl n-oxido, 9-anthrylmethyl, 9-fluorenylmethyl, xanthenyl, ferrocenylmethyl and the like;
Diphenylmethyl, bis(4-methoxyphenyl)methyl, 5-dibenzosuberyl, triphenylmethyl, diphenyl-4-pyridylmethyl, phenyl, 2,4-dinitrophenyl, tert-butyl, 1-adamantyl and the like;
Methoxymethyl, isobutoxymethyl, benzyloxymethyl, 2-tetrahydropyranyl, benzylthiomethyl, phenylthiomethyl, acetamidomethyl, trimethylacetamidomethyl, benzamidomethyl, allyloxycarbonylaminomethyl, phenylacetamidomethyl, phthalimidomethyl, acetyl, carboxy-, cyanomethyl and the like;
(2-nitro-1-phenyl)ethyl, 2-(2,4-dinitrophenyl)ethyl, 2-(4'-pyridyl)ethyl, 2-cyanoethyl, 2-(trimethylsilyl)ethyl, 2,2-bis(carboethoxy)ethyl, 1-(3-nitrophenyl)-2-benzoyl-ethyl, 2-phenylsulfonylethyl, 1-(4-methylphenylsulfonyl)-2-methylpro4-2-yl and the like;
Trimethylsilyl, triethylsilyl, triisopropylsilyl, dimethylisopropylsilyl, diethylisopropylsilyl, dimethylhexylsilyl, tert-butyldimethylsilyl, tert-butyldiphenylsilyl, tribenzylsilyl, tri-p-xylylsilyl, triphenylsilyl, diphenylmethylsilyl, di-tert-butylmethylsilyl, tris(trimethylsilyl)silyl, (2-hydroxystyryl)dimethylsilyl, (2-hydroxystyryl)diisopropylsilyl, tert-butylmethoxyphenylsilyl, tert-butoxydiphenylsilyl and the like;
Benzoyl, trifluoroacetyl, N-[[(4-biphenylyl)isopropoxy]carbonyl]-N-methyl-γ-aminothiobutyrate, N-(t-butoxycarbonyl)-N-methyl-γ-aminothiobutyrate and the like;
2,2,2-Trichloroethoxycarbonyl, tert-butoxycarbonyl, benzyloxycarbonyl, 4-methoxybenzyloxycarbonyl and the like;
N-(Ethylamino)carbonyl, N-(methoxymethylamino)carbonyl and the like;
Ethylthio, tert-butylthio, phenylthio, substituted phenylthio and the like;
(Dimethylphosphino)thioyl, (diphenylphosphino)thioyl and the like;
Sulfonate, alkyloxycarbonylthio, benzyloxycarbonylthio, 3-nitro-2-pyridinethio and the like;
Tricarbonyl[1,2,3,4,5-η]-2,4-cyclohexadien-1-yl]-iron(1+) and the like. Other examples of thiol protecting groups are given in Greene and Wutts, above.

The term "amino acid" refers to any of the naturally occurring amino acids, as well as synthetic analogs and derivatives thereof. Alpha-Amino acids comprise a carbon atom to which is bonded an amino group, a carboxy group, a hydrogen atom, and a distinctive group referred to as a "side chain". The side chains of naturally occurring amino acids are well known in the art and include, for example, hydrogen (e.g., as in glycine), alkyl (e.g., as in alanine, valine, leucine, isoleucine, proline), substituted alkyl (e.g., as in threonine, serine, methionine, cysteine, aspartic acid, asparagine, glutamic acid, glutamine, arginine, and lysine), arylalkyl (e.g., as in phenylalanine), substituted arylalkyl (e.g., as in tyrosine), heteroarylalkyl (e.g., as in tryptophan, histidine) and the like. One of skill in the art will appreciate that the term "amino acid" can also include beta-, gamma-, delta-, omega- amino acids, and the like. Unnatural amino acids are also known in the art, as set forth in, Natchus, M. G. Organic Synthesis: Theory and Applications (2001), 5, 89-196; Ager, D. J. Current Opinion in Drug Discovery & Development (2001), 4(6), 800; Reginato, G. Recent Research Developments in Organic Chemistry (2000), 4(Pt. 1), 351-359; Dougherty, D. A. Current Opinion in Chemical Biology (2000), 4(6), 645-652; Lesley, S. A. Drugs and the Pharmaceutical Sciences (2000), 101(Peptide and Protein Drug Analysis), 191-205; Pojitkov, A. E. Journal of Molecular Catalysis B: Enzymatic (2000), 10(1-3), 47-55; Ager, D. J. Speciality Chemicals (1999), 19(1), 10-12, and all references cited therein. Stereoisomers (e.g., D-amino acids) of the twenty conventional amino acids, unnatural amino acids such as alpha, alpha-disubstituted amino acids and other unconventional amino acids may also be suitable components for compounds of the present invention. Examples of unconventional amino acids include: 4-hydroxyproline, 3-methylhistidine, 5-hydroxylysine, and other similar amino acids and imino acids (e.g., 4-hydroxyproline).

The tenn "N-protected amino acid" refers to any amino acid which has a protecting group bound to the nitrogen of the amino functionality. This protecting group prevents reactions from occurring at the amino functional group and can be removed by conventional chemical or enzymatic steps to reestablish the amino functional group.

The term "O-protected amino acid" refers to any amino acid which has a protecting group bound to the oxygen of the carboxyl functionality. This protecting group prevents reactions from occurring at the carboxyl functional group and can be removed by conventional chemical or enzymatic steps to reestablish the carboxyl functional group. The particular protecting group employed is not critical.

The term "halogen'', "halide" or "halo" includes fluorine, chlorine, bromine, and iodine.

The term "leaving group" (LG.) refers to any atom (or group of atoms) that is stable in its anion or neutral form after it has been displaced by a nucleophile and as such would be obvious to one of ordinary skill and knowledge in the art. The definition of "leaving group" includes but is not limited to: water, methanol, ethanol, chloride, bromide, iodide, an alkylsulfonate, for example methanesulfonate, ethanesulfonate and the like, an arylsulfonate, for example benzenesulfonate, tolylsulfonate and the like, a perhaloalkanesulfonate, for example trifluoromethanesulfonate, trichloromethanesulfonate and the like, an alkylcarboxylate, for example acetate and the like, a perhaloalkylcarboxylate, for example trifluoroacetate, trichloroacetate and the like, an arylcarboxylate, for example benzoate and the like.

As used herein, the term "attached" signifies a stable covalent bond, certain preferred points of attachment being apparent to those skilled in the art.

The tenns "optional" or "optionally" refer to occurrence or non-occurrence of the subsequently described event or circumstance, and that the description includes instances where said event or circumstance occurs and instances where it does not. In such context, the sentence "optionally substituted alkyl group" means that the alkyl group may or may not be substituted and the description includes both a substituted and an unsubstituted alkyl group.

The terms "alkyl" and "substituted alkyl" are interchangeable and include substituted, optionally substituted and unsubstituted C₁-C₁₀ straight chain saturated aliphatic hydrocarbon groups, substituted, optionally substituted and unsubstituted C₂-C₁₀ straight chain unsaturated aliphatic hydrocarbon groups, substituted, optionally substituted and unsubstituted C₂-C₁₀ branched saturated aliphatic hydrocarbon groups, substituted and unsubstituted C₂-C₁₀ branched unsaturated aliphatic hydrocarbon groups, substituted, optionally substituted and unsubstituted C₃-C₈ cyclic saturated aliphatic hydrocarbon groups, substituted, optionally substituted and unsubstituted C₅-C₈ cyclic unsaturated aliphatic hydrocarbon groups having the specified number of carbon atoms. For example, the definition of "alkyl" shall include but is not limited to: methyl (Me), trideuteromethyl (-CD₃), ethyl (Et), propyl (Pr), butyl (Bu), pentyl, hexyl, heptyl, octyl, nonyl, decyl, undecyl, ethenyl, propenyl, butenyl, penentyl, hexenyl, heptenyl, octenyl, nonenyl, decenyl, undecenyl, isopropyl (i-Pr), isobutyl (i-Bu), tert-butyl (t-Bu), sec-butyl (s-Bu), isopentyl, neopentyl, cyclopropyl, cyclobutyl, cyclopentyl, cyclohexyl, cycloheptyl, cyclooctyl, cyclopentenyl, cyclohexenyl, cycloheptenyl, cyclooctenyl, methylcyclopropyl, ethylcyclohexenyl, butenylcyclopentyl, adamantyl, norbornyl and the like. Alkyl substituents are independently selected from the group consisting of hydrogen, deuterium, halogen, -OH, -SH, -NH₂, -CN, -NO₂, =O =CH₂, trihalomethyl, carbamoyl, arylC₀₋₁₀alkyl, heteroarylC₀₋₁₀alkyl, C₁₋₁₀alkyloxy, arylC₀₋₁₀alkyloxy, C₁₋₁₀alkylthio, ary]C₀₋₁₀alkylthio, C₁₋₁₀alkylamino, arylC₀₋₁₀alkylamino, N-aryl-N-C₀₋₁₀alkylamino, C₁₋₁₀alkylcarbonyl, arylC₀₋₁₀alkylcarbonyl, C₁₋₁₀alkylcarboxy, arylC₀₋₁₀alkylcarboxy, C₁₋₁₀alkylcarbonylamino, arylC₀₋₁₀alkylcarbonylamino, tetrahydrofuryl, morpholinyl, piperazinyl, hydroxypyronyl, -C₀₋₁₀alkylCOOR₈₀ and -C₀₋₁₀alkylCONR₈₁R₈₂ wherein R₈₀, R₈₁, and R₈₂ are independently selected from the group consisting of hydrogen, deuterium, alkyl, aryl, or R₈₂ and R₈₃ are taken together with the nitrogen to which they are attached forming a saturated cyclic or unsaturated cyclic system containing 3 to 8 carbon atoms with at least one substituent as defined herein.

The term "alkyloxy" (e.g. methoxy, ethoxy, propyloxy, allyloxy, cyclohexyloxy) represents a substituted or unsubstituted alkyl group as defined above having the indicated number of carbon atoms attached through an oxygen bridge. The tenn "alkyloxyalkyl" represents an alkyloxy group attached through an alkyl or substituted alkyl group as defined above having the indicated number of carbon atoms.

The term "alkyloxycarbonyl" (e.g. methoxycarbonyl, ethoxycarbonyl, tert-butoxycarbonyl, allyloxycarbonyl) represents a substituted or unsubstituted alkyloxy group as defined above having the indicated number of carbon atoms attached through a carbonyl bridge.

The term "alkylsulfonate" (e.g. methylsufonate, ethylsulfonate, propylsulfonate, cyclosulfonate and the like) represents a substituted or unsubstituted alkyl group as defined above having the indicated number of carbon atoms attached through a sulfonate bridge. The term "alkylsulfoante" represents a alkylsunfonate group attached through an alkyl or substituted alkyl group as defined above having the indicated number of carbon atoms.

The term "alkylthio" (e.g. methylthio, ethylthio, propylthio, cyclohexenylthio and the like) represents a substituted or unsubstituted alkyl group as defined above having the indicated number of carbon atoms attached through a sulfur bridge. The tenn "alkylthioalkyl" represents an alkylthio group attached through an alkyl or substituted alkyl group as defined above having the indicated number of carbon atoms.

The term "alkylamino" (e.g. methylamino, diethylamino, butylamino, N-propyl-N-hexylamino, (2-cyclopentyl)propylamino, hexenylamino, and the like) represents one or two substituted or unsubstituted alkyl groups as defined above having the indicated number of carbon atoms attached through an amine bridge. The substituted or unsubstituted alkyl groups maybe taken together with the nitrogen to which they are attached forming a saturated cyclic or unsaturated cyclic system containing 3 to 10 carbon atoms with at least one substituent as defined above. The term "alkylaminoalkyl" represents an alkylamino group attached through a substituted or unsubstituted alkyl group as defined above having the indicated number of carbon atoms.

The tenn "alkylhydrazino" (e.g. methylhydrazino, diethylhydrazino, butylhydrazino, (2-cyclopentyl)propylhydrazino, cyclohexanehydrazino, and the like) represents one or two substituted or unsubstituted alkyl groups as defined above having the indicated number of carbon atoms attached through a nitrogen atom of a hydrazine bridge. The substituted or unsubstituted alkyl groups maybe taken together with the nitrogen to which they are attached forming a saturated cyclic or unsaturated cyclic system containing 3 to 10 carbon atoms with at least one substituent as defined above. The term "alkylhydrazinoalkyl" represents an alkylhydrazino group attached through a substituted or unsubstituted alkyl group as defined above having the indicated number of carbon atoms.

The term "alkylcarbonyl" (e.g. cyclooctylcarbonyl, pentylcarbonyl, 3-hexenylcarbonyl and the like) represents a substituted or unsubstituted alkyl group as defined above having the indicated number of carbon atoms attached through a carbonyl group. The term "alkylcarbonylalkyl" represents an alkylcarbonyl group attached through a substituted or unsubstituted alkyl group as defined above having the indicated number of carbon atoms.

The term "alkylcarboxy" (e.g. heptylcarboxy, cyclopropylcarboxy, 3-pentenylcarboxy and the like) represents an alkylcarbonyl group as defined above wherein the carbonyl is in turn attached through an oxygen. The tenn "alkylcarboxyalkyl" represents an alkylcarboxy group attached through an alkyl group as defined above having the indicated number of carbon atoms.

The tenn "alkylcarbonylamino" (e.g. hexylcarbonylamino, cyclopentylcarbonyl-aminomethyl, methylcarbonylaminophenyl and the like) represents an alkylcarbonyl group as defined above wherein the carbonyl is in turn attached through the nitrogen atom of an amino group. The nitrogen group may itself be substituted with a substituted or unsubstituted alkyl or aryl group. The tenn "alkylcarbonylaminoalkyl" represents an alkylcarbonylamino group attached through a substituted or unsubstituted alkyl group as defined above having the indicated number of carbon atoms.

The tenn "alkylcarbonylhydrazino" (e.g. ethylcarbonylhydrazino, tert-butylcarbonylhydrazino and the like) represents an alkylcarbonyl group as defined above wherein the carbonyl is in turn attached through the nitrogen atom of a hydrazino group.

The tenn "aryl" represents an unsubstituted, mono-, or polysubstituted monocyclic, polycyclic, biaryl aromatic groups covalently attached at any ring position capable of forming a stable covalent bond, certain preferred points of attachment being apparent to those skilled in the art (e.g., 3-phenyl, 4-naphthyl and the like). The aryl substituents are independently selected from the group consisting of hydrogen, deuterium, halogen, -OH, -SH, -CN, -NO₂, trihalomethyl, hydroxypyronyl, C₁₋₁₀alkyl, arylC₀₋₁₀alkyl, C₀₋₁₀alkyloxyC₀₋₁₀alkyl, arylC₀₋₁₀alkyloxyC₀₋₁₀alkyl, C₀₋₁₀alkylthioC₀₋₁₀alkyl, arylC₀₋₁₀alkylthioC₀₋₁₀alkyl, C₀₋₁₀alkylaminoC₀₋₁₀alkyl, arylC₀₋₁₀alkylaminoC₀₋₁₀alkyl, N-aryl-N-C₀₋₁₀alkylaminoC₀₋₁₀oalkyl, C₁₋₁₀alkylcarbonylC₀₋₁₀alkyl, arylC₀₋₁₀alkylcarbonylC₀₋₁₀alkyl, C₁₋₁₀alkylcarboxyC₀₋₁₀alkyl, arylC₀₋₁₀alkylcarboxyC₀₋₁₀alkyl, C₁₋₁₀alkylcarbonylaminoC₀₋₁₀alkyl, arylC₀₋₁₀alkylcarbonylaminoC₀₋₁₀alkyl, -C₀₋₁₀alkylCOOR₈₀, and -C₀₋₁₀alkylCONR₈₂R₈₂ wherein R₈₀, R₈₁ and R₈₂ are independently selected from the group consisting of hydrogen, deuterium, alkyl, aryl or R₈₁ and R₈₂ are taken together with the nitrogen to which they are attached forming a saturated cyclic or unsaturated cyclic system containing 3 tao 8 carbon atoms with at least one substituent as defined above.

The definition of "aryl" includes but is not limited to phenyl, pentadeuterophenyl, biphenyl, naphthyl, dihydronaphthyl, tetrahydronaphthyl, indenyl, indanyl, azulenyl, anthryl, phenanthryl, fluorenyl, pyrenyl and the like.

The tenn "arylalkyl" (e.g. ('4-hydroxyphenyl)ethyl, (2-aminonaphthyl)hexenyl and the like) represents an aryl group as defined above attached through a substituted or unsubstituted alkyl group as defined above having the indicated number of carbon atoms.

The tenn "arylcarbonyl" (e.g. 2-thiophenylcarbonyl, 3-methoxyanthrylcarbonyl and the like) represents an aryl group as defined above attached through a carbonyl group.

The term "arylalkylcarbonyl" (e.g. (2,3-dimethoxyphenyl)propylcarbonyl, (2-chloronaphthyl)pentenyl-carbonyl and the like) represents an arylalkyl group as defined above wherein the alkyl group is in turn attached through a carbonyl.

The term "aryloxy" (e.g. phenoxy, naphthoxy, 3-methylphenoxy, and the like) represents an aryl or substituted aryl group as defined above having the indicated number of carbon atoms attached through an oxygen bridge. The term "aryloxyalkyl" represents an aryloxy group attached through a substituted or unsubstituted alkyl group as defined above having the indicated number of carbon atoms.

The term "aryloxycarbonyl" (e.g. phenoxycarbonyl, naphthoxycarbonyl) represents a substituted or unsubstituted aryloxy group as defined above having the indicated number of carbon atoms attached through a carbonyl bridge.

The term "arylthio" (e.g. phenylthio, naphthylthio, 3-bromophenylthio, and the like) represents an aryl or substituted aryl group as defined above having the indicated number of carbon atoms attached through a sulfur bridge. The term "arylthioalkyl" represents an arylthio group attached through a substituted or unsubstituted alkyl group as defined above having the indicated number of carbon atoms.

The term "arylamino" (e.g. phenylamino, diphenylamino, naphthylamino, N-phenyl-N-naphthylamino, o-methylphenylamino, p-methoxyphenylamino, and the like) represents one or two aryl groups as defined above having the indicated number of carbon atoms attached through an amine bridge. The term "arylaminoalkyl" represents an arylamino group attached through a substituted or unsubstituted alkyl group as defined above having the indicated number of carbon atoms. The tenn "arylalkylamino" represents an aryl group attached through an alkylamino group as defined above having the indicated number of carbon atoms. The term "N-aryl-N-alkylamino" (e.g. N-phenyl-N-methylamino, N-naphthyl-N-butylamino, and the like) represents one aryl and one a substituted or unsubstituted alkyl group as defined above having the indicated number of carbon atoms independently attached through an amine bridge.

The tenn "arylhydrazino" (e.g. phenylhydrazino, naphthylhydrazino, 4-methoxyphenylhydrazino; and the like) represents one or two aryl groups as defined above having the indicated number of carbon atoms attached through a hydrazine bridge. The tenn "arylhydrazinoalkyl" represents an arylhydrazino group attached through a substituted or unsubstituted alkyl group as defined above having the indicated number of carbon atoms. The term "arylalkylhydrazino" represents an aryl group attached through an alkylhydrazino group as defined above having the indicated number of carbon atoms. The term "N-aryl-N-alkylhydrazino" (e.g. N-phenyl-N-methylhydrazino, N-naphthyl-N-butylhydrazino, and the like) represents one aryl and one a substituted or unsubstituted alkyl group as defined above having the indicated number of carbon atoms independently attached through an amine atom of a hydrazine bridge.

The tenn "arylcarboxy" (e.g. phenylcarboxy, naphthylcarboxy, 3-fluorophenylcarboxy and the like) represents an arylcarbonyl group as defined above wherein the carbonyl is in turn attached through an oxygen bridge. The term "arylcarboxyalkyl" represents an arylcarboxy group attached through a substituted or unsubstituted alkyl group as defined above having the indicated number of carbon atoms.

The tenn "arylcarbonylamino" (e.g. phenylcarbonylamino, naphthylcarbonylamino, 2-methylphenylcarbonylamino and the like) represents an arylcarbonyl group as defined above wherein the carbonyl is in turn attached through the nitrogen atom of an amino group. The nitrogen group may itself be substituted with a substituted or unsubstituted alkyl or aryl group. The tenn "arylcarbonylaminoalkyl" represents an arylcarbonylamino group attached through a substituted or unsubstituted alkyl group as defined above having the indicated number of carbon atoms. The Nitrogen group may itself be substituted with a substituted or unsubstituted alkyl or aryl group.

The tenn "arylcarbonylhydrazino" (e.g. phenylcarbonylhydrazino, naphthylcarbonylhydrazino, and the like) represents an arylcarbonyl group as defined above wherein the carbonyl is in turn attached through the nitrogen atom of a hydrazino group.

The terms "heteroaryl", "heterocycle" or "heterocyclic" refers to a monovalent unsaturated group having a single ring or multiple condensed rings, from 1 to 13 carbon atoms and from 1 to 10 hetero atoms selected from the group consisting of nitrogen, sulfur, and oxygen, within the ring. The heteroaryl groups in this invention can be optionally substituted with 1 to 10 substituents selected from the group consisting of: hydrogen, deuterium, halogen, -OH, -SH, -CN, -NO₂, trihalomethyl, hydroxypyronyl, C₁₋₁₀alkyl, arylC₀₋₁₀oalkyl, C₀₋₁₀alkyloxyC₀₋₁₀alkyl, arylC₀₋₁₀alkyloxyC₀₋₁₀alkyl, C₀₋₁₀alkylthioC₀₋₁₀alkyl, arylC₀₋₁₀alkylthioC₀₋₁₀alkyl, C₀₋₁₀alkylaminoC₀₋₁₀alkyl, arylC₀₋₁₀alkylaminoC₀₋₁₀alkyl, N-aryl-N -C₀₋₁₀alkylaminoC₀₋₁₀alkyl, C₁₋₁₀alkylcarbonylC₀₋₁₀alkyl, arylC₀₋₁₀alkylcarbonylC₀₋₁₀alkyl, C₁₋₁₀alkylcarboxyC₀₋₁₀alkyl, arylC₀₋₁₀alkylearboxyC₀₋₁₀alkyl, C₁₋₁₀alkylcarbonylaminoC₀₋₁₀alkyl, arylC₀₋₁₀alkylcarbonylaminoC₀₋₁₀alkyl, -C₀₋₁₀alkylCOOR₈₁, and -C₀₋₁₀oalkylCONR₈₂R₈₃ wherein R₈₁, R₈₂ and R₈₃ are independently selected from the group consisting of hydrogen, deuterium, alkyl, aryl, or R₈₂ and R₈₃ are taken together with the nitrogen to which they are attached forming a saturated cyclic or unsaturated cyclic system containing 3 to 8 carbon atoms with at least one substituent as defined above.

The definition of "heteroaryl" includes but is not limited to thienyl, benztithienyl, isobenzothienyl, 2,3-dihydrobenzothienyl, furyl, pyranyl, benzofuranyl, isobenzofuranyl, 2,3-dihydrobenzofuranyl, pyrrolyl, pyrrolyl-2,5-dione, 3-pyrrolinyl, indolyl, isoindolyl, 3H-indolyl, indolinyl, indolizinyl, indazolyl, phthalimidyl (or isoindoly-1,3-dione), imidazolyl, 2H-imidazolinyl, benzimidazolyl, deuterobenzimidazolyl, dideuterobenzimidazolyl, trideuterobenzimidazolyl, tetradeuterobenzimidazolyl, pyridyl, deuteropyridyl, dideuteropyridyl, trideuteropyridyl, tetradeuteropyridyl, pyrazinyl, pyradazinyl, pyrimidinyl, triazinyl, quinolyl, isoquinolyl, 4H-quinolizinyl, cinnolinyl, phthalazinyl, quinazolinyl, quinoxalinyl, 1,8-naphthyridinyl, pteridinyl, carbazolyl, acridinyl, phenazinyl, phenothiazinyl, phenoxazinyl, chromanyl, benzodioxolyl, piperonyl, purinyl, pyrazolyl, triazolyl, tetrazolyl, thiazolyl, isothiazolyl, benzthiazolyl, oxazolyl, isoxazolyl, benzoxazolyl, oxadiazolyl, thiadiazolyl, pyrrolidinyl-2,5₋dione, imidazolidinyl-2,4-dione, 2-thioxo-imidazolidinyl-4-one, imidazolidinyl-2,4-dithione, thiazolidinyl-2,4-dione, 4-thioxo-thiazolidinyl-2-one, piperazinyl-2,5-dione, tetrahydro-pyridazinyl-3,6-dione, 1,2-dihydro-[1,2,4,5]tetrazinyl-3,6-dione, [1,2,4,5]tetrazinanyl-3,6-dione, dihydro-pyrimidinyl-2,4-dione, pyrimidinyl-2,4,6-trione, 1H-pyrimidinyl-2,4-dione, 5-iodo-1H-pyrimidinyl-2,4-dione, 5-chloro-1H-pyrimidinyl-2,4-dione, 5-methyl-1H-pyrimidinyl-2,4-dione, 5-isopropyl-1H-pyrimidinyl-2,4-dione, 5-propynyl-1H-pyrimidinyl-2,4-dione, 5-trifluoromethyl-1H-pyrimidinyl-2,4-dione, 6-amino-9H-purinyl, 2-amino-9H-purinyl, 4-amino-1H-pyrimidinyl-2-one, 4-amino-5-fluoro-1H-pyrimidinyl-2-one, 4-amino-5-methyl-1H-pyrimidinyl-2-one, 2-amino-1,9-dihydro-purinyl-6-one, 1,9-dihydro-purinyl-6-one, 1H-[1,2,4]triazolyl-3-carboxylic acid amide, 2,6-diamino-N6-cyclopropyl-9H-purinyl, 2-amino-6-(4-methoxyphenylsulfanyl)-9H-purinyl, 5,6-dichloro-1H-benzoimidazolyl, 2-isopropylamino-5,6-dichloro-1H-benzoimidazolyl, 2-bromo-5,6-dichloro-1H-benzoimidazolyl, 5-methoxy-1H-benzoimidazolyl, 3-ethylpyridyl, 5-methyl-2-phenyl-oxazolyl, 5-methyl-2-thiophen-2-yl-oxazolyl, 2-furan-2-yl-5-methyl-oxazolyl, 3-methyl-3H-quinazolin-4-one, 4-methyl-2H-phthalazin-1-one, 2-ethyl-6-methyl-3H-pyrimidin-4-one, 5-methoxy-3-methyl-3H-imidazo[4,5-b]pyridine and the like. For the purposes of this application, the terms "heteroaryl", "heterocycle" or "heterocyclic" do not include carbohydrate rings (i.e. mono- or oligosaccharides).

The tenn "saturated heterocyclic" represents an unsubstituted, mono-, and polysubstituted monocyclic, polycyclic saturated heterocyclic group covalently attached at any ring position capable of forming a stable covalent bond, certain preferred points of attachment being apparent to those skilled in the art (e.g., 1-piperidinyl, 4-piperazinyl, DBU, and the like).

The saturated heterocyclic substituents are independently selected from the group consisting of halo, -OH, -SH, -CN, -NO₂, trihalomethyl, hydroxypyronyl, C₁₋₁₀alkyl, arylC₀₋₁₀alkyl, C₀₋₁₀alkyloxyC₀₋₁₀alkyl, arylC₀₋₁₀alkyloxyC₀₋₁₀alkyl, C₀₋₁₀alkylthioC₀₋₁₀alkyl, arylC₀₋₁₀alkylthioC₀₋₁₀alkyl, C₀₋₁₀alkylaminoC₀₋₁₀alkyl, aryl_{C0-10}alkylaminoC₀₋₁₀alky N-aryl-N-C₀₋₁₀alkylaminoC₀₋₁₀alkyl, C₁₋₁₀alkylcarbonylC₀₋₁₀alkyl, arylC₀₋₁₀alkylcarbonylC₀₋₁₀alkyl, C₁₋₁₀alkylcarboxyC₀₋₁₀alkyl, arylC₀₋₁₀alkylcarboxyC₀₋₁₀alkyl, C₁₋₁₀alkylcarbonylaminoC₀₋₁₀alkyl, arylC₀₋₁₀alkylcarbonylaminoC₀₋₁₀alkyl, -C₀₋₁₀alkylCOOR₈₁, and -C₀₋₁₀alkylCONR₈₂₋R₈₃ wherein R₈₁, R₈₂ and R₈₃ are independently selected from the group consisting of hydrogen, deuterium, alkyl, aryl, or R₈₂ and R₈₃ are taken together with the nitrogen to which they are attached forming a saturated cyclic or unsaturated cyclic system containing 3 to 8 carbon atoms with at least one substituent as defined above.

The definition of saturated heterocyclic includes but is not limited to pyrrolidinyl, pyrazolidinyl, piperidinyl, 1,4-dioxanyl, morpholinyl, 1,4-dithienyl, thiomorpholinyl, piperazinyl, quinuclidinyl, and the like.

The term "alpha-beta-unsaturated carbonyl" refers to a molecule that has a carbonyl goup directly attached to a double or triple bonded carbon and which would be obvious to one of ordinary skill and knowledge in the art. The definition of alpha-beta-unsaturated carbonyl includes but is not limited to acrolein, methyl vinyl ketone, and the like.

The term "acetal" refers to a molecule that contains a carbon atom C₁ that is directly attached to a hydrogen atom (H₁), a substituted carbon atom (C₂) and two oxygen atoms (O₁ and O₂). These oxygen atoms are in turn attached to other substituted carbon atoms (C₃ and C₄), which would be obvious to one of ordinary skill and knowledge in the art. The definition of acetal includes but is not limited to 1,1-dimethoxypropane, 1,1-bis-allyloxybutane and the like.

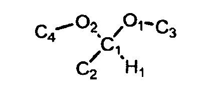

The term "cyclic acetal" refers to an acetal as defined above where C3 and C4, together with the oxygen atoms to which they are attached, combine thru an alkyl bridge to form a 5- to 10-membered ring, which would be obvious to one of ordinary skill and knowledge in the art. The definition of cycli41.5c acetal includes but is not limited to 2-methyl-[1,3]dioxolane, 2-ethyl-[1,3]dioxane, 2-phenyl-[1,3]dioxane, 2-phenyl-hexahydro-pyrano[3,2-d][1,3]dioxine and the like.

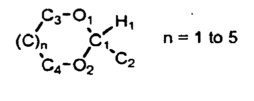

The term "ketal" refers to a molecule that contains a carbon atom C₁ that is directly attached to two substituted, carbon atom (C₂ and C₃) and two oxygen atoms (O₁ and O₂). These oxygen atoms are in turn attached to other substituted carbon atoms (C₄ and C₅), which would be obvious to one of ordinary skill and knowledge in the art. The definition of acetal includes but is not limited to 2,2-dimethoxy-butane, 3,3-diethoxy-pentane and the like.

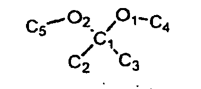

The tenn "cyclic ketal" refers to a ketal as defined above where C₄ and C₅, together with the oxygen atoms to which they are attached, combine thru an alkyl bridge to form a 5- to 10-membered ring, which would be obvious to one of ordinary skill and knowledge in the art. The definition of cyclic acetal includes but is not limited to 2,2,4,5-tetramethyl-[1,3]dioxolane, 2,2-diethyl-[1,3]dioxepane, 2,2-dimethyl-hexahydro-pyrano[3,2-d][1,3]dioxine and the like.

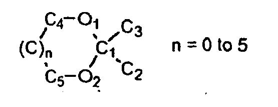

A "C-carboxy" goup refers to a -C(=O)OR groups where R is as defined herein.

An "acetyl" goup refers to a -C(=O)CH₃, group.

A "trihalomethanesulfonyl" group refers to a X₃CS(=O)₂- group where X is a halogen.

A "cyano" goup refers to a -CN group.

An "isocyanato" group refers to a -NCO group.

A "thiocyanato" group refers to a -CNS group.

An "isothiocyanato" group refers to a -NCS group.

A "sulfinyl" group refers to a -S(=O)-R group, with R as defined herein.

A "S-sulfonamido" group refers to a -S(=O)₂NR, group, with R as defined herein.

A "N-sulfonamido" group refers to a RS(=O)₂NH- group with R as defined herein.

A "trihalomethanesulfonamido" group refers to a X₃CS(=O)₂NR- group with X and R as defined herein.

An "O-carbamyl" group refers to a -OC(=O)-NR, group-with R as defined herein.

An "N-carbamyl" group refers to a ROC(=O)NH- group, with R as defined herein.

An "O-thiocarbamyl" group refers to a -OC(=S)-NR, group with R as defined herein.

An "N-thiocarbamyl" group refers to an ROC(=S)NH- group, with R as defined herein.

A "C-amido" group refers to a -C(=O)-NR₂ group with R as defined herein.

An "N-amido" group refers to a RC(=O)NH- group, with R as defined herein.

The tenn "perhaloalkyl" refer to an alkyl group where all of the hydrogen atoms are replaced by halogen atoms.

In light of the purposes described in the present disclosure, all references to "alkyl" and "aryl" groups or any groups ordinarily containing C-H bonds may include partially or fully deuterated versions as required to affect the improvements outlined herein.

### Deuterium Kinetic Isotope Effect

In an attempt to eliminate foreign substances, such as therapeutic agents, from its circulation system, the animal body expresses various enzymes, such as the cytochrome P₄₅₀ enzymes or CYPs, esterases, proteases, reductases, dehydrogenases, and monoamine oxidases, to react with and convert these foreign substances to more polar intermediates or metabolites for renal excretion. Some of the most common metabolic reactions of pharmaceutical compounds involve the oxidation of a carbon-hydrogen (C-H) bond to either a carbon-oxygen (C-O) or carbon-carbon (C-C) π-bond. The resultant metabolites may be stable or unstable under physiological conditions, and can have substantially different pharmacokinetic, pharmacodynamic, and acute and long-term toxicity profiles relative to the parent compounds. For most drugs, such oxidations are generally rapid and ultimately lead to administration of multiple or high daily doses.

The relationship between the activation energy and the rate of reaction may be quantified by the Arrhenius equation, k = Ae^{-E3ct/RT}, where E_{act} is the activation energy, T is temperature, R is the molar gas constant, k is the rate constant for the reaction, and A (the frequency factor) is a constant specific to each reaction that depends on the probability that the molecules will collide with the correct orientation. The Arrhenius equation states that the fraction of molecules that have enough energy to overcome an energy barrier, that is, those with energy at least equal to the activation energy, depends exponentially on the ratio of the activation energy to thennal energy (RT), the average amount of thermal energy that molecules possess at a certain temperature.

The transition state in a reaction is a short lived state (on the order of 10⁻¹⁴ sec) along the reaction pathway during which the original bonds have stretched to their limit. By definition, the activation energy E_{act} for a reaction is the energy required to reach the transition state of that reaction. Reactions that involve multiple steps will necessarily have a number of transition states, and in these instances, the activation energy for the reaction is equal to the energy difference between the reactants and the most unstable transition state. Once the transition state is reached, the molecules can either revert, thus reforming the original reactants, or new bonds form giving rise to the products. This dichotomy is possible because both pathways, forward and reverse, result in the release of energy. A catalyst facilitates a reaction process by lowering the activation energy leading to a transition state. Enzymes are examples of biological catalysts that reduce the energy necessary to achieve a particular transition state.

A carbon-hydrogen bond is by nature a covalent chemical bond. Such a bond forms when two atoms of similar electronegativity share some of their valence electrons, thereby creating a force that holds the atoms together. This force or bond strength can be quantified and is expressed in units of energy, and as such, covalent bonds between various atoms can be classified according to how much energy must be applied to the bond in order to break the bond or separate the two atoms.

The bond strength is directly proportional to the absolute value of the ground-state vibrational energy of the bond. This vibrational energy, which is also known as the zero-point vibrational energy, depends on the mass of the atoms that form the bond. The absolute value of the zero-point vibrational energy increases as the mass of one or both of the atoms making the bond increases. Since deuterium (D) has twice the mass of hydrogen (H), it follows that a C-D bond is stronger than the corresponding C-H bond. Compounds with C-D bonds are frequently indefinitely stable in H₂O, and have been widely used for isotopic studies. If a C-H bond is broken during a rate-determining step in a chemical reaction (i.e. the step with the highest transition state energy), then substituting a deuterium for that hydrogen will cause a decrease in the reaction rate and the process will slow down. This phenomenon is known as the Deuterium Kinetic Isotope Effect (DKIE). The magnitude of the DKIE can be expressed as the ratio between the rates of a given reaction in which a C-H bond is broken, and the same reaction where deuterium is substituted for hydrogen. The DKIE can range from about 1 (no isotope effect) to very large numbers, such as 50 or more, meaning that the reaction can be fifty, or more, times slower when deuterium is substituted for hydrogen. High DKIE values may be due in part to a phenomenon known as tunneling, which is a consequence of the uncertainty principle. Tunneling is ascribed to the small mass of a hydrogen atom, and occurs because transition states involving a proton can sometimes form in the absence of the required activation energy. Because deuterium has more mass than hydrogen, it statistically has a much lower probability of undergoing this phenomenon. Substitution of tritium for hydrogen results in yet a stronger bond than deuterium and gives numerically larger isotope effects

Discovered in 1932 by Urey, deuterium (D) is a stable and non-radioactive isotope of hydrogen. It was the first isotope to be separated from its element in pure form and has twice the mass of hydrogen, and makes up about 0.02% of the total mass of hydrogen (in this usage meaning all hydrogen isotopes) on earth. When two deuterium atoms bond with one oxygen, deuterium oxide (D₂O or "heavy water") is formed. D₂O looks and tastes like H₂O, but has different physical properties. It boils at 101.41°C and freezes at 3.79 °C. Its heat capacity, heat of fusion, heat of vaporization, and entropy are all higher than H₂O. It is more viscous and has different solubilizng properties than H₂O.

When pure D₂O is given to rodents, it is readily absorbed and reaches an equilibrium level that is usually about eighty percent of the concentration of what was consumed. The quantity of deuterium required to induce toxicity is extremely high. When 0% to as much as 15% of the body water has been replaced by D₂O, animals are healthy but are unable to gain weight as fast as the control (untreated) group. When about 15% to about 20% of the body water has been replaced with D₂O, the animals become excitable. When about 20% to about 25% of the body water has been replaced with D₂O, the animals are so excitable that they go into frequent convulsions when stimulated. Skin lesions, ulcers on the paws and muzzles, and necrosis of the tails appear. The animals also become very aggressive; males becoming almost unmanageable. When about 30%, of the body water has been replaced with D₂O, the animals refuse to eat and become comatose. Their body weight drops sharply and their metabolic rates drop far below nonnal, with death occurring at about 30 to about 35% replacement with D₂O. The effects are reversible unless more than thirty percent of the previous body weight has been lost due to D₂O. Studies have also shown that the use of D₂O can delay the growth of cancer cells and enhance the cytotoxicity of certain antineoplastic agents.

Tritium (T) is a radioactive isotope of hydrogen, used in research, fusion reactors, neutron generators and radiophannaceuticals. Mixing tritium with a phosphor provides a continuous light source, a technique that is commonly used in wristwatches, compasses, rifle sights and exit signs. It was discovered by Rutherford, Oliphant and Harteck in 1934, and is produced naturally in the upper atmosphere when cosmic rays react with H₂ molecules. Tritium is a hydrogen atom that has 2 neutrons in the nucleus and has an atomic weight close to 3. It occurs naturally in the environnent in very low concentrations, most commonly found as T₂O, a colorless and odorless liquid. Tritium decays slowly (half-life = 12.3 years) and emits a low energy beta particle that cannot penetrate the outer layer of human skin. Internal exposure is the main hazard associated with this isotope, yet it must be ingested in large amounts to pose a significant health risk. As compared with deuterium, a lesser amount of tritium must be consumed before it reaches a hazardous levels.

Deuteration of phannaceuticals to improve pharmacokinetics (PK), pharmacodynamics (PD), and toxicity profiles, has been demonstrated previously with some classes of drugs. For example, the DKIE was used to decrease the hepatotoxicity of halothane by presumably limiting the production of reactive species such as trifluoroacetyl chloride. However, this method may not be applicable to all drug classes. For example, deuterium incorporation can lead to metabolic switching. The concept of metabolic switching asserts that xenogens; when sequestered by Phase I enzymes, may bind transiently and re-bind in a variety of conformations prior to the chemical reaction (e.g., oxidation). This hypothesis is supported by the relatively vast size of binding pockets in many Phase I enzymes and the promiscuous nature of many metabolic reactions. Metabolic switching can potentially lead to different proportions of known metabolites as well as altogether new metabolites. This new metabolic profile may impart more or less toxicity. Such pitfalls are non-obvious and are not predictable *a priori* for any drug class.

### Deuterated Substituted Indole Derivatives

Sumatriptan is a substituted indole-based 5-HT_{1B} and 5-HT_{1D} receptor modulator. The carbon-hydrogen bonds of Suinatriptan contain a naturally occurring distribution of hydrogen isotopes, namely ¹H or protium (about 99.9844%), ²H or deuterium (about 0.0156%), and ³H or tritium (in the range between about 0.5 and 67 tritium atoms per 10¹⁸ protium atoms). Increase levels of deuterium incorporation may produce a detectable Kinetic Isotope Effect (KIE) that could affect the pharmacokinetic, phannacologic and/or toxicologic profiles of such 5-hydioxytryptamine receptor modulators in comparison with the compound having naturally occurring levels of deuterium.

Aspects of the present invention disclosed herein describe a novel approach to designing and synthesizing new analogs of these 5-hydroxytryptamine receptor modulating agents through chemical modifications and derivations of the carbon-hydrogen bonds of the modulators and/or of the chemical precursors used to synthesize said modulators. Suitable modifications of certain carbon-hydrogen bonds into carbon-deuterium bonds may generate novel 5-hydroxytryptamine receptor modulating agents with unexpected and non-obvious improvements of pharmacotogical, pharmacokinetic and toxicological properties in comparison to the non-isotopically enriched 5-hydroxytryptamine receptor modulating agents. This invention relies on the judicious and successful application of chemical kinetics to drug design. Deuterium incorporation levels in the compounds of the invention are significantly higher than the naturally-occurring levels and are sufficient to induce at least one substantial improvement as described herein.

Based on discoveries made in our laboratory, as well as considering the KIE literature, Sumatriptan is likely metabolized by monoamine oxidase A, in humans, at the N,N-dimethylaminoethyl C-H bonds. The deuteration approach has strong potential to slow the metabolism through MAO_{A}. This shunts the clearance through more universal pathways thus giving rise to more predictable ADMET responses throughout the dose range (which would also be lower via this invention). The toxicity and pharmacology of the resultant indoleacetic acid-based metabolites are not known in detail but are reported to lack affinity for the target receptors 5-HT_{1B} and 5-HT_{1D}. Furthermore, because an MAO_{A}, is responsible for much of the metabolism of sumatriptan, and because MAO_{A} is also responsible for oxidation of many endogenous and exogenous substances, the prevention of such interactions decreases interpatient variability, decreases drug-drug interactions, increases T_{1/2}, decreases the necessary Cₘₐₓ, and improves several other ADMET paraineters. Various deuteration patterns can be used to a) reduce or eliminate unwanted metabolites, b) increase the half-life of the parent drug, c) decrease the number of doses needed to achieve a desired effect, d) decrease the amount of a dose needed to achieve a desired effect, e) increase the formation of active metabolites, if any are formed, and/or f) decrease the production of deleterious metabolites in specific tissues and/or create a more effective drug and/or a safer drug for polypharmacy, whether the polyphannacy be intentional or not. The deuterated analogs of this invention have the potential to uniquely maintain the beneficial aspects of the non-isotopically enriched drugs while substantially increasing the half-life (T_{1/2}), lowering the maximum plasma concentration (Cₘₐₓ) of the minimum efficacious dose (MED), lowering the efficacious dose and thus decreasing the non-mechanism-related toxicity, and/or lowering the probability of drug-drug interactions. These drugs also have strong potential to reduce the cost-of-goods (COG) owing to the ready availability of inexpensive sources of deuterated reagents combined with previously mentioned potential for lowering the therapeutic dose.

In certain embodiments, the compounds disclosed herein may also contain less prevalent isotopes for other elements, including, but not limited to, ¹³C or ¹⁴C for carbon, ³³S ³⁴S, or ³⁶S for sulfur, ¹⁵N for nitrogen, and ¹⁷O or ¹⁸O for oxygen.

In certain embodiments, without being bound by any theory, the compounds disclosed herein may expose a patient to a maximum of about 0.000005% D₂O or about 0.00001% DHO, assuming that all of the C-D bonds in the compounds disclosed herein are metabolized and released as D₂O or DHO. This quantity is a small fraction of the naturally occurring background levels of D₂O or DHO in circulation. In certain embodiments, the levels of D₂O shown to cause toxicity in animals is much greater than even the maximum limit of exposure because of the deuterium enriched compounds as disclosed herein. Thus, in certain embodiments, the deuterium-enriched compounds disclosed herein should not cause any additional toxicity because of the use of deuterium.

In certain embodiments, the deuterated compounds disclosed herein maintain the beneficial aspects of the corresponding non-isotopically enriched molecules while substantially increasing the maximum tolerated dose, decreasing toxicity, increasing the half-life (T_{1/2}), lowering the maximum plasma concentration (Cₘₐₓ) of the minimum efficacious dose (MED), lowering the efficacious dose and thus decreasing the non-mechanism-related toxicity, and/or lowering the probability of drug-drug interactions.

Isotopic hydrogen can be introduced into a compound disclosed herein by synthetic techniques that employ deuterated reagents, whereby incorporation rates are pre-determined; and/or by exchange techniques, wherein incorporation rates are determined by equilibrium conditions, and may be highly variable depending on the reaction conditions. Synthetic techniques, where tritium or deuterium is directly and specifically inserted by tritiated or deuterated reagents of known isotopic content, may yield high tritium or deuterium abundance, but can be limited by the chemistry required. Exchange techniques, on the other hand, may yield lower tritium or deuterium incorporation, often with the isotope being distributed over many sites on the molecule.

The compounds disclosed herein can be prepared by methods known in the art and routine modifications thereof, and/or following procedures similar to those described in the Example section herein and routine modifications thereof, and/or procedures found in the references cited therein and routine modifications thereof. Compounds disclosed herein can also be prepared as shown in any of the following schemes and routine modifications thereof.

For example, certain compounds disclosed herein can be prepared as shown in Scheme 1.

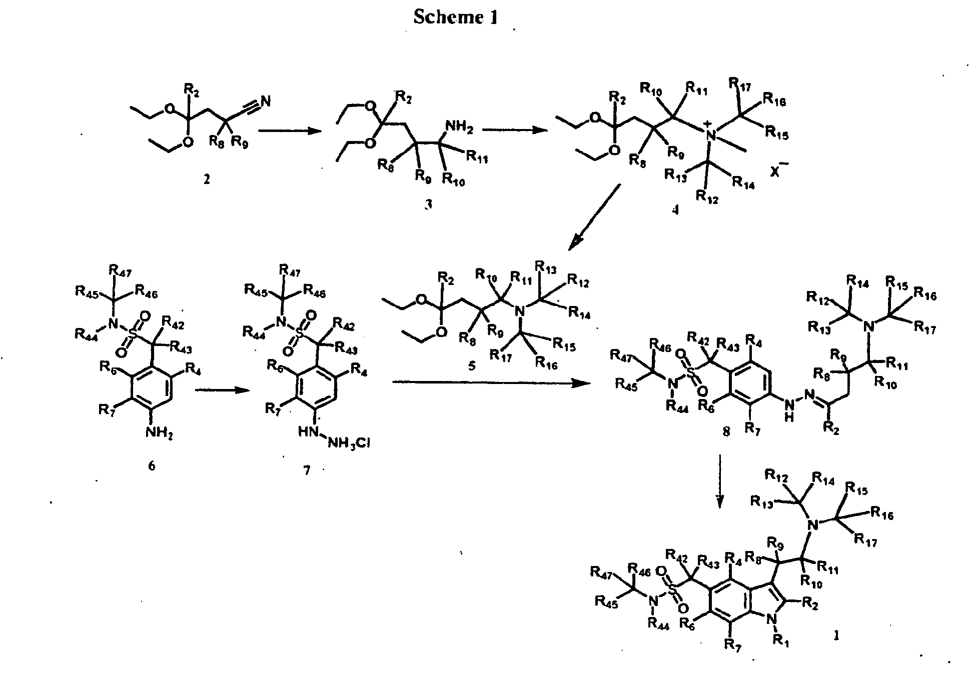

Nitrile **2** is reacted at an elevated temperature with a reducing agent, such as lithium aluminum hydride, in an appropriate solvent, such as tetrahydrofuran, to give primary amine **3,** which is reacted at an elevated temperature with an alkylating agent, such as methyl iodide or methyl methanesulfonate, to give quaternary ammonium salt **4,** wherein X⁻ is a leaving group anion, such as halogen or alkylsulfonate. Compound **4** is reacted at an elevated temperature with an aminoalcohol, such as 2-aminoethanol, to give tertiary amine **5**.

Aniline **6** is reacted with a diazonium compound, such as sodium nitrite, in the presence of a mineral acid, such as hydrochloric acid. The resulting mixture is then reacted with a reducing agent, such as tin chloride, to yield hydrazine **7.** Compound 7 is reacted with tertiary amine 5 in the presence of an acid, such as hydrochloric acid, in an appropriate solvent, such as water, to yield hydrazone **8.** Compound **8** is reacted with an acid, such as ethyl polyphosphate, in an appropriate solvent, such as chloroform, to generate compound **1**.

Certain compounds disclosed herein can be prepared as shown in Scheme 2.

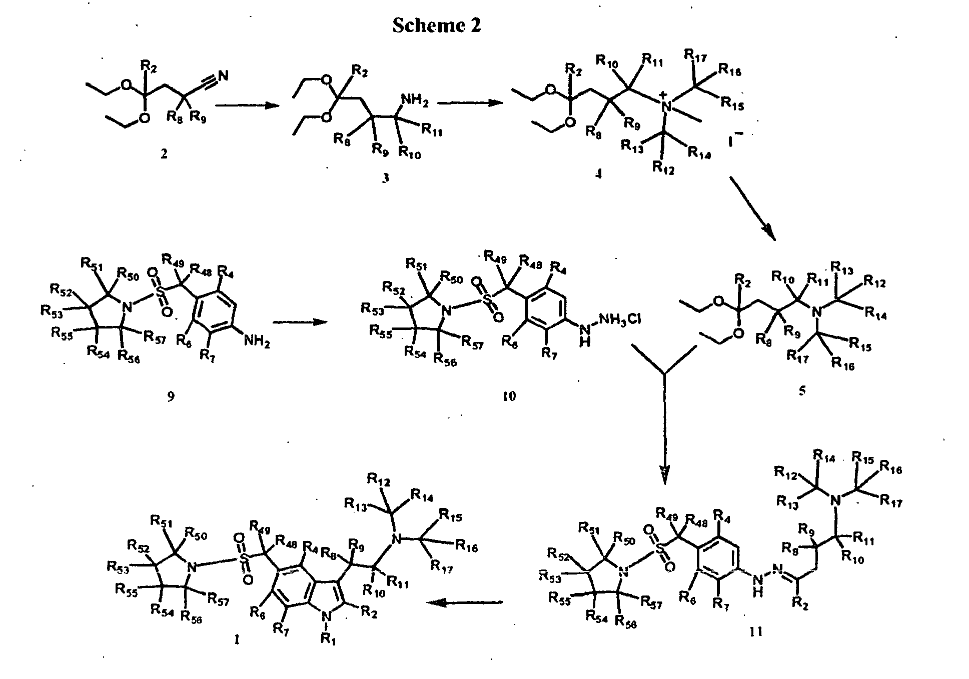

Nitrile **2** is reacted at an elevated temperature with a reducing agent, such as lithium aluminum hydride, in an appropriate solvent, such as tetrahydrofuran, to give primary amine **3,** which is reacted at an elevated temperature with an alkylating agent, such as methyl iodide or methyl methanesulfonate, to give quaternary ammonium salt **4,** wherein X⁻ is a leaving group anion, such as halogen or alkylsulfonate. Compound **4** is reacted at an elevated temperature with an aminoalcohol, such as 2-aminoethanol, to give tertiary amine **5.**

Aniline **9** is reacted with a diazonium compound, such as sodium nitrite, in the presence of a mineral acid, such as hydrochloric acid. The resulting mixture is then reacted with a reducing agent, such as tin chloride, to yield hydrazine **10.** Compound **10** is reacted with tertiary amine **5** in the presence of an acid, such as hydrochloric acid, in an appropriate solvent, such as water, to yield hydrazone **11.** Compound **11** is reacted with an acid, such as ethyl polyphosphate, in an appropriate solvent, such as chloroform, to generate compound **1.**

Deuterium can be incorporated to different positions synthetically, according to the synthetic procedures as shown in Schemes 1 and 2, by using appropriate deuterated intermediates. For example, to introduce deuterium at one or more positions of R₂, R₈, and R₉, 4,4-diethoxy-butyronitrile with the corresponding deuterium substitutions can be used. To introduce deuterium at positions R₁₀ and R₁₁, lithium aluminum deuteride can be used. To introduce deuterium at positions R₁₂, R₁₃, R₁₄, R₁₅, R₁₆, and R₁₇, methyl iodide or methyl methanesulfonate with the corresponding deuterium substitutions can be used. To introduce deuterium at positions R₄, R₆, and R₇, an aniline with the corresponding deuterium substitutions can be used. To introduce deuterium at positions R₄₅, R₄₆, and R₄₇, methylamine with the corresponding deuterium substitutions can be used. To introduce deuterium at positions R₅₀, R₅₁, R₅₂, R₅₃, R₅₄, R₅₅, R₅₆, and R₅₇, pyrrolidine with the corresponding deuterium substitutions can be used. These deuterated intermediates are either commercially available, or can be prepared by methods known to one of skill in the art or following procedures similar to those described in the Example section herein and routine modifications thereof.

Deuterium can also be incorporated to various positions having an exchangeable proton, such as the amine N-H, the indole N-H, and the protons alpha to the sulfonamide group, via proton-deuterium equilibrium exchange. For example, to introduce deuterium at R₁, R₄₂, R₄₃, R₄₄, R₄₈, and R₄₉, these protons may be replaced with deuterium selectively or non-selectively through a proton-deuterium exchange method known in the art.

Unless otherwise indicated, when a substituent is deemed to be "optionally substituted," it is meant that the substituent is a group that may be substituted with one or more group(s) individually and independently selected from the group consisting of hydrogen, deuterium, alkyl, cycloalkyl, aryl, heteroaryl, heterocyclic, hydroxy, alkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, S-sulfonamido, N-sulfonamido. C-carboxy, O-carboxy, isocyanato, thiocyanato, isothiocyanato, nitro, silyl, trihalomethanesulfonyl, and amino, including mono- and di-substituted amino groups, and the protected derivatives thereof. The protecting groups that may form the protective derivatives of the above substituents are known to those of skill in the art examples of which may be found in references such as Greene and Wuts, Projective Groups in Organic Synthesis, 3rd Ed., John Wiley & Sons, New York, NY, 1999, which is incorporated by reference herein in its entirety.

The compounds according to this invention may occur as any reasonable tautomer as recognized by one skilled in the art or a mixture of such tautomers. The term "tautomer" or "tautomerism" refers to one of two or more structural isomers that exist in equilibrium and are readily converted from one isomeric form to another. Examples include keto-enol tautomers, such as acetone/propen-2-ol and the like, ring-chain tautomers, such as glucose/ 2,3,4,5,6-pentahydroxy-hexanal and the like. The compounds described herein may have one or more tautomers and therefore include various isomers. All such isomeric forms of these compounds are expressly included in the present invention.

It is to be understood that the compounds disclosed herein may contain one or more chiral centers, chiral axes, and/or chiral planes, as described in "Stereochemistry of Carbon Compounds" Eliel and Wilen, John Wiley & Sons, New York, 1994, pp. 119-1190. Such chiral centers, chiral axes, and chiral planes may be of either the (R) or (S) configuration, or may be a mixture thereof.

A method for characterizing a composition containing a compound having at least one chiral center is by the effect of the composition on a beam of polarized light. When a beam of plane polarized light is passed through a solution of a chiral compound, the plane of polarization of the light that emerges is rotated relative to the original plane. This phenomenon is known as optical activity, and compounds that rotate the plane of polarized light are said to be optically active. One enantiomer of a compound will rotate the beam of polarized light in one direction, and the other enantiomer will rotate the beam of light in the opposite direction. The enantiomer that rotates the polarized light in the clockwise direction is the (+) enantiomer and the enantiomer that rotates the polarized light in the counterclockwise direction is the (-) enantiomer. Included within the scope of the compositions described herein are compositions containing between 0 and 100% of the (+) and/or (-) enantiomer of the compounds disclosed herein.

Where a compound of disclosed herein contains an alkenyl or alkenylene group, the compound may exist as one or mixture of geometric *cis*/*trans* (or Z/E) isomers. Where structural isomers are interconvertible *via* a low energy barrier, the compound disclosed herein may exist as a single tautomer or a mixture of tautomers. This can take the form of proton tautomerism in the compound disclosed herein that contains for example, an imino, keto, or oxime group; or so-called valence tautomerism in the compound that contain an aromatic moiety. It follows that a single compound may exhibit more than one type of isomerism.

The compounds disclosed herein may be enantiomerically pure, such as a single enantiomer or a single diastereomer, or be stereoisomeric mixtures, such as a mixture of enantiomers, a racemic mixture, or a diastereomeric mixture. As such, one of skill in the art will recognize that administration of a compound in its (R) form is equivalent, for compounds that undergo epimerization *in vivo*, to administration of the compound in its (S) form. Conventional techniques for the preparation/isolation of individual enantiomers include chiral synthesis from a suitable optically pure precursor or resolution of the racemate using, for example, chiral chromatography, recrystallization, resolution, diastereomeric salt formation, or derivatization into diastereomeric adducts followed by separation.

When the compound disclosed herein contains an acidic or basic moiety, it may also provided as a pharmaceutically acceptable salt (*See,* Berge et al., J. Pharm. Sci. 1977, 66, 1-19; and "Handbook of Pharmaceutical Salts, Properties, and Use," Stah and Wermuth, Ed.; Wiley-VCH and VHCA, Zurich, 2002).

Suitable acids for use in the preparation of phannaceutically acceptable salts include, but are not limited to, acetic acid, 2,2-dichloroacetic acid, acylated amino acids, adipic acid, alginic acid, ascorbic acid, L-aspartic acid, benzenesulfonic acid, benzoic acid, 4-acetainidobenzoic acid, boric acid, (+)-camphoric acid, camphorsulfonic acid, (+)-(1S)-camphor-10-sulfonic acid, capric acid, caproic acid, caprylic acid, cinnamic acid, citric acid, cyclamic acid, cyclohexanesulfamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hippuric acid, hydrobromic acid, hydrochloric acid, hydroiodic acid, (+)-L-lactic acid, (±)-DL-lactic acid, lactobionic acid, lauric acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, perchloric acid, phosphoric acid, L-pyroglutamic acid, saccharic acid, salicylic acid 4-amino-salicylic acid, sebacic acid, stearic acid, succinic acid, sulfuric acid, tannic acid, (+)-L-tartaric acid, thiocyanic acid, p-toluenesulfonic acid, undecylenic acid, and valeric acid.

Suitable bases for use in the preparation of pharmaceutically acceptable salts, including, but not limited to, inorganic bases, such as magnesium hydroxide, calcium hydroxide, potassium hydroxide, zinc hydroxide, or sodium hydroxide; and organic bases, such as primary, secondary, tertiary, and quaternary, aliphatic and aromatic amines, including L-arginine, benethamine, benzathine, choline, deanol, diethanolamine, diethylamine, dimethylamine, dipropylamine, diisopropylamine, 2-(diethylamino)-ethanol, ethanolamine, ethylamine, ethylenediamine, isopropylamine, N-methyl-glucamine, hydrabamine, 1H-imidazole, L-lysine, morpholine, 4-(2-hydroxyethyl)-morpholine, methylamine, piperidine, piperazine, propylamine, pyrrolidine, 1-(2-hydroxyethyl)-pyrrolidine, pyridine, quinuclidine, quinoline, isoquinoline, secondary amines, triethanolamine, trimethylamine, triethylamine, N-methyl-D-glucamine, 2-amino-2-(hydroxymethyl)-1,3-propanediol, and tromethamine.

The compounds disclosed herein may also be provided as a prodrug, which is a functional derivative of the parent compound and is readily convertible into the parent compound *in vivo*. Prodrugs are often useful because, in some situations, they may be easier to administer than the parent compound. They may, for instance, be bioavailable by oral administration whereas the parent compound is not. The prodrug may also have enhanced solubility in pharmaceutical compositions over the parent compound. A prodrug may be converted into the parent drug by various mechanisms, including enzymatic processes and metabolic hydrolysis. See Harper, Progress in Drug Research 1962, 4, 221-294; Morozowich et al. in "Design of Biophannaceutical Properties through Prodrugs and Analogs," Roche Ed., APHA Acad. Phann. Sci. 1977; "Bioreversible Carriers in Drug in Drug Design, Theory and Application," Roche Ed., APHA Acad. Phann. Sci. 1987; "Design of Prodrugs," Bundgaard, Elsevier, 1985; Wang et al., Curr. Pharm. Design 1999, 5, 265-287; Pauletti et al., Adv. Drug. Delivery Rev. 1997, 27, 235-256; Mizen et al., Pharm. Biotech. 1998, 11, 345-365; Gaignault et al., Pract. Med. Chem. 1996, 671-696; Asghamejad in "Transport Processes in Pharmaceutical Systems," Amidon et al., Ed., Marcell Dekker, 185-218, 2000; Balant et al., Eur .J. Drug Metab. Pharmacokinet. 1990, 15, 143-53; Balimane and Sinko, Adv. Drug Delivery Rev. 1999, 39, 183-209; Browne, Clin. Neuropharmacol. 1997, 20, 1-12; Bundgaard, Arch. Pharm. Chem. 1979, 86, 1-39; Bundgaard, Controlled Drug Delivery 1987, 17, 179-96; Bundgaard, Adv. Drug Delivery Rev. 1992, 8, 1-38; Fleisher et al., Adv. Drug Delivery Rev. 1996, 19, 115-130; Fleisher et al., Methods Enzymol. 1985, 112, 360-381; Farquhar et al., J. Pharm. Sci. 1983, 72, 324-325; Freeman et al., J. Chem. Soc., Chem. Commun. 1991, 875-877; Friis and Bundgaard, Eur. J. Pharm. Sci. 1996, 4, 49-59; Gangwar et al., Des. Biopharm. Prop. Prodrugs Analogs, 1977, 409-421; Nathwani and Wood, Drugs 1993, 45, 866-94; Sinhababu and Thakker, Adv. Drug Delivery Rev. 1996, 19, 241-273, Stella et al., Drugs 1985, 29, 455-73; Tan et al., Adv. Drug Delivery Rev. 1999, 39, 117-151; Taylor, Adv. Drug Delivery Rev. 1996, 19, 131-148; Valentino and Borchardt, Drug Discovery Today 1997, 2, 148-155; Wiebe and Knaus, Adv. Drug Delivery Rev. 1999, 39, 63-80; Waller et al., Br. J. Clin. Pharmac. 1989, 28, 497-507.

### Pharmaceutical Composition

Disclosed herein are pharmaceutical compositions comprising a compound disclosed herein as an active ingredient or a pharmaceutically acceptable salt, solvate, or prodrug thereof, in a pharmaceutically acceptable vehicle, carrier, diluent, or excipient, or a mixture thereof; in combination with one or more phannaceutically acceptable excipients or carriers.

In another embodiment of the invention, there are provided pharmaceutical compositions comprising at least one of the compounds disclosed herein, or a pharmaceutically acceptable salt, solvate, or prodrug thereof, in a pharmaceutically acceptable vehicle, carrier, diluent, or excipient, or a combination thereof, for enteral, intravenous infusion, oral, parenteral, intranasal, topical or ocular administration.

In yet another embodiment of the invention, there are provided pharmaceutical compositions comprising at least one of the compounds disclosed hereinor a pharmaceutically acceptable salt, solvate, or prodrug thereof, in a pharmaceutically acceptable vehicle, carrier, diluent, or excipient, or a combination thereof, for the treatment of conditions mediated by 5-hydroxytryptamine 1B and/or 1D receptor modulation.

In another embodiment of the invention, there are provided methods of modulating 5-hytrmytryptamine 1B and/or 1D receptor activity in the central nervous system, with one or more of the compounds or compositions disclosed herein, or a pharmaceutically acceptable salt, solvate, or prodrug thereof.

Further provided herein are pharmaceutical compositions in enteric coated dosage forms, which comprise a compound disclosed herein, or a mixture of diastereomers thereof; or a pharmaceutically acceptable salt, solvate, or prodrug thereof; and one or more release controlling excipients or carriers for use in an enteric coated dosage form. The pharmaceutical compositions may also comprise non-release controlling excipients or carriers.

Provided herein are pharmaceutical compositions in modified release dosage forms, which comprise a compound disclosed herein, or a pharmaceutically acceptable salt, solvate, or prodrug thereof; and one or more release controlling excipients or carriers as described herein. Suitable modified release dosage vehicles include, but are not limited to, hydrophilic or hydrophobic matrix devices, water-soluble separating layer coatings, enteric coatings, osmotic devices, multiparticulate devices, and combinations thereof. The pharmaceutical compositions may also comprise non-release controlling excipients or carriers.

Further provided herein are pharmaceutical compositions in effervescent dosage forms, which comprise a compound disclosed herein, or a phannaceutically acceptable salt, solvate, or prodrug thereof; and one or more release controlling excipients or carriers for use in an enteric coated dosage form. The pharmaceutical compositions may also comprise non-release controlling excipients or carriers.

Additionally provided are phannaceutical compositions in a dosage form that has an instant releasing component and at least one delayed releasing component, and is capable of giving a discontinuous release of the compound in the form of at least two consecutive pulses separated in time from 0.1 up to 24 hours. The pharmaceutical compositions comprise a compound disclosed herein, or a pharmaceutically acceptable salt, solvate, or prodrug thereof; and one or more release controlling and non-release controlling excipients or carriers, such as those excipients or carriers suitable for a disruptable semi-permeable membrane and as swellable substances.

Provided herein also are pharmaceutical compositions in a dosage form for oral administration to a subject, which comprise a compound disclosed herein, or a pharmaceutically acceptable salt, solvate, or prodrug thereof; and one or more pharmaceutically acceptable excipients or carriers, enclosed in an intennediate reactive layer comprising a gastric juice-resistant polymeric layered material partially neutralized with alkali and having cation exchange capacity and a gastric juice-resistant outer layer.

Provided herein are phannaceutical compositions that comprise about 0.1 to about 1000 mg, about 1 to about 500 mg, about 2 to about 100 mg, about 1 mg, about 2 mg, about 3 mg, about 5 mg, about 10 mg, about 20 mg, about 30 mg, about 40 mg, about 50 mg, about 100 mg, about 300mg, about 500 mg, about 1000 mg of one or more compounds disclosed herein.

The pharmaceutical compositions provided herein may be provided in unit-dosage forms or multiple-dosage forms. Unit-dosage forms, as used herein, refer to physically discrete units suitable for administration to human and animal subjects and packaged individually as is known in the art. Each unit-dose contains a predetermined quantity of the active ingredient(s) sufficient to produce the desired therapeutic effect, in association with the required pharmaceutical carriers or excipients. Examples of unit-dosage forms include ampoules, syringes, and individually packaged tablets and capsules. Unit-dosage forms may be administered in fractions or multiples thereof. A multiple-dosage form is a plurality of identical unit-dosage forms packaged in a single container to be administered in segregated unit-dosage form. Examples of multiple-dosage forms include vials, bottles of tablets or capsules, or bottles of pints or gallons.

The compounds disclosed herein may be administered alone, or in combination with one or more other compounds disclosed herein, one or more other active ingredients. The pharmaceutical compositions that comprise a compound disclosed herein may be formulated in various dosage forms for oral, parenteral, and topical administration. The pharmaceutical compositions may also be formulated as a modified release dosage form, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art (*see.* Remington: The Science and Practice of Pharmacy, supra; Modified-Release Drug Deliver Technology, Rathbone et al., Eds., Drugs and the Pharmaceutical Science, Marcel Dekker, Inc.: New York, NY, 2002; Vol. 126).

The pharmaceutical compositions disclosed herein may be administered at once, or multiple times at intervals of time. It is understood that the precise dosage and duration of treatment may vary with the age, weight, and condition of the patient being treated, and may be determined empirically using known testing protocols or by extrapolation from *in vivo* or *in vitro* test or diagnostic data. It is further understood that for any particular individual, specific dosage regimens should be adjusted over time according to the individual need and the professional judgment of the person administering or supervising the administration of the formulations.

In the case wherein the patient's condition does not improve, upon the doctor's discretion the administration of the compounds may be administered chronically, that is, for an extended period of time, including throughout the duration of the patient's life in order to ameliorate or otherwise control or limit the symptoms of the patient's disease or condition.

In the case wherein the patient's status does improve, upon the doctor's discretion the administration of the compounds may be given continuously or temporarily suspended for a certain length of time (*i.e*., a "drug holiday").

Once improvement of the patient's conditions has occurred, a maintenance dose is administered if necessary. Subsequently, the dosage or the frequency of administration, or both, can be reduce, as a function of the symptoms; to a level at which the improved disease, disorder or condition is retained. Patients can, however, require intermittent treatment on a long-term basis upon any recurrence of symptoms.

### A. Oral Administration

The pharmaceutical compositions disclosed herein may be provided in solid, semisolid, or liquid dosage forms for oral administration. As used herein, oral administration also include buccal, lingual, and sublingual administration. Suitable oral dosage forms include, but are not limited to, tablets, capsules, pills, troches, lozenges, pastilles, cachets, pellets, medicated chewing gum, granules, bulk powders, effervescent or non-effervescent powders or granules, solutions, emulsions, suspensions, solutions, wafers, sprinkles, elixirs, and syrups. In addition to the active ingredient(s), the pharmaceutical compositions may contain one or more pharmaceutically acceptable carriers or excipients, including, but not limited to, binders, fillers, diluents, disintegrants, wetting agents, lubricants, glidants, coloring agents, dye-migration inhibitors, sweetening agents, and flavoring agents.

Binders or granulators impart cohesiveness to a tablet to ensure the tablet remaining intact after compression. Suitable binders or granulators include, but are not limited to, starches, such as corn starch, potato starch, and pre-gelatinized starch (e.g., STARCH 1500); gelatin; sugars, such as sucrose, glucose, dextrose, molasses, and lactose; natural and synthetic gums, such as acacia, alginic acid, alginates, extract of Irish moss, Panwar gum, ghatti gum, mucilage of isabgol husks, carboxymethylcellulose, methylcellulose, polyvinylpyrrolidone (PVP), Veegum, larch arabogalactan, powdered tragacanth, and guar gum; celluloses, such as ethyl cellulose, cellulose acetate, carboxymethyl cellulose calcium, sodium carboxymethyl cellulose, methyl cellulose, hydroxyethylcellulose (HEC), hydroxypropylcellulose (HPC), hydroxypropyl methyl cellulose (HPMC); microcrystalline celluloses, such as AVICEL-PH-101, AVICEL-PH-103, AVICEL RC-581, AVICEL-PH-105 (FMC Corp., Marcus Hook, PA); and mixtures thereof. Suitable fillers include, but are not limited to, talc, calcium carbonate, microcrystalline cellulose, powdered cellulose, dextrates, kaolin, mannitol, silicic acid, sorbitol, starch, pre-gelatinized starch, and mixtures thereof. The binder or filler may be present from about 50 to about 99% by weight in the phannaceutical compositions disclosed herein.

Suitable diluents include, but are not limited to, dicalcium phosphate, calcium sulfate, lactose, sorbitol, sucrose, inositol, cellulose, kaolin, mannitol, sodium chloride, dry starch, and powdered sugar. Certain diluents, such as mannitol, lactose, sorbitol, sucrose, and inositol, when present in sufficient quantity, can impart properties to some compressed tablets that pennit disintegration in the mouth by chewing. Such compressed tablets can be used as chewable tablets.

Suitable disintegrants include, but are not limited to, agar; bentonite; celluloses, such as methylcellulose and carboxymethylcellulose; wood products; natural sponge; cation-exchange resins; alginic acid; gums, such as guar gum and Veegum HV; citrus pulp; cross-linked celluloses, such as croscarmellose; cross-linked polymers, such as crospovidone; cross-linked starches; calcium carbonate; microcrystalline cellulose, such as sodium starch glycolate; polacrilin potassium; starches, such as corn starch, potato starch, tapioca starch, and pre-gelatinized starch; clays; aligns; and mixtures thereof. The amount of disintegrant in the phannaceutical compositions disclosed herein varies upon the type of formulation, and is readily discernible to those of ordinary skill in the art. The pharmaceutical compositions disclosed herein may contain from about 0.5 to about 15% or from about 1 to about 5% by weight of a disintegrant.

Suitable lubricants include, but are not limited to, calcium stearate; magnesium stearate; mineral oil; light mineral oil; glycerin; sorbitol, mannitol; glycols, such as glycerol behenate and polyethylene glycol (PEG); stearic acid; sodium lauryl sulfate; talc; hydrogenated vegetable oil, including peanut oil, cottonseed oil, sunflower oil, sesame oil, olive oil, corn oil, and soybean oil; zinc stearate; ethyl oleate; ethyl laureate; agar; starch; lycopodium; silica or silica gels, such as AEROSIL^{®} 200 (W.R. Grace Co., Baltimore, MD) and CAB-O-SIL^{®} (Cabot Co. of Boston, MA); and mixtures thereof. The pharmaceutical compositions disclosed herein may contain about 0.1 to about 5% by weight of a lubricant.

Suitable glidants include colloidal silicon dioxide, CAB-O-SIL^{®} (Cabot Co. of Boston, MA), and asbestos-free talc. Coloring agents include any of the approved, certified, water soluble FD&C dyes, and water insoluble FD&C dyes suspended on alumina hydrate, and color lakes and mixtures thereof. A color lake is the combination by adsorption of a water-soluble dye to a hydrous oxide of a heavy metal, resulting in an insoluble form of the dye. Flavoring agents include natural flavors extracted from plants, such as fruits, and synthetic blends of compounds which produce a pleasant taste sensation, such as peppermint and methyl salicylate. Sweetening agents include sucrose, lactose, mannitol, syrups, glycerin, and artificial sweeteners, such as saccharin and aspartame. Suitable emulsifying agents include gelatin, acacia, tragacanth, bentonite, and surfactants, such as polyoxyethylene sorbitan monooleate (TWEEN^{®} 20), polyoxyethylene sorbitan monooleate 80 (TWEEN^{®} 80), and triethanolamine oleate. Suspending and dispersing agents include sodium carboxymethylcellulose, pectin, tragacanth, Veegum, acacia, sodium carbomethylcellulose, hydroxypropyl methylcellulose, and polyvinylpyrolidone. Preservatives include glycerin, methyl and propylparaben, benzoic add, sodium benzoate and alcohol. Wetting agents include propylene glycol monostearate, sorbitan monooleate, diethylene glycol monolaurate, and polyoxyethylene lauryl ether. Solvents include glycerin, sorbitol, ethyl alcohol, and syrup. Examples of non-aqueous liquids utilized in emulsions include mineral oil and cottonseed oil. Organic acids include citric and tartaric acid. Sources of carbon dioxide include sodium bicarbonate and sodium carbonate.

It should be understood that many carriers and excipients may serve several functions, even within the same formulation.

The pharmaceutical compositions disclosed herein may be provided as compressed tablets, tablet triturates, chewable lozenges, rapidly dissolving tablets, multiple compressed tablets, or enteric-coating tablets, sugar-coated, or film-coated tablets. Enteric-coated tablets are compressed tablets coated with substances that resist the action of stomach acid but dissolve or disintegrate in the intestine, thus protecting the active ingredients from the acidic environment of the stomach. Enteric-coatings include, but are not limited to, fatty acids, fats, phenylsalicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms may be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions disclosed herein may be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganism. Suitable preservatives are those as described herein, including methyl- and propyl-parabens, and sorbic acid. The liquid, semisolid, and solid dosage forms discloses herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions disclosed herein may be provided in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable non-aqueous liquids or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde (the tenn "lower" means an alkyl having between 1 and 6 carbon atoms), e.g., acetaldehyde diethyl acetal; and a water-miscible solvent having one or more hydroxyl groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, *e.g*., water, to be measured conveniently for administration.

Other useful liquid and semisolid dosage forms include, but are not limited to, those containing the active ingredient(s) disclosed herein, and a dialkylated mono- or poly-alkylene glycol, including, 1,2-dimethoxymethane, diglyme, triglyme, tetraglyme, polyethylene glycol-350-dimethyl ether, polyethylene glycol-550-dimethyl ether, polyethylene glycol-750-dimethyl ether, wherein 350, 550, and 750 refer to the approximate average molecular weight of the polyethylene glycol. These formulations may further comprise one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium metabisulfite, thiodipropionic acid and its esters, and dithiocarbamates.

The pharmaceutical compositions disclosed herein for oral administration may be also provided in the forms of liposomes, micelles, microspheres, or nanosystems. Micellar dosage forms can be prepared as described in U.S. Pat. No. 6,350,458.

The pharmaceutical compositions disclosed herein may be provided as non-effervescent or effervescent, granules and powders, to be reconstituted into a liquid dosage form. Pharmaceutically acceptable carriers and excipients used in the non-effervescent granules or powders may include diluents, sweeteners, and wetting agents. Pharmaceutically acceptable carriers and excipients used in the effervescent granules or powders may include organic acids and a source of carbon dioxide.

Coloring and flavoring agents can be used in all of the above dosage forms.

The pharmaceutical compositions disclosed herein may be formulated as immediate or modified release dosage forms, including delayed-, sustained, pulsed-, controlled, targeted-, and programmed-release forms.

The pharmaceutical compositions disclosed herein may be co-fonnulated with other active ingredients which do not impair the desired therapeutic action, or with substances that supplement the desired action, such as drotrecogin-α, and hydrocortisone. B. Parenteral Administration

The pharmaceutical compositions disclosed herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

The pharmaceutical compositions disclosed herein may be formulated in any dosage forms that are suitable for parenteral, administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (*see.* Remington: The Science and Practice of Pharmacy*,* supra).

The pharmaceutical compositions intended for parenteral, administration may include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, corn oil, cottonseed oil, olive oil, peanut oil, peppennint oil, safflower oil, sesame oil, soybean oil, hydrogenated vegetable oils, hydrogenated soybean oil, and medium-chain triglycerides of coconut oil, and palm seed oil. Water-miscible vehicles include, but are not limited to, ethanol, 1,3-butanediol, liquid polyethylene glycol (e.g., polyethylene glycol 300 and polyethylene glycol 400), propylene glycol, glycerin, *N*-methyl-2-pyrrolidone, dimethylacetamide, and dimethylsulfoxide.

Suitable antimicrobial agents or preservatives include, but are not limited to, phenols, cresols, mercurials, benzyl alcohol, chlorobutanol, methyl and propyl p-hydroxybenzates, thimerosal, benzalkonium chloride, benzethonium chloride, methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents include those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including α-cyclodextrin, β-cyclodextrin, hydroxypropyl-β-cyclodextrin, sulfobutylether-β-cyclodextrin, and sulfobutylether 7-β-cyclodextrin (CAPTISOL^{®}, CyDex, Lenexa, KS).

The pharmaceutical compositions disclosed herein may be formulated for single or multiple dosage administration. The single dosage fonnulations are packaged in an ampule, a vial, or a syringe. The multiple dosage parenteral formulations must contain an antimicrobial agent at bacteriostatic or fungistatic concentrations. All parenteral formulations must be sterile, as known and practiced in the art.

In one embodiment, the pharmaceutical compositions are provided as ready-to-use sterile solutions. In another embodiment, the phannaceutical compositions are provided as sterile dry soluble products, including lyophilized powders and hypodermic tablets, to be reconstituted with a vehicle prior to use. In yet another embodiment, the phannaceutical compositions are provided as ready-to-use sterile suspensions. In yet another embodiment, the pharmaceutical compositions are provided as sterile dry insoluble products to be reconstituted with a vehicle prior to use. In still another embodiment, the pharmaceutical compositions are provided as ready-to-use sterile emulsions.

The pharmaceutical compositions disclosed herein may be formulated as immediate or modified release dosage forms, including delayed-, sustained, pulsed-, controlled, targeted-, and programmed-release forms.

The phannaceutical compositions may be formulated as a suspension, solid, semi-solid, or thixotropic liquid, for administration as an implanted depot. In one embodiment, the phannaceutical compositions disclosed herein are dispersed in a solid inner matrix, which is surrounded by an outer polymeric membrane that is insoluble in body fluids but allows the active ingredient in the pharmaceutical compositions diffuse through.

Suitable inner matrixes include polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers, such as hydrogels of esters of acrylic and methacrylic acid, collagen, cross-linked polyvinylalcohol, and cross-linked partially hydrolyzed polyvinyl acetate.

Suitable outer polymeric membranes include polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

### C. Topical Administration

The pharmaceutical compositions disclosed herein may be administered topically to the skin, orifices, or mucosa. The topical administration, as used herein, include (intra)dermal, conjuctival, intracorneal, intraocular, ophthalmic, auricular, transdermal, nasal, vaginal, uretheral, respiratory, and rectal administration.

The pharmaceutical compositions disclosed herein may be formulated in any dosage forms that are suitable for topical administration for local or systemic effect, including emulsions, solutions, suspensions, creams, gels, hydrogels, ointments, dusting powders, dressings, elixirs, lotions, suspensions, tinctures, pastes, foams, films, aerosols, irrigation, sprays, suppositories, bandages, dermal patches. The topical formulation of the pharmaceutical compositions disclosed herein may also comprise liposomes, micelles, microspheres, nanosystems, and mixtures thereof.

Pharmaceutically acceptable carriers and excipients suitable for use in the topical formulations disclosed herein include, but are not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryopretectants, lyoprotectants, thickening agents, and inert gases.

The pharmaceutical compositions may also be administered topically by electroporation, iontophoresis, phonophoresis, sonophoresis and microneedle or needle-free injection, such as POWDERJECT™ (Chiron Corp., Emeryville, CA), and BIOJECT™ (Bioject Medical Technologies Inc., Tualatin, OR).

The pharmaceutical compositions disclosed herein may be provided in the forms of ointments, creams, and gels. Suitable ointment vehicles include oleaginous or hydrocarbon vehicles, including, such as lard, benzoinated lard, olive oil, cottonseed oil, and other oils, white petrolatum; emulsifiable or absorption vehicles, such as hydrophilic petrolatum, hydroxystearin sulfate, and anhydrous lanolin; water-removable vehicles, such as hydrophilic ointment; water-soluble ointment vehicles, including polyethylene glycols of varying molecular weight; emulsion vehicles, either water-in-oil (W/O) emulsions or oil-in-water (O/W) emulsions, including cetyl alcohol, glyceryl monostearate, lanolin, and stearic acid (*see,* Remington: The Science and Practice of Pharmacy*,* supra). These vehicles are emollient but generally require addition of antioxidants and preservatives.

Suitable cream base can be oil-in-water or water-in-oil. Cream vehicles may be water-washable, and contain an oil phase, an emulsifier, and an aqueous phase. The oil phase is also called the "internal" phase, which is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation may be a nonionic, anionic, cationic, or amphoteric surfactant.

Gels are semisolid, suspension-type systems. Single-phase gels contain organic macromolecules distributed substantially uniformly throughout the liquid carrier. Suitable gelling agents include crosslinked acrylic acid polymers, such as carbomers, carboxypolyalkylenes, Carbopol®; hydrophilic polymers, such as polyethylene oxides, polyoxyethylene-polyoxypropylene copolymers, and polyvinylalcohol; cellulosic polymers, such as hydroxypropyl cellulose, hydroxyethyl cellulose, hydroxypropyl methylcellulose, hydroxypropyl methylcellulose phthalate, and methylcellulose; gums, such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

The pharmaceutical compositions disclosed herein may be administered rectally, urethrally, vaginally, or perivaginally in the fonns of suppositories, pessaries, bougies, poultices or cataplasm, pastes, powders, dressings, creams, plasters, contraceptives, ointments, solutions, emulsions, suspensions, tampons, gels, foams, sprays, or enemas. These dosage forms can be manufactured using conventional processes as described in Remington: The Science and Practice of Pharmacy*,* supra.

Rectal, urethral, and vaginal suppositories are solid bodies for insertion into body orifices, which are solid at ordinary temperatures but melt or soften at body temperature to release the active ingredient(s) inside the orifices. Pharmaceutically acceptable carriers utilized in rectal and vaginal suppositories include bases or vehicles, such as stiffening agents, which produce a melting point in the proximity of body temperature, when formulated with the pharmaceutical compositions disclosed herein; and antioxidants as described herein, including bisulfite and sodium metabisulfite. Suitable vehicles include, but are not limited to, cocoa butter (theobroma oil), glycerin-gelatin, carbowax (polyoxyethylene glycol), spermaceti, paraffin, white and yellow wax, and appropriate mixtures of mono-, di- and triglycerides of fatty acids, hydrogels, such as polyvinyl alcohol, hydroxyethyl methacrylate, polyacrylic acid; glycerinated gelatin. Combinations of the various vehicles may be used. Rectal and vaginal suppositories may be prepared by the compressed method or molding. The typical weight of a rectal and vaginal suppository is about 2 to about 3 g.

The pharmaceutical compositions disclosed herein may be administered ophthalmically in the forms of solutions, suspensions, ointnents, emulsions, gel-forming solutions, powders for solutions, gels, ocular inserts, and implants.

The pharmaceutical compositions disclosed herein may be administered intranasally or by inhalation to the respiratory tract. The phannaceutical compositions may be provided in the form of an aerosol or solution for delivery using a pressurized container, pump, spray, atomizer, such as an atomizer using electrohydrodynamics to produce a fine mist, or nebulizer, alone or in combination with a suitable propellant, such as 1,1,1,2-tetrafluoroethane or 1,1,1,2,3,3,3-heptafluoropropane. The pharmaceutical compositions may also be provided as a dry powder for insufflation, alone or in combination with an inert carrier such as lactose or phospholipids; and nasal drops. For intranasal use, the powder may comprise a bioadhesive agent, including chitosan or cyclodextrin.

Solutions or suspensions for use in a pressurized container, pump, spray, atomizer, or nebulizer may be formulated to contain ethanol, aqueous ethanol, or a suitable alternative agent for dispersing, solubilizing, or extending release of the active ingredient disclosed herein, a propellant as solvent; and/or an surfactant, such as sorbitan trioleate, oleic acid, or an oligolactic acid.

The pharmaceutical compositions disclosed herein may be micronized to a size suitable for delivery by inhalation, such as about 50 micrometers or less, or about 10 micrometers or less. Particles of such sizes may be prepared using a comminuting method known to those skilled in the art, such as spiral jet milling, fluid bed jet milling, supercritical fluid processing to form nanoparticles, high pressure homogenization, or spray drying.

Capsules, blisters and cartridges for use in an inhaler or insufflator may be formulated to contain a powder mix of the pharmaceutical compositions disclosed herein; a suitable powder base, such as lactose or starch; and a performance modifier, such as /leucine, mannitol, or magnesium stearate. The lactose may be anhydrous or in the form of the monohydrate. Other suitable excipients or carriers include dextran, glucose, maltose, sorbitol, xylitol, fructose, sucrose, and trehalose. The pharmaceutical compositions disclosed herein for inhaled/intranasal administration may further comprise a suitable flavor, such as menthol and levomenthol, or sweeteners, such as saccharin or saccharin sodium.

The pharmaceutical compositions disclosed herein for topical administration may be formulated to be immediate release or modified release, including delayed-, sustained-, pulsed-, controlled-, targeted, and programmed release.

### D. Modified Release

The pharmaceutical compositions disclosed herein may be formulated as a modified release dosage form. As used herein, the term "modified release" refers to a dosage form in which the rate or place of release of the active ingredient(s) is different from that of an immediate dosage fonn when administered by the same route. Modified release dosage forms include delayed-, extended-, prolonged-, sustained-, pulsatile-, controlled-, accelerated- and fast-, targeted-, programmed-release, and gastric retention dosage forms. The pharmaceutical compositions in modified release dosage forms can be prepared using a variety of modified release devices and methods known to those skilled in the art, including, but not limited to, matrix controlled release devices, osmotic controlled release devices, multiparticulate controlled release devices, ion-exchange resins, enteric coatings, multilayered coatings, microspheres, liposomes, and combinations thereof. The release rate of the active ingredient(s) can also be modified by varying the particle sizes and polymorphorism of the active ingredient(s).

Examples of modified release include, but are not limited to, those described in U.S. Pat. Nos.: 3,845,770; 3,916,899; 3,536,809; 3,598,123; 4,008,719; 5,674,533; 5,059,595; 5,591,767; 5,120,548: 5,073,543; 5,639,476; 5,354,556; 5,639,480; 5,733,566; 5,739,108; 5,891,474; 5,922,356; 5,972,891; 5,980,945: 5,993,855; 6,045,830; 6,087,324; 6,113,943; 6,197,350; 6,248,363; 6,264,970; 6,267,981; 6,376,461; 6,4 9,961; 6,589,548; 6,613,358: and 6,699,500.

### 1. Matrix Controlled Release Devices

The pharmaceutical compositions disclosed herein in a modified release dosage form may be fabricated using a matrix controlled release device known to those skilled in the art *(see,* Takada et al in "Encyclopedia of Controlled Drug Delivery," Vol. 2, Mathiowitz ed., Wiley, 1999).

In one embodiment, the pharmaceutical compositions disclosed herein in a modified release dosage form is formulated using an erodible matrix device, which is water-swellable, erodible, or soluble polymers, including synthetic polymers, and naturally occurring polymers and derivatives, such as polysaccharides and proteins.

Materials useful in forming an erodible matrix include, but are not limited to, chitin, chitosan, dextran, and pullulan; gum agar, gum arabic, gum karaya, locust bean gum, gum tragacanth, carrageenans, gum ghatti, guar gum, xanthan gum, and seleroglucan; starches, such as dextrin and maltodextrin; hydrophilic colloids, such as pectin; phosphatides, such as lecithin; alginates; propylene glycol alginate; gelatin; collagen; and cellulosics, such as ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl ethyl cellulose (HPMC), HPMCP, HPMCAS, hydroxypropyl methyl cellulose acetate trimellitate (HPMCAT), and ethylhydroxy ethylcellulose (EHEC); polyvinyl pyrrolidone; polyvinyl alcohol; polyvinyl acetate; glycerol fatty acid esters; polyacrylamide; polyacrylic acid; copolymers of ethacrylic acid or methacrylic acid (EUDRAGIT^{®}, Rohm America, Inc., Piscataway, NJ); poly(2-hydroxyethyl-methacrylate); polylactides; copolymers of L-glutamic acid and ethyl-L-glutamate; degradable lactic acid-glycolic acid copolymers; poly-D-(-)-3-hydroxybutyric acid; and other acrylic acid derivatives, such as homopolymers and copolymers of butylmethacrylate, methylmethacrylate, ethylmethacrylate, ethylacrylate, (2-dimethylaminoethyl)methacrylate, and (trimethylaminoethyl)methacrylate chloride.

In further embodiments, the pharmaceutical compositions are formulated with a non-erodible matrix device. The active ingredient(s) is dissolved or dispersed in an inert matrix and is released primarily by diffusion through the inert matrix once administered. Materials suitable for use as a non-erodible matrix device included, but are not limited to, insoluble plastics, such as polyethylene, polypropylene, polyisoprene, polyisobutylene, polybutadiene, polymethylmethacrylate, polybutylmethacrylate, chlorinated polyethylene, polyvinylchloride, methyl acrylate-methyl methacrylate copolymers, ethylene-vinylacetate copolymers, ethylene/propytene copolymers, ethylene/ethyl acrylate copolymers, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, polyvinyl chloride, plasticized nylon, plasticized polyethyleneterephthalate, natural rubber, silicone rubbers, polydimethylsiloxanes, silicone carbonate copolymers, and ; hydrophilic polymers, such as ethyl cellulose, cellulose acetate, crospovidone, and cross-linked partially hydrolyzed polyvinyl acetate,; and fatty compounds, such as carnauba wax, microcrystalline wax, and triglycerides.

In a matrix controlled release system, the desired release kinetics can be controlled, for example, via the polymer type employed, the polymer viscosity, the particle sizes of the polymer and/or the active ingredient(s), the ratio of the active ingedient(s) versus the polymer, and other excipients or carriers in the compositions.

The pharmaceutical compositions disclosed herein in a modified release dosage form may be prepared by methods known to those skilled in the art, including direct compression, dry or wet granulation followed by compression, melt-granulation followed by compression.

### 2. Osmotic Controlled Release Devices

The pharmaceutical compositions disclosed herein in a modified release dosage form may be fabricated using an osmotic controlled release device, including one-chamber system, two-chamber system, asymmetric membrane technology (AMT), and extruding core system (ECS). In general, such devices have at least two components: (a) the core which contains the active ingredient(s); and (b) a semipermeable membrane with at least one delivery port, which encapsulates the core. The semipermeable membrane controls the influx of water to the core from an aqueous environment of use so as to cause drug release by extrusion through the delivery port(s).

In addition to the active ingredient(s), the core of the osmotic device optionally includes an osmotic agent, which creates a driving force for transport of water from the environment of use into the core of the device. One class of osmotic agents water-swellable hydrophilic polymers, which are also referred to as "osmopolymers" and "hydrogels," including, but not limited to, hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP), crosslinked PVP, polyvinyl alcohol (PVA), PVA/PVP copolymers, PVA/PVP copolymers with hydrophobic monomers such as methyl methacrylate and vinyl acetate, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl, cellulose (CEC), sodium alginate, polycarbophil, gelatin, xanthan gum, and sodium starch glycolate.

The other class of osmotic agents are osmogens, which are capable of imbibing water to affect an osmotic pressure gradient across the barrier of the surrounding coating. Suitable osmogens include, but are not limited to, inorganic salts, such as magnesium sulfate, magnesium chloride, calcium chloride, sodium chloride, lithium chloride, potassium sulfate, potassium phosphates, sodium carbonate, sodium sulfite, lithium sulfate, potassium chloride, and sodium sulfate; sugars, such as dextrose, fructose, glucose, inositol, lactose, maltose, mannitol, raffinose, sorbitol, sucrose, trehalose, and xylitol,; organic acids, such as ascorbic acid, benzoic acid, fumaric acid, citric acid, maleic acid, sebacic acid, sorbic acid, adipic acid, edetic acid, glutamic acid, p-tolunesulfonic acid, succinic acid, and tartaric acid; urea; and mixtures thereof.

Osmotic agents of different dissolution rates may be employed to influence how rapidly the active ingredient(s) is initially delivered from the dosage form. For example, amorphous sugars, such as Mannogeme EZ (SPI Phanna, Lewes, DE) can be used to provide faster delivery during the first couple of hours to promptly produce the desired therapeutic effect, and gradually and continually release of the remaining amount to maintain the desired level of therapeutic or prophylactic effect over an extended period of time. In this case, the active ingredient(s) is released at such a rate to replace the amount of the active ingredient metabolized and excreted.

The core may also include a wide variety of other excipients and carriers as described herein to enhance the performance of the dosage form or to promote stability or processing.

Materials useful in forming the semipermeable membrane include various grades of acrylics, vinyls, ethers, polyamides, polyesters, and cellulosic derivatives that are water-permeable and water-insoluble at physiologically relevant pHs, or are susceptible to being rendered water-insoluble by chemical alteration, such as crosslinking. Examples of suitable polymers useful in forming the coating, include plasticized, unplasticized, and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethylaminoacetate, CA ethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxtated ethylene-vinylacetale, EC, PEG, PPG, PEG/PPG copolymers, PVP, HEC, HPC, CMC, CMEC, HPMC, HPMCP, HPMCAS, HPMCAT, poly(acrylic) acids and esters and poly-(methacrylic) acids and esters and copolymers thereof, starch, dextran, dextrin, chitosan, collagen, gelatin, [polyalkenes, polyethers, polysulfones, polyethersulfones, polystyrenes, polyvinyl halides, polyvinyl esters and ethers, natural waxes, and synthetic waxes.

Semipermeable membrane may also be a hydrophobic microporous membrane, wherein the pores are substantially filled with a gas and are not wetted by the aqueous medium but are permeable to water vapor, as disclosed in U.S. Pat. No. 5,798,119. Such hydrophobic but water-vapor permeable membrane are typically composed of hydrophobic polymers such as polyalkenes, polyethylene, polypropylene, polytetrafluoroethylene, polyacrylic acid derivatives, polyethers, polysulfones, polyethersulfones, polystyrenes, polyvinyl halides, polyvinylidene fluoride, pulyvinyl esters and ethers, natural waxes, and synthetic waxes.

The delivery port(s) on the semipermeable membrane may be fonned post-coating by mechanical or laser drilling. Delivery port(s) may also be fonned in situ by erosion of a plug of water-soluble material or by rupture of a thinner portion of the membrane over an indentation in the core. In addition, delivery ports may be fonned during coating process, as in the case of asymmetric membrane coatings of the type disclosed in U.S. Pat. Nos. 5,612,059 and 5,698,220.

The total amount of the active ingredient(s) released and the release rate can substantially by modulated via the thickness and porosity of the semipermeable membrane, the composition of the core, and the number, size, and position of the delivery ports.

The pharmaceutical compositions in an osmotic controlled-release dosage form may further comprise additional conventional excipients or carriers as described herein to promote performance or processing of the formulation.

The osmotic controlled-release dosage fonns can be prepared according to conventional methods and techniques known to those skilled in the art (*see.* Remington: The Science and Practice of Pharmacy supra; Santus and Baker, J. Controlled Release 1995, 35, 1-21; Venna et al., Drug Development and Industrial Pharmacy 2000, 26, 695-708; Venna et al., J. Controlled Release 2002, 79, 7-27).

In certain embodiments, the pharmaceutical compositions disclosed herein are formulated as AMT controlled-release dosage form, which comprises an asymmetric osmotic membrane that coats a core comprising the active ingredient(s) and other pharmaceutically acceptable excipients or carriers. *See*, U.S. Pat. No. 5,612,059 and WO 2002/17918. The AMT controlled -release dosage forms can be prepared according to conventional methods and techniques known to those skilled in the art, including direct compression, dry granulation, wet granulation, and a dip-coating method.

In certain embodiments, the pharmaceutical compositions disclosed herein are formulated as ESC controlled-release dosage form, which comprises an osmotic membrane that coats a core comprising the active ingredient(s), a hydroxylethyl cellulose, and other pharmaceutically acceptable excipients or carriers.

### 3. Multiparticulate Controlled Release Devices

The pharmaceutical compositions provided herein in a modified release dosage form may be fabricated a multiparticulate controlled release device, which comprises a multiplicity of particles, granules, or pellets, ranging from about 10 µm to about 3 mm, about 50 µm to about 2.5 mm, or from about 100 µm to about 1 mm in diameter. Such multiparticulates may be made by the processes know to those skilled in the art, including wet-and dry-granulation, extrusion/spheronization, roller-compaction, melt-congealing, and by spray-coating seed cores. *See,* for example, Multiparticulate Oral Drug Delivery; Marcel Dekker: 1994; and Pharmaceutical Pelletization Technology; Marcel Dekker: 1989.

Other excipients or carriers as described herein may be blended with the pharmaceutical compositions to aid in processing and forming the multiparticulates. The resulting particles may themselves constitute the multiparticulate device or may be coated by various film-forming materials, such as enteric polymers, water-swellable, and water-soluble polymers. The multiparticulates can be further processed as a capsule or a tablet.

### 5. Targeted Delivery

The pharmaceutical compositions disclosed herein may also be formulated to be targeted to a particular tissue, receptor, or other area of the body of the subject to be treated, including liposome-, reseated erythrocyte-, and antibody-based delivery systems. Examples include, but are not limited to, U.S. Pat. Nos. 6,316,652; 6,274,552; 6,271,359; 6,253,872; 6,139,865; 6,131,570; 6,120,751; 6,071,495; 6,060,082; 6,048,736; 6,039,975; 6,004,534; 5,985,307; 5,972,366; 5,900,252; 5,840,674; 5,759,542; and 5,709,874.

In certain embodiments, tablet formulations for oral administration contain about 0.5 mg to about 300 mg of a pharmaceutically acceptable salt of a compound as disclosed herein, preferably the succinate salt. Certain tablet formulations also contain croscarmellose sodium, dibasic calcium phosphate, magnesium stearate, microcrystalline cellulose, and sodium bicarbonate. Other tablet formualtions also contain hypromellose, iron oxide, titanium dioxide, and triacetin.

In certain embodiments, tablet formulations for oral administration contain about 0.5 mg to about 300 mg of a pharmaceutically acceptable salt of a compound as disclosed herein, preferably the malate salt. Certain tablet formulations also contain mannitol, cellulose, povidone, sodium starch glycolate, sodium stearyl fuinarate, titanium dioxide, hypromellose, polyethylene glycol, propylene glycol, iron oxide, and carnauba wax.

In certain embodiments, tablet formulations similar to those disclosed in U.S. Patents No. 6,368,627 (issued April 9, 2002), 6,020,001 (issued February 1, 2000), and 5,863,559 (issued January 26, 1999) contain a core which contains about 280 mg of granules consisting of equal parts of a compound as disclosed herein as the succinate salt and lactose, about 15.5 mg microcystaline cellulose, about 3.0 mg of croscarmellose sodium, about 1.25 to about 1.75 magnesium stearate, and coated with an aqueous mixture of 10% (w/w) hydroxypropyl methylcellulose and about 5 % (w/w) opaspray white; where the maximum weight of solids applied during coating is 11 mg per tablet, and the tablet core is coated with an aqueous mixture of about 5.3% (w/w) of opadry pink, where the maximum weight of solids applied during coating is 9 mg per tablet.

In certain embodiments, direct compression tablet formulations similar to those disclosed in U.S. Patent No. 4,816,470 (issued March 28, 1989) contain about 10 mg per tablet of a compound as disclosed herein, about 188.5 mg of microcrystalline cellulose, and about 1.5 mg magnesium stearate.

In certain embodiments, wet granulation tablet formulations similar to those disclosed in U.S. Patent No. 4,816,470 (issued March 28, 1989) contain about 10 mg per tablet of a compound as disclosed herein, about 143.5 mg of lactose, about 30 mg of starch, about 15 mg of pregelatinized maize starch, and about 1.5 mg magnesium stearate.

In certain embodiments, intranasal formulations contain about 0.5 mg to about 50 mg of a compound as disclosed herein in about 50 microliter to about 200 microliter unit dose aqueous buffered solution containing monobasic potassium phosphate NF, anhydrous dibasic sodium phosphate USP, sulfuric acid NF, sodium hydroxide NF, and purified water USP, with a pH in the range of about 5 to about 7, and an osmolality in the range of about 300 to about 800 mOsmol.

In certain embodiments, formulations for injection contain about 0.5 mg per milliliter to about 50 mg per milliliter of a compound as disclosed herein as a pharmaceutical acceptable salt, preferably the succinate salt, USP sodium chloride in USP water for injection, with a pH in the range of about 4 to about 7 and an osmolality in the range of about 200 to about 400 mOsmol.

In certain embodiments, formulations for injection contain about 0.5 mg per milliliter to about 50 mg per milliliter of a compound as disclosed herein as a pharmaceutically acceptable salt, preferably the malate salt, USP sodium chloride in USP water for injection, with a pH in the range of about 4 to about 7 and an osmolality in the range of about 200 to about 400 mOsmol.

In certain embodiments, formulations for injection similar to those disclosed in U.S. Patent No. 5,565,447 (issued October 15, 1996) in the form of ampoules contain 10 mg each of a compound as disclosed herein as the hydrocloride salt and can be prepared by dissolving about 200 g of almotriptan hydrochloride and about 200 g of sodium chloride in about 40 L water, passing the resulting solution through a bacteria-retaining filter, and filling the ampoules in a known manner.

In certain embodiments, formulations for injection similar to those disclosed in U.S. Patent No. 5,037,845 (issued August 6, 1991) contain about 0.896 mg/ml of a compound as disclosed herein dissolved in 0.9% (w/v) of aqueous sodium chloride.

Provided are methods for treating, preventing, or ameliorating one or more symptoms of a 5-hydroxytryptamine 1B and/or 1D receptor-mediated disease, disorder or condition comprising administering to a subject having or being suspected to have such a disease, disorder, or condition, a therapeutically effective amount of a compound disclosed herein or a pharmaceutically acceptable salt, solvate, or prodrug thereof.

5-hytroxytryptamine 1B and/or 1D receptor-mediated diseases, disorders, and conditions include, but are not limited to, headaches including migraine headaches, with or without aura, movement disorders, depression, and anxiety and/or any disease, disorder or condition ameliorated by modulating 5-hydroxytryptamine 1B and/or 1D receptors.

Also provided are methods of treating, preventing, or ameliorating one or more symptoms of a disease, disorder or condition associated with 5-hydroxytryptamine 1B and/or 1D receptors, by administering to a subject having or being suspected to have such a disease, disorder, or condition, a therapeutically effective amount of a compound disclosed herein, or a pharmaceutically acceptable salt, solvate, or prodrug thereof.

Further provided are methods of treating, preventing, or ameliorating one or more symptoms of a disease, disorder, or condition responsive to modulation of 5-hydroxytryptamine 1B and/or 1D receptors, comprising administering to a subject having or being suspected to have such a disease, disorder, or condition, a therapeutically effective amount of a compound disclosed herein, or a phannaceutically acceptable salt, solvate, or prodrug thereof.

Furthermore, disclosed herein are methods of modulating the activity of 5-hydroxytryptamine 1B and/or 1D receptor, comprising contacting the receptors with at least one compound disclosed herein, or a pharmaceutically acceptable salt, solvate, or prodrug thereof. In one embodiment, the 5-hydroxytryptamine 1B and/or 1D receptors are expressed by a cell.

Disclosed herein are methods for treating a subject, including a human, having or suspected of having a disease, disorder, or condition, involving, but not limited to, headaches including migraine headaches with or without aura, movement disorders, depression, and anxiety and/or any disease, disorder, or condition ameliorated by modulating 5-hydroxytryptamine 1B and/or 1D receptors, or for preventing such disease, disorder, or condition, in a subject prone to the disease, disorder, or condition; comprising administering to the subject a therapeutically effective amount of a compound disclosed herein, or a pharmaceutically acceptable salt, solvate, or prodrug thereof: so as to affect decreased inter-individual variation in plasma levels of the compound or a metabolite thereof, during the treatment of the disease, disorder or condition as compared to the corresponding non-isotopically enriched compound.

In certain embodiments, the inter-individual variation in plasma levels, of the compounds disclosed herein, or metabolites thereof, is decreased by greater than about 5%, greater than about 10%, greater than about 20%, greater than about 30%, greater than about 40%, or by greater than about 50% as compared to the corresponding non-isotopically enriched compound.

Disclosed herein are methods for treating a subject, including a human, having or suspected of having a disease, disorder or condition involving, but not limited to, headaches including migraine headaches with or without aura, movement disorders, depression, and anxiety and/or any disease, disorder, or condition ameliorated by modulating 5-hydroxytryptamine 1B and/or 1D receptors, or for preventing such disease, disorder, or condition, in a subject prone to the disease, disorder, or condition; comprising administering to the subject a therapeutically effective amount of a compound disclosed herein, or a pharmaceutically acceptable salt, solvate, or prodrug thereof so as to affect increased average plasma levels of the compound or decreased average plasma levels of at least one metabolite of the compound per dosage unit as compared to the corresponding non-isotopically enriched compound.

In certain embodiments, the average plasma levels of the compound disclosed herein are increased by greater than about 5%, greater than about 10%, greater than about 20%, greater than about 30%, greater than about 40%, or greater than about 50% as compared to the corresponding non-isotopically enriched compounds.

In certain embodiments, the average plasma levels of a metabolite of the compound disclosed herein are decreased by greater than about 5%, greater than about 10%, greater than about 20%, greater than about 30%, greater than about 40%, or greater than about 50% as compared to the corresponding non-isotopically enriched compounds

Plasma levels of the compounds disclosed herein, or metabolites thereof, may be measured using the methods described by Li et al. (Rapid Communications in Mass Spectrometry 2005, 19, 1943-1950).

Disclosed herein are methods for treating a subject, including a human, having or suspected of having a disease, disorder, or condition involving, but not limited to, headaches including migraine headaches with or without aura, movement disorders, depression, and anxiety and/or any disease, disorder, or condition ameliorated by modulating 5-hydroxytryptamine) 1B and/or) 1D receptors, or for preventing such disease, disorder, or condition, in a subject prone to the disease, disorder, or condition; comprising administering to the subject a therapeutically effective amount of a compound disclosed herein, or a pharmaceutically acceptable salt, solvate, or prodrug thereof; so as to affect a decreased inhibition of, and/or metabolism by at least one cytochrome P₄₅₀ or monoamine oxidase isoform in the subject during the treatment of the disease as compared to the corresponding non-isotopically enriched compound.

Examples of cytochrome P₄₅₀ isoforms in a mammalian subject include, but are not limited to, CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, and CYP51.

Examples of monoamine oxidase isoforms in a mammalian subject include, but are not limited to, MAO_{A}, and MAO_{B}.

In certain embodiments, the decrease in inhibition of the cytochrome P₄₅₀ or monoamine oxidase isofonn by a compound disclosed herein is greater than about 5%, greater than about 10%, greater than about 20%, greater than about 30%, greater than about 40%, or greater than about 50% as compared to the corresponding non-isotopically enriched compounds.

The inhibition of the cytochrome P₄₅₀ isoform is measured by the method of Ko et al. (British Journal of Clinical Pharmacology, 2000, 49, 343-351). The inhibition of the MAO_{A} isofonn is measured by the method of Weyler et al. (J. Biol Chem. 1985, 260, 13199-13207). The inhibition of the MAO_{B} isoform is measured by the method of Uebelhack et al. (Pharmacopsychiatry, 1998, 31, 187-192*).*

In other embodiments, the decrease in metabolism of compounds of the invention by the cytochrome P₄₅₀ or monoamine oxidase isoform is greater than about 30%, as compared to the non-isotopically enriched compound. In other embodiments, the decrease in metabolism of compounds of the invention by the cytochrome P₄₅₀ or monoamine oxidase isofonn is greater than about 40%, as compared to the non-isotopically enriched compound. In other embodiments, the decrease in metabolism of compounds of the invention by the cytochrome P₄₅₀ or monoamine oxidase isofonn is greater than about 50%, as compared to the non-isotopically enriched compound.

Disclosed herein are methods for treating a subject, including a human, having or suspected of having a disease, disorder, or condition involving, but not limited to, headaches including migraine headaches with or without aura, movement disorders, depression, and anxiety and/or any disease, disorder, or condition ameliorated by modulating 5-hydroxytryptamine 1B and/or 1D receptors, or for preventing such disease, disorder, or condition, in a subject prone to the disease, disorder, or condition; comprising administering to the subject a therapeutically effective amount of a compound disclosed herein, or a phannaceutically acceptable salt, solvate, or prodrug thereof; so as to affect a decreased metabolism via at least one polymorphically-expressed cytochrome P₄₅₀ isoform in the subject during the treatment of the disease as compared to the corresponding non-isotopically enriched compound.

Examples of polymorphically-expressed cytochrome P₄₅₀ isoforms in a mammalian subject include, but are not limited to, CYP2C8, CYP2C9, CYP2C19, and CYP2D6.

In certain embodiments, the decrease in metabolism of the compound disclosed herein by at least one polymorphically-expressed cytochrome P₄₅₀ isoforms cytochrome P₄₅₀ isoform is greater than about 5%, greater than about 10%, greater than about 20%, greater than about 30%, greater than about 40%, or greater than about 50% as compared to the corresponding non-isotopically enriched compound.

The metabolic activities of liver microsomes and the cytochrome P₄₅₀ isoforms are measured by the methods described in Example 6 and 7. The metabolic activities of the monoamine oxidase isoforms are measured by the methods described in Examples 8, 9 and 11.

In another embodiment of the invention, there are provided methods for treating a mammal, particularly a human having, suspected of having, or being prone to a disease, disorder or condition alleviated by modulating 5-hydroxytryptamine 1B and/or 1D receptors, comprising administering to a mammal in need thereof a therapeutically effective amount of a 5-hydroxytryptamine 1B and/or 1D receptor modulator comprising at least one of the compounds disclosed herein, or a pharmaceutically acceptable salt, solvate, or prodrug thereof; provided that the compound disclosed herein contains at least one deuterium atom.

In another embodiment of the invention, there are provided methods for treating a subject, particularly a human having, suspected of having, or being prone to a disease or condition alleviated by 5-hydroxytryptamine 1B and/or 1D receptor modulation, comprising administering to a mammal in need thereof a therapeutically effective amount of a 5-hydroxytryptamine 1B and/or 1D receptor modulator comprising at least one of the compounds disclosed herein, or a pharmaceutically acceptable salt, solvate, or prodrug thereof, so as to cause directly or indirectly, a statistically-significant improvement in a psychological endpoint, as compared to the non-isotopically enriched compound.

Examples of an improved psychological endpoint includes, but is not limited to, a statistically-significant improvement in depression, flashbacks associated with post-traumatic-stress-disorder), as compared to the corresponding non-isotopically enriched compound when given under the same dosing protocol including the same number of doses per day and the same quantity of drug per dose.

Disclosed herein are methods for treating a subject, including a human, having or suspected of having a disease, disorder, or condition involving, but not limited to, headaches including migraine headaches with or without aura, movement disorders, depression, and anxiety and/or any disease, disorder, or condition ameliorated by modulating 5-hydroxytryptamine 1B and/or 1D preceptors, or for preventing such disease, disorder, or condition, in a subject prone to the disease, disorder, or condition; comprising administering to the subject a therapeutically effective amount of a compound disclosed herein or a pharmaceutically acceptable salt, solvate, or prodrug thereof; so as to affect at least one statistically-significantly improved disease-control and/or disease eradication endpoints, as compared to the corresponding non-isotopically enriched compound.

Examples of improved disease-control and/or disease-eradication endpoints include, but are not limited to, statistically-significant improvement in vasoplegia, lactic acidosis, tissue necrosis, prevention of irreversible arterial hypotension, multiple organ dysfunction syndrome, decreased mortality, normalization of heart rate, normalization of body temperature, normalization of blood gases, normalization of white blood cell count, reduction in need for hemodialysis, and/or diminution of toxicity including but not limited to, hepatotoxicity or other toxicity, or a decrease in aberrant liver enzyme levels as measured by standard laboratory protocols, as compared to the corresponding non-isotopically enriched compound.

Disclosed herein are methods for treating a subject, including a human, having or suspected of having a disease, disorder, or condition involving, but not limited to, headaches including migraine headaches with or without aura, movement disorders, depression, and anxiety and/or any disease, disorder, or condition ameliorated by modulating 5-hydroxytryptamine 1B and/or 1D receptors, or for preventing such disease, disorder, or condition, in a subject prone to the disease, disorder, or condition; comprising administering to the subject a therapeutically effective amount of a compound disclosed herein or a pharmaceutically acceptable salt, solvate, or prodrug thereof: so as to affect an improved clinical effect as compared to the corresponding non-isotopically enriched compound.

Disclosed herein are methods for treating a subject, including a human, having or suspected of having a disease, disorder, or condition involving, but not limited to, headaches including migraine headaches with or without aura, movement disorders, depression, and anxiety and/or any disease, disorder, or condition ameliorated by modulating 5-hydroxytryptamine 1B and/or 1D receptors, or for preventing such disease, disorder, or condition, in a subject prone to the disease, disorder, or condition; comprising administering to the subject a therapeutically effective amount of a compound disclosed herein or a pharmaceutically acceptable salt, solvate, or prodrug thereof; so as to affect prevention of recurrence, or delay of decline or appearance, of abnormal alimentary or hepatic parameters as the primary clinical benefit, as compared to the corresponding non-isotopically enriched compound.

Disclosed herein are methods for treating a subject, including a human, having or suspected of having a disease, disorder, or condition involving, but not limited to, headaches including migraine headaches with or without aura, movement disorders, depression, and anxiety and/or any disease, disorder, or condition ameliorated by modulating 5-hydroxytryptamine 1B and/or 1D receptors, or for preventing such disease, disorder, or condition, in a subject prone to the disease, disorder, or condition; comprising administering to the subject a therapeutically effective amount of a compound disclosed herein or a pharmaceutically acceptable salt, solvate, or prodrug thereof; so as to allow the treatment of headaches including migraine headaches with or without aura, movement disorders, depression, and anxiety and/or any disease, disorder, or condition ameliorated by modulating 5-hydroxytryptamine 1B and/or 1D receptors while reducing or eliminating deleterious changes in any diagnostic hepatobiliary function endpoints as compared to the corresponding non-isotopically enriched compound.

Examples of diagnostic hepatobiliary function endpoints include, but are not limited to, alanine aminotransferase ("ALT"), serum glutamic-pyruvic transaminase ("SGPT"), aspartate aminotransferase ("AST" or "SGOT"), ALT/AST ratios, serum aldolase, alkaline phosphatase ("ALP"), ammonia levels, bilirubin, gamma-glutamyl transpeptidase ("GGTP," "γ-GTP," or "GGT"), leucine aminopeptidase ("LAP"), liver biopsy, liver ultrasonography, liver nuclear scan, 5'-nucleotidase, and blood protein. Hepatobiliary endpoints are compared to the stated nonnal levels as given in "Diagnostic and Laboratory Test Reference", 4th edition, Mosby, 1999. These assays are run by accredited laboratories according to standard protocol.

Depending on the disease to be treated and the subject's condition, the compounds disclosed herein may be administered by oral, parenteral (e.g., intramuscular, intraperitoneal, intravenous, ICV, intracistemal injection or infusion, subcutaneous injection, or implant), inhalation, nasal, vaginal, rectal, sublingual, or topical (e.g., transdermal or local) routes of administration, and may be formulated, alone or together, in suitable dosage unit with pharmaceutically acceptable carriers, adjuvants and vehicles appropriate for each route of administration.

The dose may be in the form of one, two, three, four, five, six, or more sub-doses that are administered at appropriate intervals per day. The dose or sub-doses can be administered in the form of dosage units containing from about 0.1 to about 300 milligram, from about 0.1 to about 200 milligrams, or from 0.5 about to about 100 milligram active ingredient(s) per dosage unit, and if the condition of the patient requires, the dose can, by way of alternative, be administered as a continuous infusion.

The pharmaceutical compositions will preferably contain at least about 0.1 volume % by weight of the active ingredient. The actual concentration will depend on the human subject and the chosen administering route. In general this concentration will lie between about 0.1 and about 100% for the above applications and indications. The dose of the active ingredient to be administered can further vary between about I microgram and about 100 milligram per kilogram body weight per day, preferably between about microgram and 50 milligram per kilogram body weight per day, and most preferably between about I microgram and 20 milligram per kilogram body weight per day.

In certain embodiments, an appropriate dosage level is about 0.01 to about 100 mg per kg patient body weight per day (mg/kg per day), about 0.01 to about 50 mg/kg per day, about 0.01 to about 25 mg/kg per day, or about 0.05 to about 10 mg/kg per day, which may be administered in single or multiple doses. A suitable dosage level may be about 0.01 to about 100 mg/kg per day, about 0.05 to about 50 mg/kg per day, or about 0.1 to about 10 mg/kg per day. Within this range the dosage may be about 0.01 to about 0.1, about 0.1 to about 1.0, about 1.0 to about 10, or about 10 to about 50 mg/kg per day.

### Combination Therapy

The compounds disclosed herein may also be combined or used in combination with other agents useful in the treatment, prevention, or amelioration of one or more symptoms of, but not limited to, headaches including migraine headaches with or without aura, movement disorders, depression, and anxiety and/or any disease, disorder, or condition ameliorated by modulating 5-hydroxytryptamine 1B and/or 1D receptors. Or, by way of example only, the therapeutic effectiveness of one of the compounds disclosed herein may be enhanced by administration of an adjuvant (*i.e*., by itself the adjuvant may only have minimal therapeutic benefit, but in combination with another therapeutic agent, the overall therapeutic benefit to the patient is enhanced).

Such other agents, adjuvants, or drugs, may be administered, by a route and in an amount commonly used therefor, simultaneously or sequentially with a compound disclosed herein. When a compound disclosed herein is used contemporaneously with one or more other drugs, a pharmaceutical composition containing such other drugs in addition to the compounds disclosed herein may be utilized, but is not required. Accordingly, the pharmaceutical compositions disclosed herein include those that also contain one or more other active ingredients or therapeutic agents, in addition to the compounds disclosed herein.

In certain embodiments, the compounds disclosed herein can be combined with one or more sepsis treatments known in the art, including, but not limited to drotrecogin-α or a biosimilar of activated protein C.

In certain embodiments, the compounds disclosed herein can be combined with one or more steroidal drugs known in the art, including, but not limited to, aldosterone, beclometasone, betamethasone, deoxycorticosterone acetate, fludrocortisone acetate, hydrocortisone (cortisol), prednisolone, prednisone, methylprenisolone, dexamethasone, and triamcinolone.

In certain embodiments, the compounds disclosed herein can be combined with one or more antibacterial agents known in the art, including, but not limited to the group including amikacin, amoxicillin, ampicillin, arsphenamine, azithromycin, aztreonam, azlocillin, bacitracin, carbenicillin, cefaclor, cefadroxil, cefamandole, cefazolin, cephalexin, cefdinir, cefditorin, cefepime, cefixime, cefoperazone, cefotaxime, cefoxitin, cefpodoxime, cefprozil, ceftazidime, ceftibuten, ceftizoxime, ceftriaxone, cefuroxime, chloramphenicol, cilastin, ciprofloxacin, clarithromycin, clindamycin, cloxacillin, colistin, dalfopristan, demeclocycline, dicloxacillin, dirithromycin, doxycycline, erythromycin, enafloxacin, ertepenem, ethambutol, flucloxacillin, fosfomycin, furazolidone, gatifloxacin, geldanamycin, gentamicin, herbimicin, imipenem, isoniazide, kanamicin, levofloxacin, linezolid, lomefloxacin, loracarbef, mafenide, moxifloxacin, meropenem, metronidazole, mezlocillin, minocycline, mupirozin, nafcillin, neomycin, netilmicin, nitrofurantoin, norfloxacin, ofloxacin, oxytetracycline, penicillin, piperacillin, platensimycin, polymixin B, prontocil, pyrazinamide, quinupristine, rifampin, roxithromycin, spectinomycin, streptomycin, sulfacetamide, sulfamethizole, sulfamethoxazole, teicoplanin, telithromycin, tetracycline, ticarcillin, tobramycin, trimethoprim, troleandomycin, trovafloxacin, and vancomycin.

In certain embodiments, the compounds disclosed herein can be combined with one or more antifungal agents known in the art, including, but not limited to the group including amorolfine, amphotericin B, anidulafungin, bifonazole, butenafine, butoconazole, caspofungin, ciclopirox, clotrimazole, econazole, fenticonazole, filipin, fluconazole, isoconazole, itraconazole, ketoconazole, micafungin, miconazole, naftifine, natamycin, nystatin, oxyconazole, ravuconazole, posaconazole, rimocidin, sertaconazole, sulconazole, terbinafine, terconazole, tioconazole, and voriconazole.

In certain embodiments, the compounds disclosed herein can be combined with one or more anticoagulants known in the art, including, but not limited to the group including acenocoumarol, argatroban, bivalirudin, lepirudin, fondaparinux, heparin, phenindione, warfarin, and ximalagatran.

In certain embodiments, the compounds disclosed herein can be combined with one or more thrombolytics known in the art, including, but not limited to the group including anistreplase, reteplase, t-PA (alteplase activase), streptokinase, tenecteplase, and urokinase.

In certain embodiments, the compounds disclosed herein can be combined with one or more non-steroidal anti-inflammatory agents known in the art, including, but not limited to the group including aceclofenac, acemetacin, amoxiprin, aspirin, azapropazone, benorilate, bromfenac, carprofen, celecoxib, choline magnesium salicylate, diclofenac, diflunisal, etodolac, etoracoxib, faislamine, fenbuten, fenoprofen, flurbiprofen, ibuprofen, indometacin, ketoprofen, ketorolac, lomoxicam, loxoprofen, lumiracoxib, meclofenamic acid, mefenamic acid, meloxicam, metamizole, methyl salicylate, magnesium salicylate, nabumetone, naproxen, nimesulide, oxyphenbutazone, parecoxib, phenylbutazone, piroxicam, salicyl salicylate, sulindac, sulfinprazone, suprofen, tenoxicam, tiaprofenic acid, and tolmetin.

In certain embodiments, the compounds disclosed herein can be combined with one or more antiplatelet agents known in the art, including, but not limited to the group including abciximab, cilostazol, clopidogrel, dipyridamole, ticlopidine, and tirofibin.

The compounds disclosed herein can also be administered in combination with other classes of compounds, including, but not limited to, endothelin converting enzyme (ECE) inhibitors, such as phosphoramidon; thromboxane receptor antagonists, such as ifetroban; potassium channel openers; thrombin inhibitors, such as hirudin; growth factor inhibitors, such as modulators of PDGF activity; platelet activating factor (PAF) antagonist; anti-platelet agents, such as GPIIb/IIIa blockers (e.g., abdximab, eptifibatide, and tirofiban), P2Y(AC) antagonists (e.g., clopidogrel, ticlopidine and CS-747), and aspirin; anticoagulants, such as warfarin; low molecular weight heparins, such as enoxaparin; Factor VIIa Inhibitors and Factor Xa Inhibitors; renin inhibitors; neutral endopeptidase (NEP) inhibitors; vasopepsidase inhibitors (dual NEP-ACE inhibitors), such as omapatrilat and gemopatrilat; HMG CoA reductase inhibitors, such as pravastatin, lovastatin, atorvastatin, simvastatin, NK-104 (a.k.a. itavastatin, nisvastatin, or nisbastatin), and ZD-4522 (also known as rosuvastatin, or atavastatin or visastatin); squalene synthetase inhibitors; fibrates; bile acid sequestrants, such as questran; niacin; anti-atherosclerotic agents, such as ACAT inhibitors; MTP Inhibitors; calcium channel blockers, such as amlodipine besylate; potassium channel activators; alpha-adrenergic agents; beta-adrenergic agents, such as carvedilol and metoprolol; antiarrhythmic agents; diuretics, such as chlorothlazide, hydrochiorothiazide, flumethiazide, hydroflumethiazide, bendroflumethiazide, methylchlorothiazide, trichioromethiazide, polythiazide, benzothlazide, ethacrynic acid, tricrynafen, chlorthalidone, furosenilde, musolimine, bumetanide, triamterene, amiloride, and spironolactone; thrombolytic agents, such as tissue plasminogen activator (tPA), recombinant tPA, streptokinase, urokinase, prourokinase, and anisoylated plasminogen streptokinase activator complex (APSAC); anti-diabetic agents, such as biguanides (e.g. metformin), glucosidase inhibitors (e.g., acarbose), insulins, meglitinides (e.g., repaglinide), sulfonylureas (e.g., glimepiride, glyburide, and glipizide), thiozolidinediones (e.g. troglitazone, rosiglitazone and pioglitazone), and PPAR-gamma agonists; mineralocorticoid receptor antagonists, such as spironolactone and eplerenone; growth honnone secretagogues; aP2 inhibitors; phosphodiesterase inhibitors, such as PDE III inhibitors (e.g., cilostazol) and PDE V inhibitors (e.g., sildenafil, tadalafil, vardenafil); protein tyrosine kinase inhibitors; antiinflammatories; antiproliferatives, such as methotrexate, FK506 (tacrolimus, Prograf), mycophenolate mofetil; chemotherapeutic agents; immunosuppressants; anticancer agents and cytotoxic agents (e.g., alkylating agents, such as nitrogen mustards, alkyl sulfonates, nitrosoureas, ethylenimines, and triazenes); antimetabolites, such as folate antagonists, purine analogues, and pyrridine analogues; antibiotics, such as anthracyclines, bleomycins, mitomycin, dactinomycin, and plicamycin; enzymes, such as L-asparaginase; farnesyl-protein transferase inhibitors; hormonal agents, such as glucocorticoids (e.g., cortisone), estrogens/antiestrogens, androgens/antiandrogens, progestins, and luteinizing, hormone-releasing hormone anatagonists, and octreotide acetate; microtubule-disruptor agents, such as ecteinascidins; microtubule-stablizing agents, such as pacitaxel, docetaxel, and epothilones A-F; plant-derived products, such as vinca alkaloids, epipodophyllotoxins, and taxanes; and topoisomerase inhibitors; prenyl-protein transferase inhibitors; and cyclosporins; steroids, such as prednisone and dexamethasone; cytotoxic drugs, such as azathiprine and cyclophosphamide; TNF-alpha inhibitors, such as tenidap; anti-TNF antibodies or soluble TNF receptor, such as etanercept, rapamycin, and leflunimide; and cyclooxygenase-2 (COX-2) inhibitors, such as celecoxib and rofecoxib; and miscellaneous agents such as, hydroxyurea, procarbazine, mitotane, hexamethylmelamine, gold compounds, platinum coordination complexes, such as cisplatin, satraplatin, and carboplatin.

### Kits/Articles of Manufacture

For use in the therapeutic applications described herein, kits and articles of manufacture are also described herein. Such kits can comprise a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. The containers can be formed from a variety of materials such as glass or plastic.

For example, the container(s) can comprise one or more compounds described herein, optionally in a composition or in combination with another agent as disclosed herein. The container(s) optionally have a sterile access port (for example the container can be an intravenous solution bag or a vial having a stopper pierceable by a hypodermic injection needle). Such kits optionally comprise a compound with an identifying description or label or instructions relating to its use in the methods described herein.

A kit will typically comprise one or more additional containers, each with one or more of various materials (such as reagents, optionally in concentrated form, and/or devices) desirable from a commercial and user standpoint for use of a compound described herein. Non-limiting examples of such materials include, but are not limited to, buffers, diluents, filters, needles, syringes; carrier, package, container, vial and/or tube labels listing contents and/or instructions for use, and package inserts with instructions for use. A set of instructions will also typically be included.

A label can be on or associated with the container. A label can be on a container when letters, numbers or other characters forming the label are attached, molded or etched into the container itself; a label can be associated with a container when it is present within a receptacle or carrier that also holds the container, e.g., as a package insert. A label can be used to indicate that the contents are to be used for a specific therapeutic application. The label can also indicate directions for use of the contents, such as in the methods described herein. These other therapeutic agents may be used, for example, in the amounts indicated in the Physicians' Desk Reference (PDR) or as otherwise determined by one of ordinary skill in the art.

The invention is further illustrated by the following examples.

### Chemistry

The compounds disclosed herein can be prepared by methods known in the art and routine modifications thereof, and/or following procedures similar to those described in the Example section herein and routine modifications thereof, and/or procedures found in the references cited therein and routine modifications thereof.

### EXAMPLE 1

### d₂-C-[3-(2-Dimethylamino-ethyl)-1H-indol-5-yl]-N-methyl-methanesulfonimide (d₂-sumatriptan)

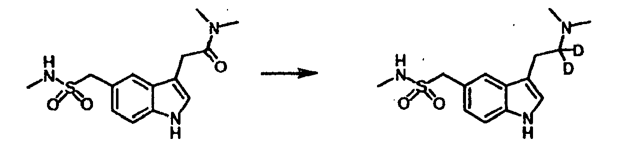

The procedure was carried out as described in Bosch, Tetrahedron 2001, 57(6), 1041-1048, with substitution of LiAlD₄ for LiAlH₄, which is hereby incorporated by reference in its entirety.

### EXAMPLE 2

### d₂-C-[3-(2-Dimethylamino-ethyl)-1H-indol-5-yl]-N-methyl-methanesulfonamide (d₂-sumatriptan)

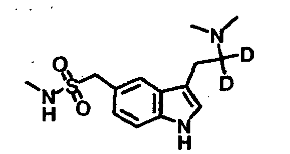

### Step 1

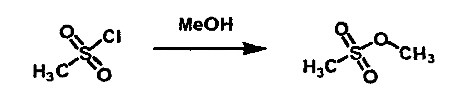

Methyl methanesulfonate A solution of methanol (15 g, 0.469 mol) in dry methylene chloride (400 mL) was cooled to -30°C and treated with triethylamine (71.5 g, 0.705 mol). The mixture was stirred under a nitrogen atmosphere for 15 minutes, and a solution of methanesulfonyl chloride (59.05g, 0.515 mol) was added dropwise. The resulting mixture was stirred at -20°C for 1 hour. The organic phase was washed with water, 1N hydrochloric acid and saturated aqueous sodium bicarbonate, dried over magnesium sulfate, and concentrated to give the title compound. ¹H-NMR (300 MHz, CDCl₃) δ 3.91 (s, 3H), 3.01 (s, 3H). GC-MS (m/z): 110 (M⁺).

### Step 2

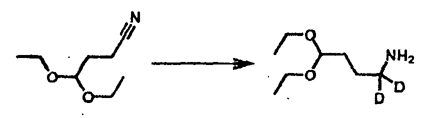

d₂-4,4-Diethoxybutylamine: A solution of 4,4-diethoxy-butyronitrile (9.3 g, 59.5 mmol) in dry tetrahydrofuran (100 mL) was added dropwise to a solution of lithium aluminum deuteride (5.0 g, 119 mmol) in dry tetrahydrofuran (100 mL) at reflux. The resulting mixture was heated to reflux for 4 hours, cooled to 0°C and water (200 mL) was added dropwise. After filtering off the solids, the organic phase was washed with water, dried over magnesium sulfate and concentrated to give the title compound. ¹H-NMR (300 MHz, CDCl₃) δ 4.50 (t, J=5.4 Hz, 1H), 3.59-3.67 (m, 2H), 3.46-3.51 (m, 2H), 1.60-1.68(m, 2H), 1.47-1.53(m, 2H), 1.18 (t, J=7.0 Hz, 6H). GC-MS (m/z): 163 (M⁺).

### Step 3

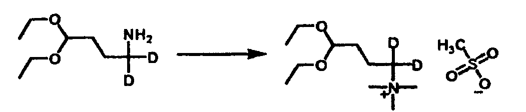

d₂-Methanesulfonate (4,4-diethoxy-butyl)-trimethylammonium: A solution of 4,4-diethoxybutylamine (9.7 g, 59.5 mmol) and methyl methanesulfonate (22.9 g, 208 mmol) in chloroform (200 mL) was treated with potassium carbonate (24.6 g, 178.5 mmol), and heated to reflux overnight. The reaction mixture was filtered. The filtrate was concentrated in vacuo to give the title compound. LC-MS (m/z): 206 (M⁺).

### Step 4

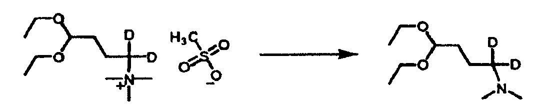

d₂-(4,4-Diethoxy-butyl)-dimethylamine: A solution of d₂-methanesulfonate (4,4-diethoxy-butyl)-trimethylammonium (18.0 g, 59.5 mmol) in 2-aminoethanol (18 mL) was stirred at reflux for 4 hours. The reaction mixture was diluted with water (20 mL), and extracted with chloroform (100 mL). The organic extract was washed with brine and concentrated to give the title compound. LC-MS (m/z): 192 (M+1)⁺.

### Step 5

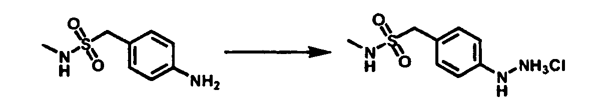

*C-*(4-hydrazino-phenyl)-*n-*methyl-methanesulfonamide hydrochloride: A solution of sodium nitrite (7.6 g, 110 mmol) in water was added dropwise to a suspension of *C-*(4-aminophenyl)-N-methyl-methanesulfonamide (20 g, 100 mmol) in concentrated hydrochloric acid (106 mL) at -10°C. The resulting mixture was stirred at -5°C for 30 minutes and filtered into a pre-cooled flask. The solution was added dropwise to a cooled and stirred solution of tin chloride dihydrate (90.3 g, 400 mmol) in concentrated hydrochloric acid (106 mL) at -5°C. The resulting suspension was wanned to ambient temperature, filtered and the solid product was washed with ether and hexane, dried under vacuum to give the title compound. ¹H-NMR (300 MHz, DMSO-d₆) δ 10.30 (s, 3H), 7.24 (d, J=8.4 Hz, 2H), 6.94 (d, J=8.4 Hz, 2H), 4.22 (s, 2H), 2.50 (s, 3H). LC-MS (m/z): 216 (M+1)⁺.

### Step 6

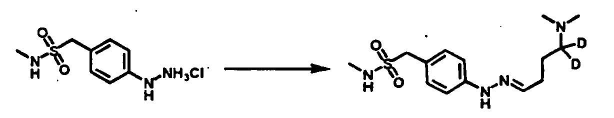

d₂-C-(4-N'-[4-Dimethylamino-but-(E)-ylidene]-hydrazino)-phenyl-N-methyl-methanesulfonamide: A solution of C-(4-hydrazino-phenyl)-N-methyl-methanesulfonamide hydrochloride (7.2 g, 28.6 mmol) and d₂-(4,4-diethoxy-butyl)-dimethylamine (4.5 g, 23.8 mmol) in water (27 mL) was treated with 2N hydrochloric acid (9 mL), and stirred at ambient temperature overnight. The reaction mixture was basified with sodium carbonate and extracted with 200 mL of chloroform. The organic extract was washed with brine and concentrated to give the title compound. LC-MS (m/z): 315 (M+1)⁺.

### Step 7

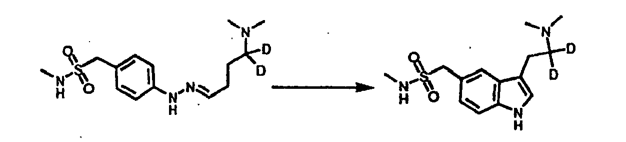

d₂-*C-*[3-(2-Dimethylamino-ethyl)-1H-indol-5-yl]-N-methyl-methanesulfonamide: A solution of d₂-C-(4-N'-[4-dimethylamino-but-(E)-ylidene]-hydrazino)-phenyl-N-methyl-methanesulfonamide (3.97 g, 13.1 mmol) in chloroform (200 mL) was treated with ethyl polyphosphate (27.8 g), and stirred at 30°C for 4 hours. Water (100 mL) was added, the organic layer was separated and the aqueous layer was basified with potassium carbonate and extracted with 200 mL of ethyl acetate. The organic extract was washed with brine and concentrated. The residue was purified by column chromatography to give the title compound. ¹H-NMR (300 MHz, CDCl₃) δ 8.19 (s, 1H), 7.59 (s, 1H), 7.36 (d, J=8.4 Hz, 1H), 7.19 (d, J=8.4 Hz, I H), 7.06 (s, 1H), 4.36 (s, 1H), 4.14 (s, 1H), 2.93(s, 2H), 2.71(s, 3H), 2.36(s, 6H). LC-MS (m/z): 298 (M+1)⁺. Purity > 97% (HPLC, 214 nm UV).

### EXAMPLE 3

### d₆-C-[3-(2-Dimethylamino-ethyl)-1H-indol-5-yl]-N-methyl-methanesulfonamide (d₆-sumatriptan)

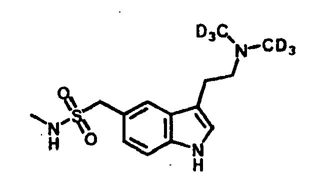

### Step 1

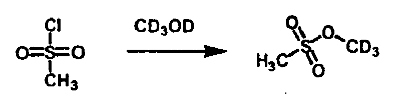

d₃-Methyl methanesulfonate: Prepared according to Example 2 by substituting methanol-d₄ for methanol. ¹H-NMR (300 MHz, CDCl₃) δ 3.01 (m, 3H). GC-MS (m/z): 113 (M⁺).

### Step 2

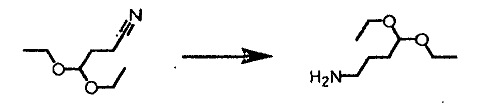

4,4-Diethoxybutyl-amine: Prepared according to Example 2 by substituting lithium aluminum hydride for lithium aluminum deuteride. ¹H-NMR (300 MHz, CDCl₃) δ 4.50 (t, J=5.4 Hz, 1H), 3.59-3.67 (m, 2H), 3.46-3.51 (m, 2H), 2.69 (t, 2H), 1.60-1.68(m, 2H), 1.47-1.53(m, 2H), 1.18 (t, J=7.0 Hz, 6H). GC-MS (m/z): 161 (M⁺).

### Step 3

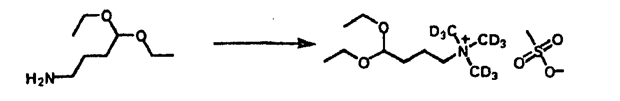

d₉-Methanesulfonate (4,4-diethoxy-butyl)-trimethylammonium: Prepared according to Example 2 by substituting d₃-methyl methanesulfonate for methyl methanesulfonate. LC-MS (m/z): 213 (M⁺).

### Step 4

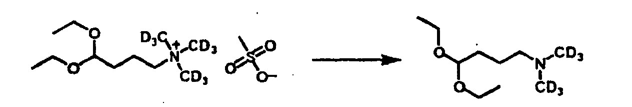

d₆-(4,4-Diethoxybutyl)-dimethylamine: Prepared according to Example 2 by substituting d₉-methanesulfonate (4,4-diethoxy-butyl)-trimethylammonium for d₂-methanesulfonate (4,4-diethoxy-butyl)-trimethylammonium. LC-MS (m/z): 196 (M+1)⁺.

### Step 5

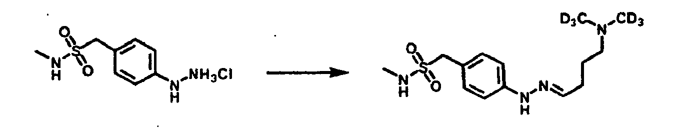

d₆-*C-*(4-N'-[4-Dimethylamino-but-(E)-ylidene]-hydrazino)-phenyl-N-methyl-methanesulfonamide: Prepared according to Example 2 by substituting (d₆-(4,4-diethoxybutyl)-dimethylamine for d₂-(4,4-diethoxybutyl)-dimethylamine. LC-MS (m/z): 319 (M+1)⁺.

### Step 6

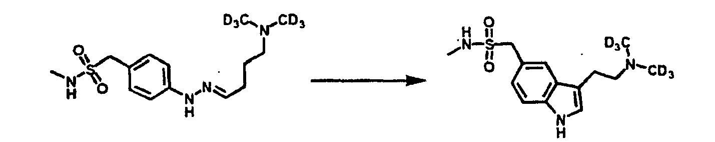

d₆-C-[3-(2-Dimethylamino-ethyl)-1H-indol-5-yl]-N-methyl-methanesulfonamide: Prepared according to Example 2 by substituting d₆-C-(4-N'-[4-dimethylamino-but-(E)-ylidene]-hydrazino)-phenyl-N-methyl-methanesulfonamide for d₂-C-(4-N'-[4-dimethylamino-but-(E)-ylidene]-hydrazino)-phenyl-N-methyl-methanesulfonamide. ¹H-NMR (300 MHz, CDCl₃) δ 8.19 (s, 1H), 7.63 (s, 1H), 7.36 (d, J=8.4 Hz, 1H), 7.22 (d, J=8.4 Hz, 1H), 7.01 (s, 1H), 4.38 (s, 1H), 4.16 (s, 1H), 2.96 (dd, J= 8.4 Hz, 7.2 Hz, 2H), 2.65-2.74 (m, 5H). LC-MS (m/z): 302 (M+1)⁺. Purity: > 99% (HPLC, 214 nm, UV).

### EXAMPLE 4

### d₈-C-[3-(2-Dimethylamino-ethyl)-1H-indol-5-yl]-N-methyl-methanesulfonamide (d₈-sumatriptan)

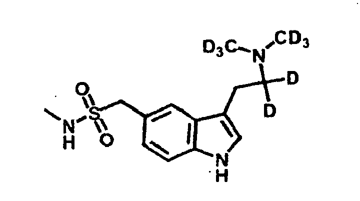

### Step 1

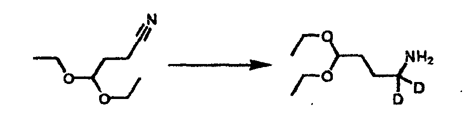

### d₂-4,4-Diethoxybutylamine: Prepared according to Example 2.

### Step 2

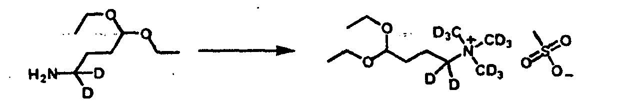

d₁₁-Methanesulfonate (4,4-diethoxybutyl)-trimethylammonium: Prepared according to Example 3 by substituting d₂-4,4-diethoxybutylamine for 4,4-diethoxybutylamine. LC-MS (m/z): 215 (M⁺).

### Step 3

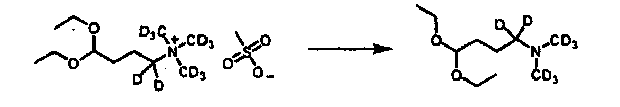

d₈-(4,4-Diethoxy-butyl)-dimethyl-amine: Prepared according to Example 3 by substituting d₁₁-methanesulfonate (4,4-diethoxybutyl)-trimethylammonuim for d₉-methanesulfonate (4,4-diethoxybutyl)-trimethylammonium. LC-MS (m/z): 198 (M+1)⁺.

### Step 4

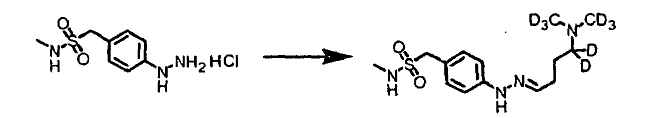

d₈-*C-*(4-N'-[4-Dimethylamino-but-(E)-ylidene]-hydrazino)-phenyl-N-methyl-methanesulfonamide: Prepared according to Example 2 by substituting d₈-(4,4-diethoxybutyl)-dimethylamine for d₂-(4,4-diethoxybutyl)-dimethylamine. LC-MS (m/z): 321 (M+1)⁺.

### Step 5

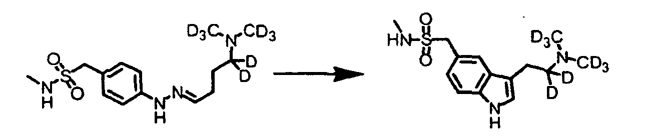

d₈-C-[3-(2-Dimethylamino-ethyl)-1H-indol-5-yl]-N-methyl-methanesulfonamide: Prepared according to Example 2 by substituting d₈-C-(4-N'-[4-dimethylamino-but-(E)-ylidene]-hydrazino)-phenyl-N-methyl-methanesulfonamide for d₂-C-(4-N'-[4-dimethylamino-but-(E)-ylidene]-hydrazino)-pheny)-N-methyl-methanesulfonamide. ¹H-NMR (300 MHz, CDCl₃) δ 8.19 (s, 1H), 7.60 (s, 1H), 7.39 (d, J= 8.4 Hz, 1H), 7.24 (d, J= 8.4 Hz, 1H), 7.01 (s, 1H), 4.37 (s, 2H), 4.00 (s, 1H), 3.00 (s, 2H), 2.70-2.72 (m, 3H). LC-MS (m/z): 304 (M+1)⁺. Purity > 99% (HPLC, 214 nm, UV).

### EXAMPLE 5

### d₂-5-Pyrrolidine-sulfonylmethyl-3-N,N-dimethylaminoethyl-indole (d₂-Almotriptan)

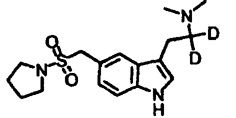

### Step 1

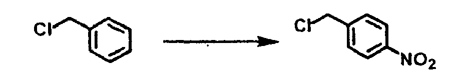

1-(chloromethyl)-4-nitrobenzene: To a three-necked round bottom flask containing 245 mL of benzyl chloride was added a mixture of concentrated nitric acid (98 mL) and cold concentrated sulfuric acid (122.5 mL). The reaction mixture was wanned to ambient temperature and stirred for 2 hours. The mixture was poured into ice, and the precipitate was filtered and recrystallized from ethanol to give the title compound. ¹H-NMR (300 MHz, CDCl₃) δ 4.65 (s, 2H), 7.56 (d, J=8.4Hz, 2H), 8.22 (d, J=8.4Hz, 2H). GC-MS (m/z): 171 (M⁺).

### Step 2

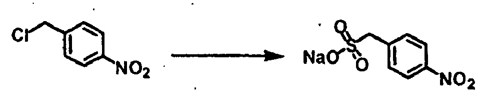

sodium (4-nitrophenyl)-methanesulfonate: A three-necked round bottom flask containing 285 mL of water was charged with sodium sulfite (76.6 g, 0.61 mol), methanol (190 mL) and 1-(chloromethyl)-4-nitrobenzene (94.8 g, 0.55 mol). The reaction mixture was heated to reflux overnight, cooled to ambient temperature. The precipitate was filtered, washed with ethanol and dried to give to give the title compound. LC-MS (m/z): 240 (M+1)⁺.

### Step 3

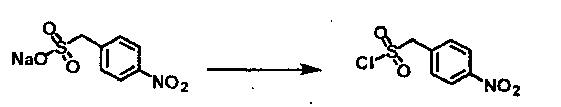

(4-nitrophenyl)methanesulfonyl chloride: A mixture containing sodium (4-nitrophenyl)-methanesulfonate (100 g, 0.42 mol), phosphorous pentachloride (100.5 g, 0.48 mol) and toluene (600 mL) was heated to reflux for 1 hour. The reaction mixture was cooled to ambient temperature, filtered and concentrated to give the title compound.

### Step 4

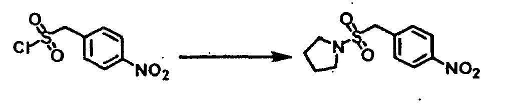

4-nitrobenzylsulfonyl-pyrrolidine: Pyrrolidine (74.2 g, 1.05 mol) was added dropwise to a solution of (4-nitrophenyl)-methanesulfonyl chloride (98 g, 0.42 mol) in dichloromethane (150 mL) at 10°C. The reaction mixture was wanned to ambient temperature and stirred overnight. The relation mixture was washed with water, dried over anhydrous sodium sulfate, concentrated and recrystallized from ethanol and dichloromethane to give the title compound. ¹H-NMR (300 MHz, CDCl₃) δ 1.86(m, 4H), 3.22(t, J=6.6Hz, 4H), 4.32(s, 2H), 7.59(d, J=8.7Hz, 2H), 8.24(d, J=8.7Hz, 2H).

### Step 5

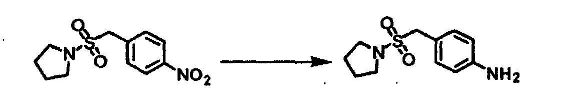

4-(pyrrolidine-1-sulfonylmethyl)aniline: A mixture of 4-nitrobenzylsulfonyl-pyrrolidine (40 g, 0.15 mol) and 10% palladium on carbon (10 g) in dichloromethane (300 mL) was stirred at ambient temperature for 24 hours under hydrogen atmosphere. The reaction mixture was filtered and was concentrated to give the title compound. ¹H-NMR (300 MHz, CDCl₃) δ 1.78 (m, 4H), 3.14 (t, J=6.3Hz, 4H), 3.74 (br, 2H), 4.14 (s, 2H), 6.65 (d, J=8.1Hz, 2H), 7.15 (d, J=8.1Hz, 2H).

### Step 6

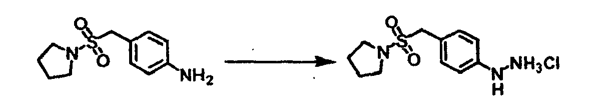

4-(pyrrolidine-1-sulfonylmethyl) phenylhydrazine hydrochloride: A solution of sodium nitrite (5.2 g, 75 mmol) in 22.5 mL of water was added dropwise to a suspension of 4-(pyrrolidine-1-sulfonylmethyl)-aniline (15 g, 62.5 mmol) in concentrated hydrochloric acid (66 mL) at -5°C. The reaction mixture was stirred at -5°C for I hour. Then the mixture was added dropwise to a cooled and stirred solution of tin chloride dihydrate (56.4 g, 250 mmol) in concentrated hydrochloric acid (66 mL) at -5°C. The resulting suspension was wanned to ambient temperature, filtered and the solid was washed with ether and hexane to give the title compound. LC-MS (m/z): 256 (M+1)⁺.

### Step 7

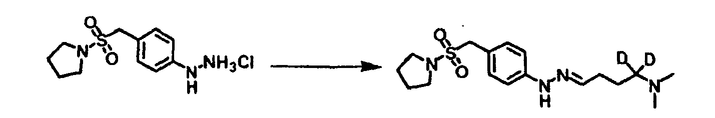

d₂-4-[2-[4-(Dimethylamino)butylidene]hydrazinyl]-benzylsulfonylpyrrolidine: A solution of d₂-(4,4-diethoxybutyl)-dimethylamine (3.76 g, 19.7 mmol) and 4-(pyrrolidine-1-sulfonylmethyl) phenylhydrazine hydrochloride (4.5 g, 23.6 mmol) in water (30ml) was treated with 2N hydrochloric acid (10 mL). After stirring at ambient temperature for 4 hours, the reaction mixture was basified with sodium carbonate and extracted with chlorofonn. The organic extract was washed with brine and concentrated to give the title compound. LC-MS (m/z): 355 (M+1)⁺.

### Step 8

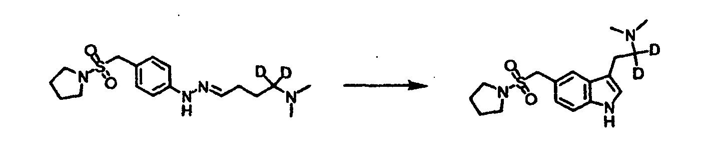

d₂-5-Pyrrolidine-sulfonylmethyl-3-N,N-dimethylaminoethyl-indole: A solution of d₂-4-[2-[4-(dimethylamino)-butylidene]hydrazinyl]-benzylsulfonyl-pyrrolidine (6.97 g, 19.69 mmol) in chloroform (200 mL) was treated with ethyl polyphosphate (29.3 g). After stirring at 30°C for 4 hours, water (100 mL) was added. The organic layer was separated and the aqueous layer was basified with potassium carbonate and extracted with chloroform. The organic extract was washed with brine, concentrated and the residue was purified by column chromatography to give the title compound. ¹H-NMR (300 MHz, CDCl₃) δ 1.78 (t, J=6.3Hz, 4H), 2.43 (s, 6H), 3.00 (s, 2H), 3.15 (t, J=6.3Hz, 4H), 4.38 (s, 2H), 7.07 (s, 1H), 7.25 (d, J=8.4Hz, 1H), 7.34 (d, J=8.4Hz, 1H), 7.61 (s, 1H), 8.19 (s, 1H). LC-MS (m/z): 338. Purity > 95% (HPLC).

### Biological Assays

Changes in the metabolic properties of the compounds in Examples 1-5 as compared to their non-isotopically enriched analogs can be shown using the following assays. Other compounds listed above, which have not yet been made and/or tested, are predicted to have changed metabolic properties as shown by one or more of these assays as well.

### EXAMPLE 6

### In vitro metabolism using human cytochrome P₄₅₀ enzymes

The cytochrome P₄₅₀ enzymes are expressed from the corresponding human cDNA using a baculovirus expression system (BD Biosciences, San Jose, CA). A 0.25 milliliter reaction mixture containing 0.8 milligrams per milliliter protein, 1.3 millimolar NADP⁺, 3.3 millimolar glucose-6-phosphate, 0.4 U/mL glucose-6-phosphate dehydrogenase, 3.3 millimolar magnesium chloride and 0.2 millimolar of a compound of Fonnula I, the corresponding non-isotopically enriched compound or standard or control in 100 millimolar potassium phosphate (pH 7.4) is incubated at 37 °C for 20 min. After incubation, the reaction is stopped by the addition of an appropriate solvent (e.g., acetonitrile, 20% trichloroacetic acid, 94% acetonitrile/6% glacial acetic acid, 70% perchloric acid, 94% acetonitrile/6% glacial acetic acid) and centrifuged (10,000 g) for 3 min. The supernatant is analyzed by HPLC/MS/MS.

| **Cytochrome P₄₅₀** | **Standard** |
|---|---|
| CYP1A2 | Phenacetin |
| CYP2A6 | Coumarin |
| CYP2B6 | [¹³C]-(S)-mephenytoin |
| CYP2C8 | Paclitaxel |
| CYP2C9 | Diclofenac |
| CYP2C19 | [¹³C]-(S)-mephenytoin |
| CYP2D6 | (+/-)-Bufuralol |
| CYP2E1 | Chlorzoxarone |
| CYP3A4 | Testosterone |
| CYP4A | [¹³C]-Lauric acid |

### EXAMPLE 7

### In vitro Liver Microsomal Stability Assay

Liver microsomal stability assays were conducted at 1 mg per mL liver microsome protein with an NADPH-generating system in 2%NaHCO₃ (2.2 mM NADPH, 25.6 mM glucose 6-phosphate, 6 units per mL glucose 6-phosphate dehydrogenase and 3.3 mM MgCl₂). Test compounds were prepared as solutions in 20% acetonitrile-water and added to the assay mixture (final assay concentration 5 microgram per mL) and incubated at 37°C. Final concentration of acetonitrile in the assay were <1%. Aliquots (50µL) were taken out at times 0, 15, 30, 45, and 60 minutes, and diluted with ice cold acetonitrile (200 µL) to stop the reactions. Samples were centrifuged at 12000 RPM for 10 minutes to precipitate proteins. Supernatants were transferred to microcentrifuge tubes and stored for LC/MS/MS analysis of the degradation half-life of the test compounds. It has thus been found that the compounds of formula (1) according to the present invention that have been tested in this assay showed an increase of 10% or more in the degradation half life, as compared to the non-isotopically enriched drug. For example, the degradation half-life of 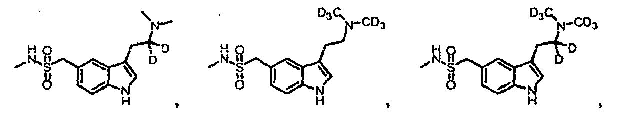 and 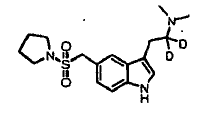 were increased by 20-250% as compared to non-isotopically enriched compounds.

### EXAMPLE 8

### Monoamine Oxidase A Inhibition and Oxidative Turnover

The procedure is carried out using the methods described by Weyler, Journal of Biological Chemistry 1985, 260, 13199-13207, which is hereby incorporated by reference in its entirety. Monoamine oxidase A activity is measured spectrophotometrically by monitoring the increase in absorbance at 314 nm on oxidation of kynuramine with formation of 4-hydroxyquinoline. The measurements are carried out, at 30°C, in 50mM NaPᵢ buffer, pH 7.2, containing 0.2% Triton X-100 (monoamine oxidase assay buffer), plus 1 mM kynuramine, and the desired amount of enzyme in 1 mL total volume.

### EXAMPLE 9

### Monooamine Oxidase B Inhibition and Oxidative Turnover

The procedure is carried out as described in Uebelhack, *Pharmacopsychiatry* **1998**, *31*(5), 187-192, which is hereby incorporated by reference in its entirety.

### EXAMPLE 10

### Preparation of Platelet-Rich Plasma and Platelets

Venous blood from healthy subjects is collected between 8 and 8:30 a.m. after overnight fasting into EDTA-containing vacutainer tubes (11.6 mg EDTA/mL blood).

After centrifugation of the blood at 250 x g for 15 minutes at 20°C, the supernatant plalelet-rich plasma (PRP) is collected and the number of platelets in PRP counted with a cell counter (MÖLAB, Hilden, Gennany). PRP (2 mL) is spun at 1500 x g for 10 minutes to yield a platelet pellet. The pellet is washed three times with ice-cold saline, resuspended in 2 mL Soerensen phosphate buffer, pH 7.4 and stored at -18°C for one day.

### EXAMPLE 11

### MAO assay

Fresh PRP or frozen platelet suspension (100 µl) is generally preincubated for 10 minutes in the absence or presence of drugs at 37°C in 100 µl of 0.9% NaCl solution or phosphate buffer pH 7.4, respectively, at 37°C. 2-Phenyllethylamine-[ethyl-1-¹⁴C]hydrochloride (PEA) solution (specific activity 56 Ci/mol, Amersham, 50 µl) is then added in a final concentration of 5 µM and the incubation is continued for 30 minutes. The reaction is terminated by the addition of 50 µl 4M HClO₄. The reaction product of MAO, phenylacetaldehyde, is extracted into 2 mL of n-hexane. An aliquot of the organic phase is added to scintillator cocktail and the radioactivity is determined using a liquid scintillation counter. Product formation is linear with time for at least 60 min with appropriate platelet numbers. Blank values are obtained by including 2 mM pargyline in the incubation mixtures.

### EXAMPLE 12

### Hydroxytryptamine 1B Binding Assay

The procedure is carried out as described in Hoyer, European Journal of Pharmacology 1985, 118(1-2), 1-12, and Hoyer, European Journal of Pharmacology 1985, 118(1-2), 13-23 which are hereby incorporated by references in their entirety.

### EXAMPLE 13

### 5-Hydroxytryptamine 1D Binding Assay

The procedure is carried out as described in Heuring, Journal of Neuroscience 1987, 7(3), 894-903, which is hereby incorporated by reference in its entirety.

The examples set forth above are provided to give a complete disclosure and description of how to make and use the claimed embodiments, and are not intended to limit the scope of what is disclosed herein. Modifications that are obvious, in the art, are intended to be within the scope of the following claims. All publications, patents, and patent applications cited in this specification are incorporated herein by reference as if each such publication, patent or patent application were specifically and individually indicated to be incorporated herein by reference. However, with respect to any similar or identical tenns found in both the incorporated publications or references and those explicitly put forth or defined in this document, then those tenns definitions or meanings explicitly put forth in this document shall control in all respects.

## Claims

1. A compound having structural Formula I 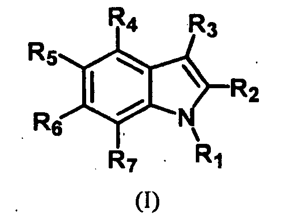 or a pharmaceutically acceptable salt or solvate thereof, wherein:
R₃ is selected from the group consisting of: 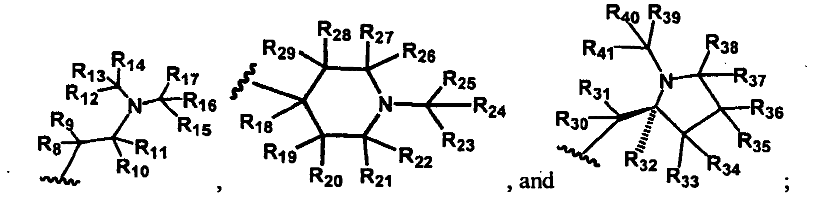
R₅ is selected from the group consisting of: 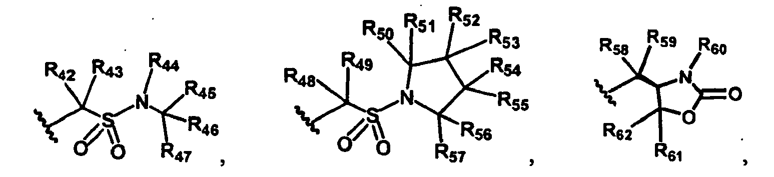 and 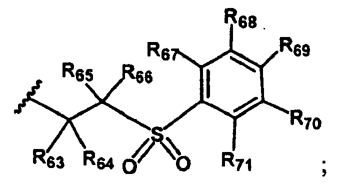
R₁, R₂, R₄, R₆, R₇, R₈, R₉, R₁₀ R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, and R₇₁ are independently selected from the group consisting of hydrogen, and deuterium;
provided that at least one of R₁, R₂, R₄, R₆, R₇, R₈, R₉, R₁₀, R₁₁, R₁₂, R_{13,} R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, P₄₁, R₄₂, N₄₃, R₄₄, R₄₅, R₄₆, R₄₇, R_{48,} N₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, and R₇₁ is deuterium; and
with the proviso that compounds having structural Formula I cannot be:

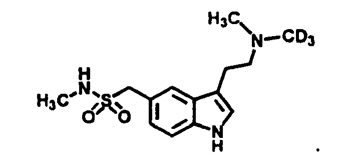

2. The compound as recited in Claim 1, having structural Formula II 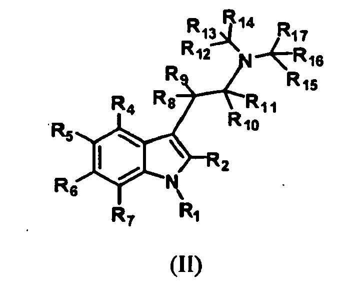 or a pharmaceutically acceptable salt or solvate thereof, wherein:
R₅ is selected from the group consisting of: 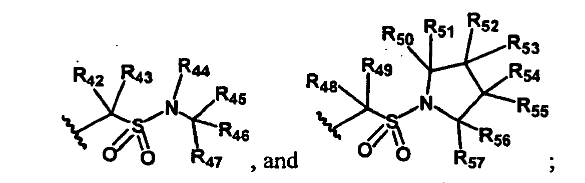
R₁, R₂, R₄, R₆, R₇, R₈, R₉, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, and R₅₇ are independently selected from the group consisting of hydrogen, and deuterium; and
R₁₀, R₁₁, R₁₂, R₁₃, R₁₄, R₁₅, R₁₆, and R₁₇ are each deuterium, R₁₀ and R₁₁ are each hydrogen and R₁₂, R₁₃, R₁₄, R₁₅, R₁₆, and R₁₇ are each deuterium, or R₁₀ and R₁₁ are each deuterium and R₁₂, R₁₃, R₁₄, R₁₅, R₁₆, and R₁₇ are each hydrogen.

3. The compound as recited in Claim 2, wherein R₁₀, R₁₁, R₁₂, R₁₃, R₁₄, R₁₅, R₁₆, and R₁₇ each have deuterium enrichment of at least 50%, R₁₀ and R₁₁ are each hydrogen and R₁₂, R₁₃, R₁₄, R₁₅, R₁₆, and R₁₇ each have deuterium enrichment of at least 50%, or R₁₀ and R₁₁ each have deuterium enrichment of at least 50% and R₁₂, R₁₃, R₁₄, R₁₅, R₁₆, and R₁₇ are each hydrogen.

4. The compound as recited in Claim 1, selected from the group consisting of: 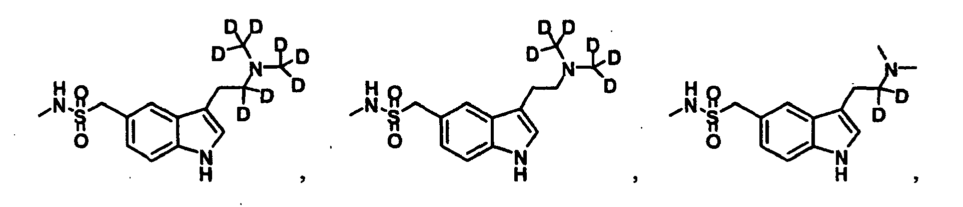 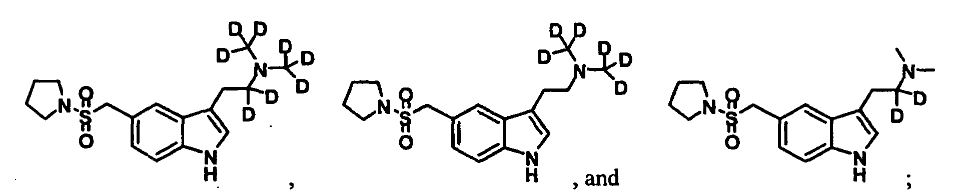 or a pharmaceutically acceptable salt or solvate thereof.

5. The compound as recited in Claim 4, wherein each of said deuteriums have deuterium enrichment of at least 50%.

6. A pharmaceutical composition comprising a pharmaceutically acceptable carrier together with a compound having structural Formula III 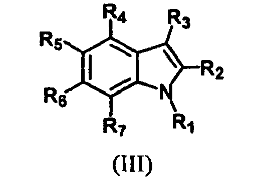 or a pharmaceutically acceptable salt or solvate thereof, wherein:
R₃ is selected from the group consisting of: 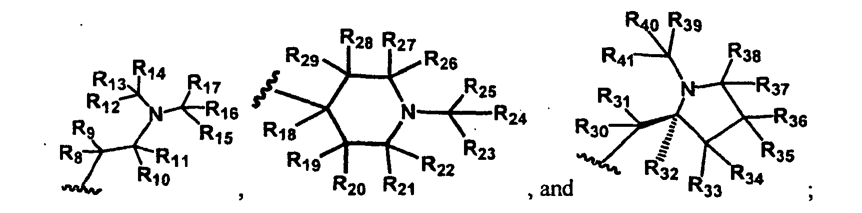
R₅ is selected from the group consisting of: 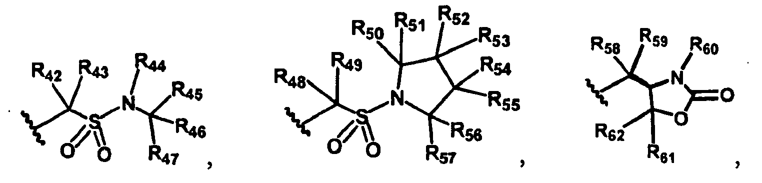 and 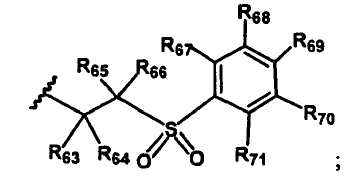
R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₃, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, and R₇₁ are independently selected from the group consisting of hydrogen, and deuterium; and
provided that at least one of R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, P₄₆. R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, and R₇₁ is deuterium amd with proviso that said compounds cannot be:

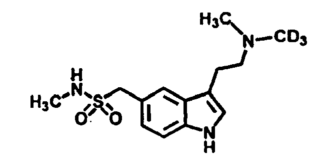

7. The pharmaceutical composition as recited in Claim 6, wherein said compound is selected from the group consisting of: 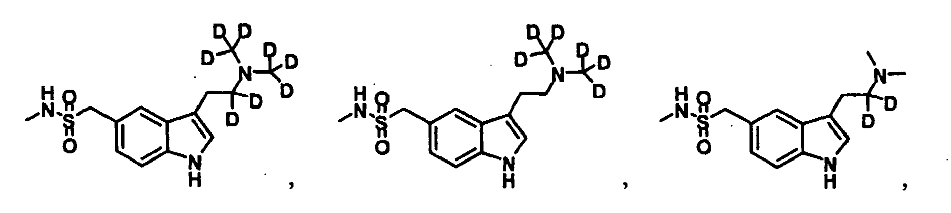 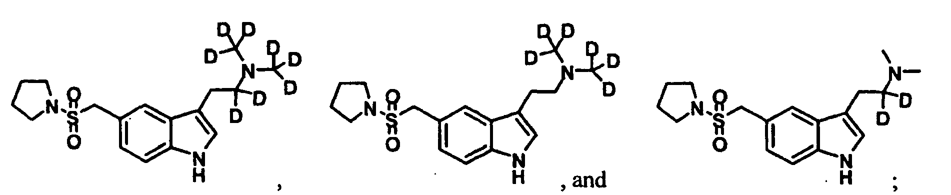 or a pharmaceutically acceptable salt or solvate thereof.

8. A compound having structural Formula III 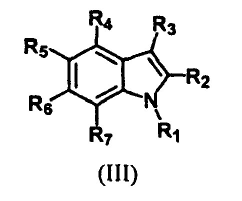 or a pharmaceutically acceptable salt or solvate thereof, wherein:
R₃ is selected from the group consisting of: 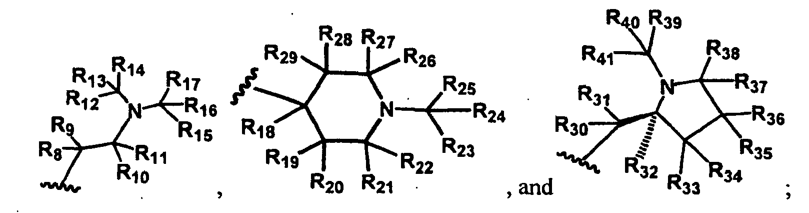
R₅ is selected from the group consisting of: 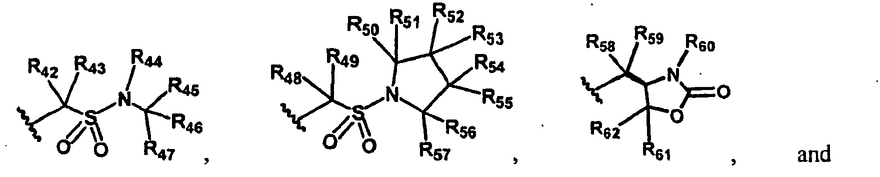 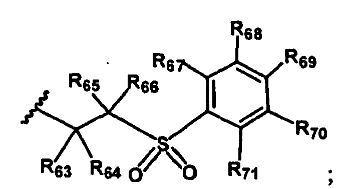
R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, and R₇₁ are independently selected from the group consisting of hydrogen, and deuterium; and
provided that at least one of R₁, R₂, R₄, R₆, R₇, R₈, R₉, R₁₀, R₁₁, R₁₂, R_{13,} R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, and R₇₁ is deuterium; for use in a method of treating a subject suffering from a disorder selected from the group consisting of headaches, movement disorders, depression and anxiety.

9. The compound of Claim 8, wherein said disorder is a migraine headache.

10. The compound of Claim 8, wherein said compound has at least one of the following properties:
a) decreased inter-individual variation in plasma levels of said compound or a metabolite thereof as compared to the non-isotopically enriched compound;
b) increased average plasma levels of said compound per dosage unit thereof as compared to the non-isotopically enriched compound;
c) decreased average plasma levels of at least one metabolite of said compound per dosage unit thereof as compared to the non-isotopically enriched compound; and
d) an improved clinical effect during the treatment of said subject per dosage unit thereof as compared to the non-isotopically enriched compound.

11. The compound of Claim 8, wherein said compound has at least two of the following properties:
a) decreased inter-individual variation in plasma levels of said compound or a metabolite thereof as compared to the non-isotopically enriched compound;
b) increased average plasma levels of said compound per dosage unit thereof as compared to the non-isotopically enriched compound;
c) decreased average plasma levels of at least one metabolite of said compound per dosage unit thereof as compared to the non-isotopically enriched compound; and
d) an improved clinical effect during the treatment of said subject per dosage unit thereof as compared to the non-isotopically enriched compound.

12. The compound of Claim 8, wherein said compound has a decreased metabolism by at least one polymorphically-expressed cytochrome P₄₅₀ isoform in said subject per dosage unit thereof as compared to the non-isotopically enriched compound.

13. The compound of Claim 12, wherein said cytochrome P₄₅₀ isoform is selected from the group consisting of CYP2C8, CYP2C9, CYP2C19, and CYP2D6.

14. The compound of Claim 8, wherein said compound is **characterized by** decreased inhibition of at least one cytochrome P₄₅₀ or monoamine oxidase isoform in said subject per dosage unit thereof as compared to the non-isotopically enriched compound.

15. The compound of Claim 14, wherein said cytochrome P₄₅₀ or monoamine oxidase isoform is selected from the group consisting of CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, CYP51, MAO_{A}, and MAO_{B}.

16. The compound as recited in Claim 9, wherein said compound has structural Formula II 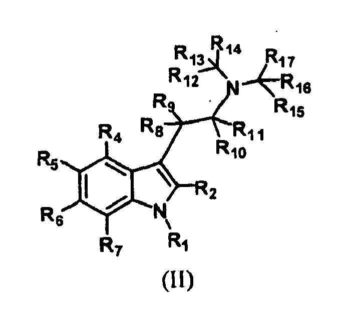 or a pharmaceutically acceptable salt or solvate thereof, wherein:
R₅ is selected from the group consisting of: 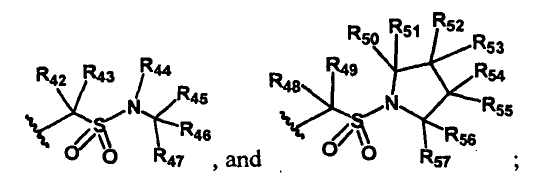
R₁, R₂, R₄, R₆, R₇, R₈, R₉, R₄₂, R₄₃, R₄₄, R₄₅, R₄₆, P₄₇, R₄₈, R₄₉, R₅₀, R₅₁, R₅₂, R₅₃, R₅₄, R₅₅, R₅₆, and R₅₇ are independently selected from the group consisting of hydrogen, and deuterium; and
R₁₀, R₁₁, R₁₂, R₁₃, R₁₄, R₁₅, R₁₆, and R₁₇ are each deuterium, R₁₀ and R₁₁ are each hydrogen and R₁₂, R₁₃, R₁₄, R₁₅, R₁₆, and R₁₇ are each deuterium, or R₁₀ and R₁₁ are each deuterium and R₁₂, R_{13,} R₁₄, R₁₅, R₁₆, and R₁₇ are each hydrogen.

17. The compound as recited in Claim 9, wherein said compound is selected from the group consisting of: 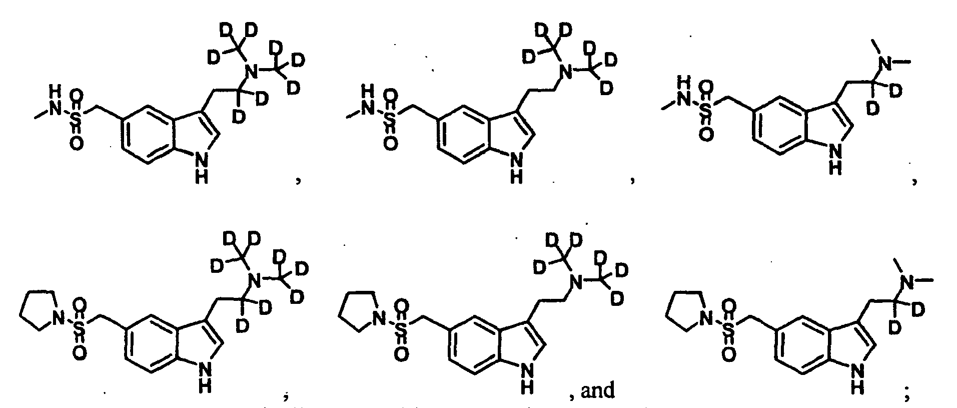 or a pharmaceutically acceptable salt or solvate thereof.

## Patentansprüche

1. Verbindung mit der Strukturformel I 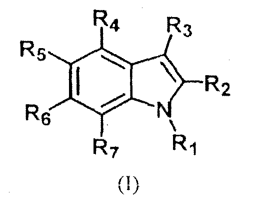 oder ihr pharmazeutisch zulässiges Salz oder Solvat, worin
R₃ ausgewählt ist aus der Gruppe bestehend aus 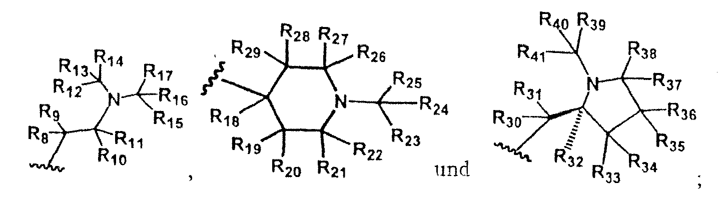 R₅ ausgewählt ist aus der Gruppe bestehend aus 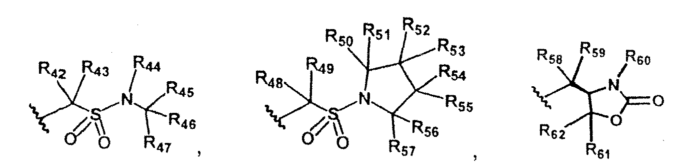 und 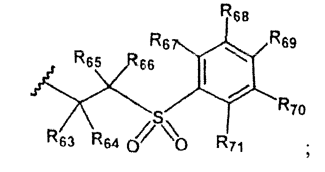 R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₃, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ et R₇₁ unabhägig aus der aus Wasserstoff und Deuterium bestehenden Gruppe ausgewählt sind,
vorausgesetzt, daß wenigstens einer der R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ und R₇₁ Deuterium ist und
vorausgesetzt, daß Verbindungen mit Strukturformel I nicht 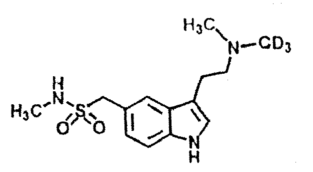 sein können.

2. Verbindung wie in Anspruch 1 angegeben mit der Strukturformel II 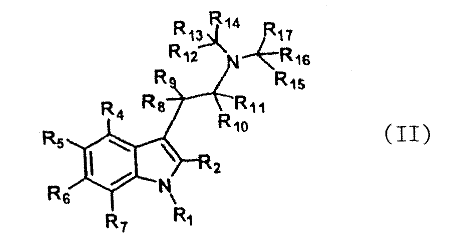 oder ihr pharmazeutisch zulässiges Salz oder Solvat, worin
R₅ ausgewählt ist aus der Gruppe bestehend aus 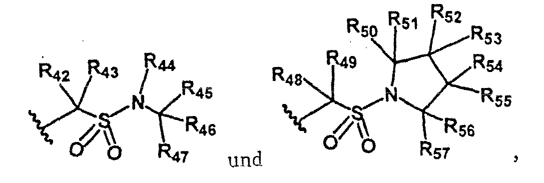 R₁ , R₂ , R₄ , R₆ , R₇ , R₈ , R₉ , R₄₂, R₄₃, R₄₄, R₄₅, R₄₆, R₄₇, R₄₈, R₄₉, R₅₀, R₅₁, R₅₂, R₅₃, R₅₄, R₅₅, R₅₆ und R₅₇ unabhängig aus der aus Wasserstoff und Deuterium bestehenden Gruppe ausgewählt sind und
R₁₀, R₁₁, R₁₂, R₁₃, R₁₄, R₁₅, R₁₆ und R₁₇ jeweils Deuterium sind, R₁₀ und R₁₁ jeweils Wasserstoff sind und R₁₂, R₁₃, R₁₄, R₁₅, R₁₆ und R₁₇ jeweils Deuterium sind oder R₁₀ und R₁₁ jeweils Deuterium sind und R₁₂, R₁₃, R₁₄, R₁₅, R₁₆ und R₁₇ jeweils Wasserstoff sind.

3. Verbindung wie in Anspruch 2 angegeben, bei der R₁₀, R₁₁, R₁₂, R₁₃, R₁₄, R₁₅, R₁₆ und R₁₇ jeweils Deuteriumanreicherung von wenigstens 50% haben, R₁₀ und R₁₁ jeweis Wasserstoff sind und R₁₂, R₁₃, R₁₄, R₁₅, R₁₆ und R₁₇ jeweis Deuteriumanreicherung von wenigstens 50% haben oder R₁₀ und R₁₁ jeweils Deuteriumanreichung von wenigstens 50% haben und R₁₂, R₁₃, R₁₄, R₁₅, R₁₆ und R₁₇ jeweils Wasserstoff sind.

4. Verbindung wie in Anspruch 1 angegeben, die ausgewählt ist aus der Gruppe bestehend aus 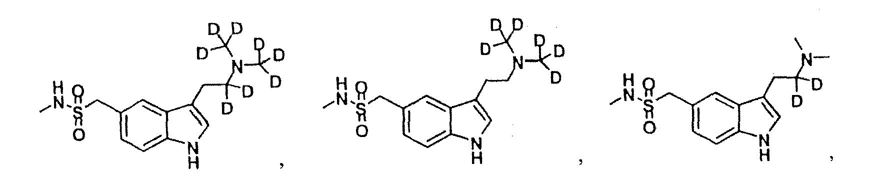 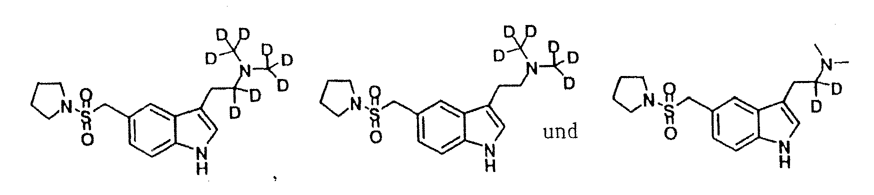 oder ihr pharmazeutisch zulässiges Salz oder Solvat.

5. Verbindung wie in Anspruch 4 angegeben, bei der jedes der genannt Deuterien eine Deuteriumanreicherung von wenigstens 50% haben.

6. Pharmazeutische Zusammensetzung mit einem pharmazeutisch zulässigen Träger zusammen mit einer Verbindung mit der Strukturformel III 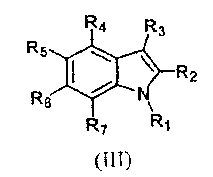 oder ihr pharmazeutisch zulässiges Salz oder Solvat, worin R₃ aus der Gruppe ausgewählt ist bestehend aus 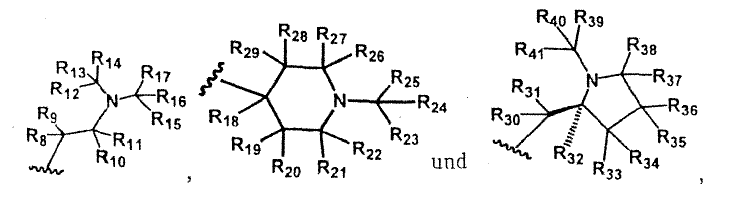 R₅ aus der Gruppe ausgewählt ist bestehend aus 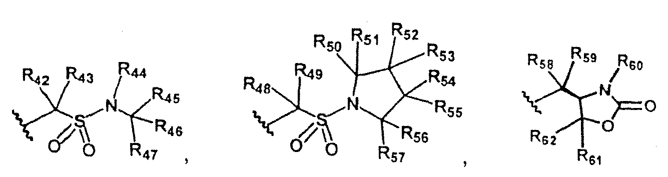 und 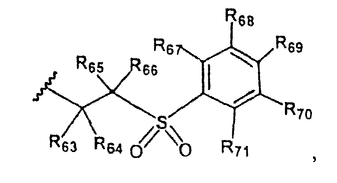 R₁ , R₂ , R₄ , R₅ , R₆ , R₇ , R₈ , R₉ , R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ und R₇₁ unabhängig aus der aus Wasserstoff und Deuterium bestehenden Gruppe ausgewählt sind,
vorausgesetzt, daß wenigstens einer der R₁, R₂, R₄, R₆, R₇, R₈, R₉, R₁₀, R₁₁, R₁₂, R₁₃, R₁₄,R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R_{43,} R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ und R₇₁ Deuterium ist und vorausgesetzt, daß die genannten Verbindungen nicht 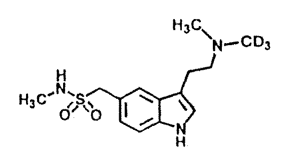 sein können.

7. Pharmazeutische Zusammensetzung wie in Anspruch 6 angegeben, bei der die genannte Verbindung aus der Gruppe ausgewählt ist, bestehend aus 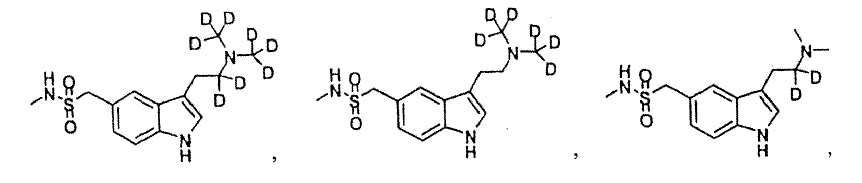 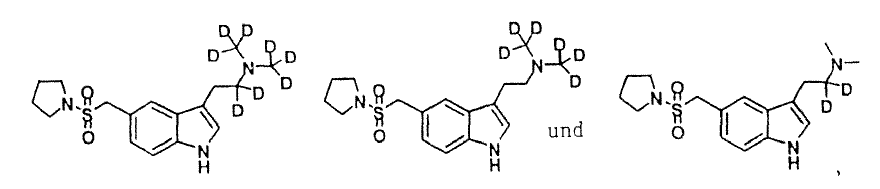 oder ihr pharmazeutisch zulässiges Salz oder Solvat.

8. Verbindung mit der Strukturformel III 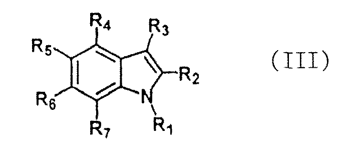 oder ihr pharmazeutisch zulässiges Salz oder Solvat, worin R₃ aus der Gruppe ausgewählt ist bestehend aus 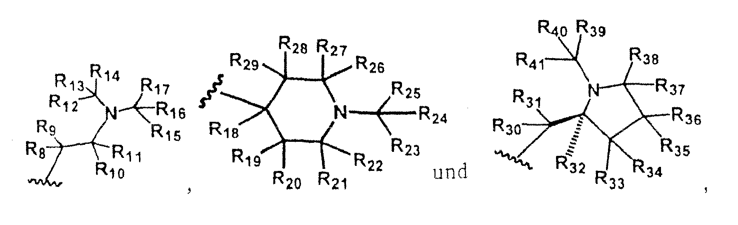 R₅ aus der Gruppe ausgewählt ist bestehend aus 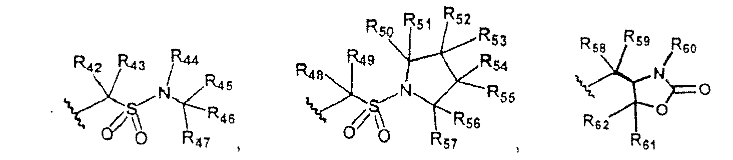 und 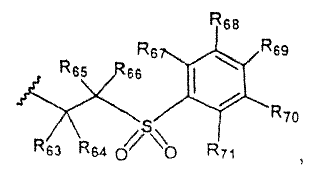 R₁ , R₂ , R₄ , R₅ , R₆ , R₇ , R₈, R₉, R₁₀, R₁₁, R₁₂, R₁₃, R₁₄, R₁₅, R₁₆, R₁₇. R₁₈, R₁₉, R₂₀, R₂₁, R₂₂, R₂₃, R₂₄, R₂₅, R₂₆, R₂₇, R₂₈, R₂₉, R₃₀, R₃₁, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ und R₇₁ unabhängig aus der aus Wasserstoff und Deuterium bestehenden Gruppe ausgewählt sind,
vorausgesetzt, daß wenigstens einer der R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ und R₇₁ Deuterium ist zur Anwendung bei einer Methode der Behandlung eines Patienten, der an einer Erkrankung leidet, die aus der aus Kopfschmerzen, Bewegungstörungen, Depression und Angst bestehenden Gruppe ausgewählt ist.

9. Verbindung des Anspruchs 8, bei der die genannte Krankheit ein Migräne-Kopfschmerz ist.

10. Verbindung des Anspruchs 8, die wenigstens eine der folgenden Eigenschaften hat:
a) herabgesetzte interindividuelle Variation der Plasmawerte der genannten Verbindung oder eines Metaboliten von ihr verglichen mit der nicht-isotopisch angereicherten Verbindung,
b) erhöhte mittlere Plasmawerte der genannten Verbindung je Dosiseinheit verglichen mit der nicht-isotopisch angereicherten Verbindung,
c) herabgesetzte mittlere Plasmawerte wenigstens eines Metaboliten der genannten Verbindung je Dosiseinheit von ihr verglichen mit der nicht-isotopisch angereicherten Verbindung und
d) eine verbesserte klinische Wirkung während der Behandlung des genannten Patienten je Dosiseinheit verglichen mit der nicht-isotopisch angereicherten Verbindung.

11. Verbindung des Anspruchs 8, die wenigstens zwei der folgenden Eigenschaften hat:
a) herabgesetzte interindividuelle Variation der Plasmawerte der genannten Verbindung oder eines Metaboliten von ihr verglichen mit der nicht-isotopisch angereicherten Verbindung,
b) erhöhte mittlere Plasmawerte der genannten Verbindung je Dosiseinheit verglichen mit der nicht-isotopisch angereicherten Verbindung,
c) herabgesetzte mittlere Plasmawerte wenigstens eines Metaboliten der genannten Verbindung je Dosiseinheit von ihr verglichen mit der nicht-isotopisch angereicherten Verbindung und
d) eine verbesserte klinische Wirkung während der Behandlung des genannten Patienten je Dosiseinheit verglichen mit der nicht-isotopisch angereicherten Verbindung.

12. Verbindung des Anspruchs 8, die durch wenigstens eine polymorphisch exprimierte Cytochrom-P₄₅₀-Isoform in dem Patienten je Dosiseinheit verglichen mit der nicht-isotopisch angereicherten Verbindung einen herabgesetzten Stoffwechsel hat.

13. Verbindung des anspruchs 12, bei der die genannte Cytochrom-P -Isoform aus der aus CYP2C8, CYP2C9, CYP2C19 und CYP2D6 bestehenden Gruppe ausgewählt ist.

14. Verbindung des Anspruchs 8, die **gekennzeichnet ist durch** herabgesetzte Hemmung wenigstens einer Cytochrom-P₄₅₀- oder Monoaminoxidase-Isoform in den genannten Patienten je Dosiseinheit davon verglichen mit der nicht-isotopisch angereicherten Verbindung.

15. Verbindung des Anspruchs 14, bei der die genannte Cytochrom-P₄₅₀- oder Monoaminoxidase-Isoform aus der Gruppe ausgewählt ist, die aus CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, CYP51, MAO_{A} und MAO_{B}.

16. Verbindung wie in Anspruch 9 angegeben, mit der Strukturformel II 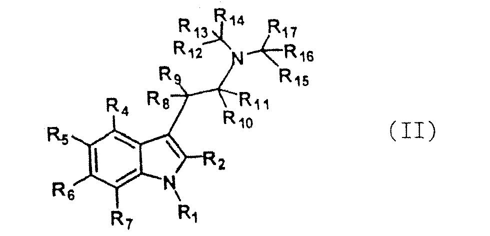 oder ihr pharmazeutisch zulässiges Salz oder Solvat, worin R₅ ausgewählt ist aus der Gruppe bestehend aus 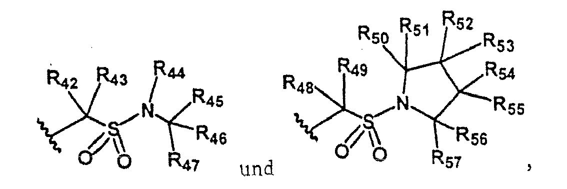 R₁, R₂ R_{4,} R_{6,} R_{7,} R₈, R₉, R₄₂, R₄₃, R₄₄, R_{45,} R_{46,} R_{47,} R_{48,} R_{49,} R_{50,} R₅₁, R_{52,} R_{53,} R_{54,} R_{55,} R₅₆ und R₅₇ unabhängig aus der aus Wasserstoff und Deuterium bestehenden Gruppe ausgewählt sind und
R₁₀, R₁₁, R₁₂, R₁₃, R₁₄, R₁₅, R₁₆ und R₁₇ jeweils Deuterium sind, R₁₀ und R₁₁ jeweils Wasserstoff sind und R₁₂, R₁₃, R₁₄, R₁₅, R₁₆ und R₁₇ jeweils Deuterium sind oder R₁₀ und R₁₁ jeweils Deuterium sind und R₁₂, R₁₃, R₁₄, R₁₅, R₁₆ und R₁₇ jeweils Wasserstoff sind.

17. Verbindung wie in Anspruch 9 angegeben, die ausgewählt ist aus der Gruppe bestehend aus 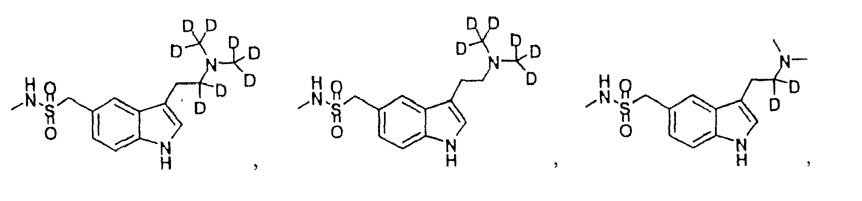 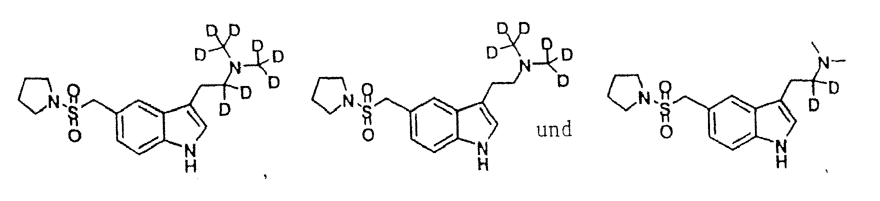 oder ihr pharmazeutisch zulässiges Salz oder Solvat.

## Revendications

1. Composé ayant une formule structurelle I 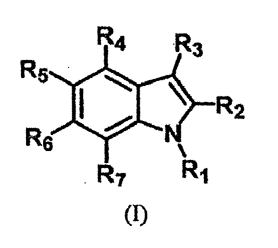 ou son sel ou solvate pharmaceutiquement acceptable, dans lequel :
R₃ est choisi dans le groupe constitué de : 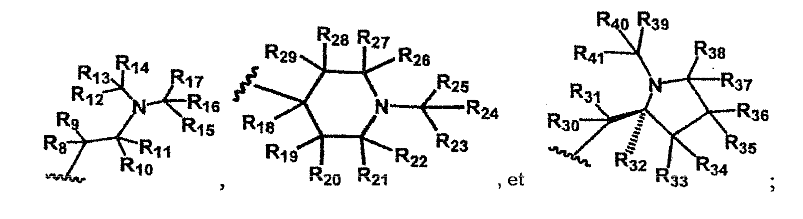
R₅ est choisi dans le groupe constitué de : 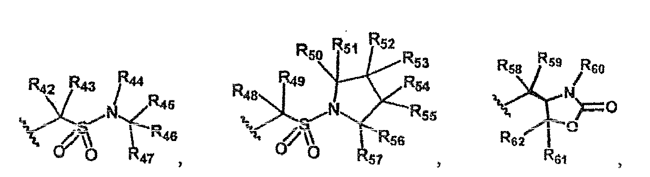 et 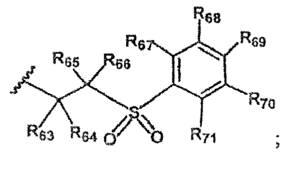
R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₃, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ et R₇₁ sont indépendamment choisis dans le groupe constitué d'hydrogène et de deutérium ;
à condition qu'au moins un parmi R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ et R₇₁ soit un deutérium ; et
à condition que les composés ayant la formule structurelle I ne puissent pas être :

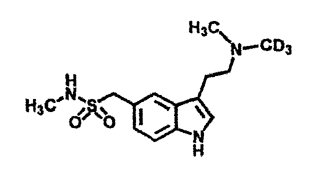

2. Composé selon la revendication 1, ayant une formule structurelle II 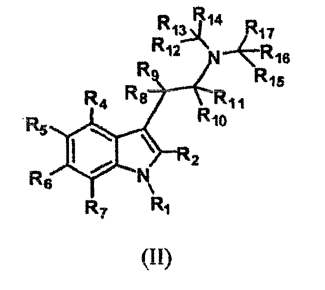 ou son sel ou solvate pharmaceutiquement acceptable, dans lequel :
R₅ est choisi dans le groupe constitué de : 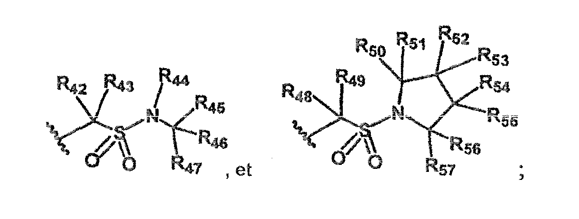
R₁, R₂, R₄, R₆, R₇, R₈, R₉, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, et R₅₇, sont indépendamment choisis dans le groupe constitué d'hydrogène, et de deutérium ; et
R₁₀, R₁₁, R₁₂, R₁₃, R₁₄, R₁₅, R₁₆, et R₁₇, sont chacun un deutérium, R₁₀ et R₁₁ sont chacun un hydrogène, et R₁₂, R₁₃, R₁₄, R₁₅, R₁₆, et R₁₇ sont chacun un deutérium, ou R₁₀ et R₁₁ sont chacun un deutérium et R₁₂, R₁₃, R₁₄, R₁₅, R₁₆, et R₁₇ sont chacun un hydrogène.

3. Composé selon la revendication 2, dans lequel R₁₀, R₁₁, R₁₂, R₁₃, R₁₄, R₁₅, R₁₆, et R₁₇, ont chacun un enrichissement en deutérium d'au moins 50%, R₁₀ et R₁₁ sont chacun un hydrogène et R₁₂, R₁₃, R₁₄, R₁₅, R₁₆, et R₁₇ ont chacun un enrichissement en deutérium d'au moins 50 %, ou R₁₀ et R₁₁ ont chacun un enrichissement en deutérium d'au moins 50 % et R₁₂, R₁₃, R₁₄, R₁₅, R₁₆, et R₁₇ sont chacun un hydrogène.

4. Composé selon la revendication 1, choisi dans le groupe constitué de : 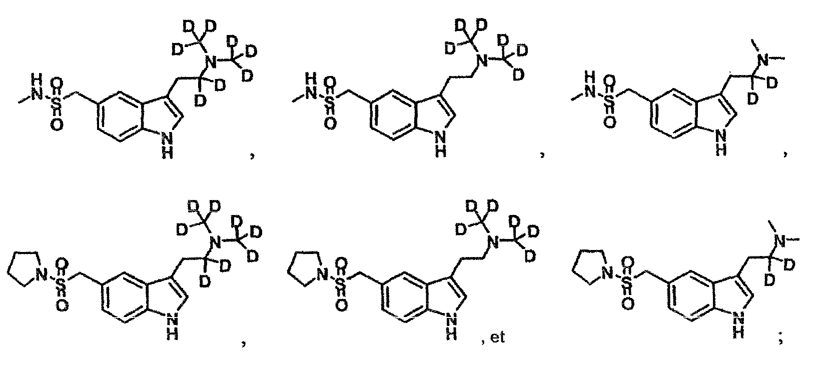 ou son sel ou solvate pharmaceutiquement acceptable.

5. Composé selon la revendication 4, dans lequel chacun desdits deutériums a un enrichissement en deutérium d'au moins 50 %.

6. Composition pharmaceutique comprenant un excipient pharmaceutiquement acceptable avec un composé ayant la formule structurelle III 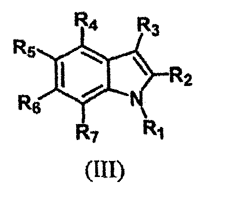 ou son sel ou solvate pharmaceutiquement acceptable, dans laquelle :
R₃ est choisi dans le groupe constitué de : 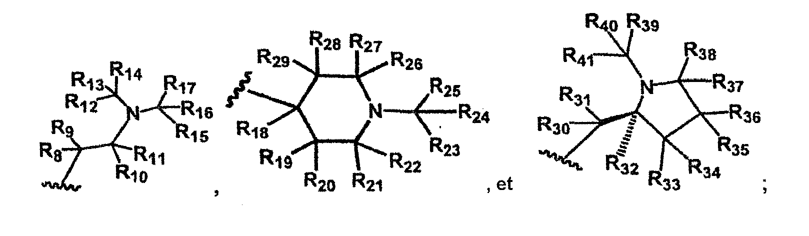
R₅ est choisi dans le groupe constitué de : 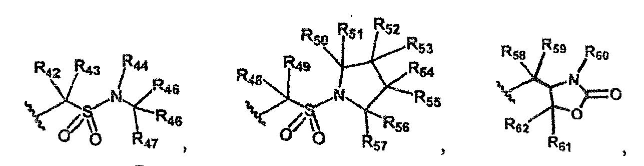 et 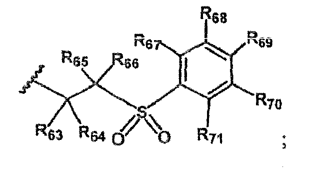
R₁, R₂, R_{4,} R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, P₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ et R₇₁ sont indépendamment choisis dans le groupe constitué d'hydrogène et de deutérium ;
à condition qu'au moins un parmi R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ et R₇₁ soit un deutérium et à condition que les composés ne puissent pas être :

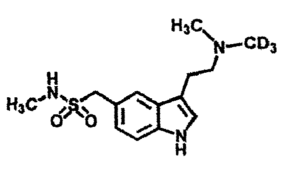

7. Composition pharmaceutique selon la revendication 6, dans laquelle ledit composé est choisi dans le groupe constitué de : 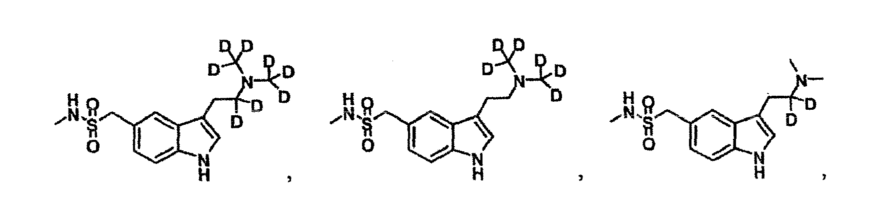 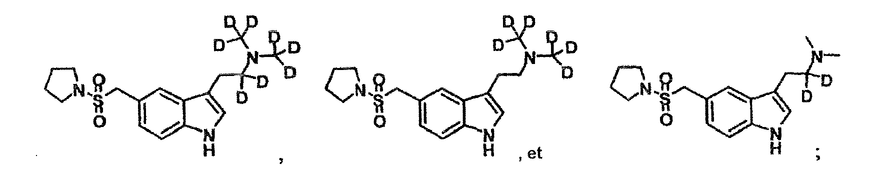 ou son sel ou solvate pharmaceutiquement acceptable.

8. Composé ayant une formule structurelle III 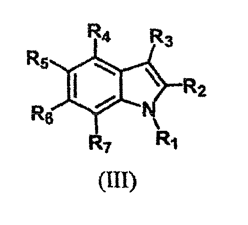 ou son sel ou solvate pharmaceutiquement acceptable, dans lequel :
R₃ est choisi dans le groupe constitué de : 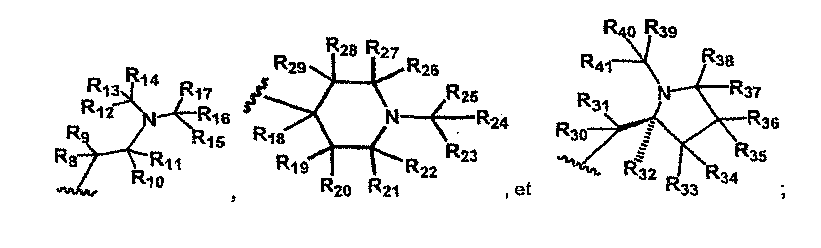
R₅ est choisi dans le groupe constitué de : 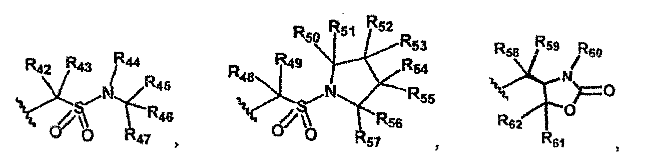 et 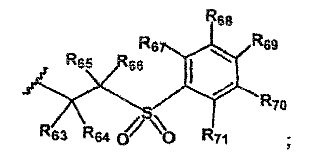
R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ et R₇₁ sont indépendamment choisis dans le groupe constitué d'hydrogène et de deutérium ; et
à condition qu'au moins un parmi R₁, R₂, R₄, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R₂₉, R₃₀, R₃₁, R₃₂, R₃₃, R_{34,} R₃₅, R₃₆, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀ et R₇₁ soit un deutérium ; à utiliser dans un procédé de traitement d'un sujet soufflant d'un trouble choisi dans le groupe constitué de maux de tête, de troubles des mouvements, de dépression et d'anxiété.

9. Composé selon la revendication 8, dans lequel ledit trouble est la migraine.

10. Composé selon la revendication 8, dans lequel ledit composé a au moins une des propriétés suivantes :
a) variation réduite inter-individus des niveaux de plasma dudit composé ou de son métabolite par rapport au composé enrichi de manière non-isotopique ;
b) augmentation des niveaux de plasma moyens dudit composé par unité de dosage correspondante par rapport au composé enrichi de manière non-isotopique ;
c) diminution des niveaux de plasma moyens d'au moins un métabolite dudit composé par unité de dosage correspondante par rapport au composé enrichi de manière non-isotopique ; et
d) un effet clinique amélioré pendant le traitement dudit sujet par unité de dosage correspondante par rapport au composé enrichi de manière non-isotopique.

11. Composé selon la revendication 8, dans lequel ledit composé a au moins deux des propriétés suivantes :
a) variation réduite inter-individus des niveaux de plasma dudit composé ou de son métabolite par rapport au composé enrichi de manière non-isotopique ;
b) augmentation des niveaux de plasma moyens dudit composé par unité de dosage correspondante par rapport au composé enrichi de manière non-isotopique ;
c) diminution des niveaux de plasma moyens d'au moins un métabolite dudit composé par unité de dosage correspondante par rapport au composé enrichi de manière non-isotopique ; et
d) un effet clinique amélioré pendant le traitement dudit sujet par unité de dosage correspondante par rapport au composé enrichi de manière non-isotopique.

12. Composé selon la revendication 8, dans lequel ledit composé a un métabolisme réduit par au moins une isoforme P₄₅₀ de cytochrome exprimée de manière polymorphique dans ledit sujet par unité de dosage correspondante par rapport au composé enrichi de manière non-isotopique.

13. Composé selon la revendication 12, dans lequel ladite isoforme P₄₅₀ de cytochrome est choisie dans le groupe constitué de CYP2C8, CYP2C9, CYP2C19 et CYP2D6.

14. Composé selon la revendication 8, dans lequel ledit composé est **caractérisé par** une inhibition réduite d'au moins une isoforme P₄₅₀ de cytochrome ou d'oxydase de monoamine dans ledit sujet par unité de dosage correspondante par rapport au composé enrichi de manière non-isotopique.

15. Composé selon la revendication 14, dans lequel ladite isoforme P₄₅₀ de cytochrome ou de monoamine oxydase est choisie dans le groupe constitué de CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, CYP51, MAO_{A} et MAO_{B}.

16. Composé selon la revendication 9, dans lequel ledit composé a une formule structurelle II 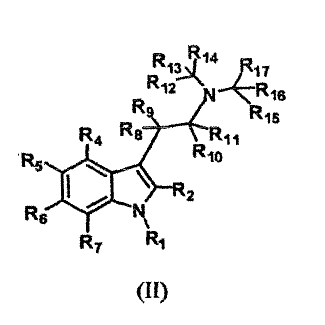 ou son sel ou solvate pharmaceutiquement acceptable, dans lequel :
R₅ est choisi dans le groupe constitué de : 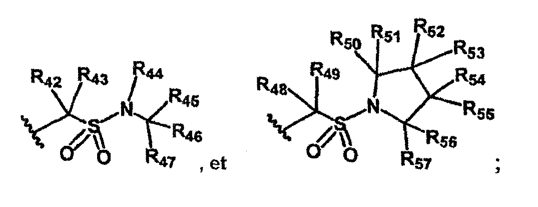
R₁, R₂, R₄, R₆, R₇, R₈, R₉, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, et R₅₇, sont indépendamment choisis dans le groupe constitué d'hydrogène, et de deutérium ; et
R₁₀, R₁₁, R₁₂, R₁₃, R₁₄, R₁₅, R₁₆, et R₁₇, sont chacun un deutérium, R₁₀ et R₁₁ sont chacun un hydrogène, et R₁₂, R₁₃, R₁₄, R₁₅, R₁₆, et R₁₇ sont chacun un deutérium, ou R₁₀ et R₁₁ sont chacun un deutérium et R₁₂, R₁₃, R₁₄, R₁₅, R₁₆, et R₁₇ sont chacun un hydrogène.

17. Composé selon la revendication 9, dans lequel ledit composé est choisi dans le groupe constitué de : 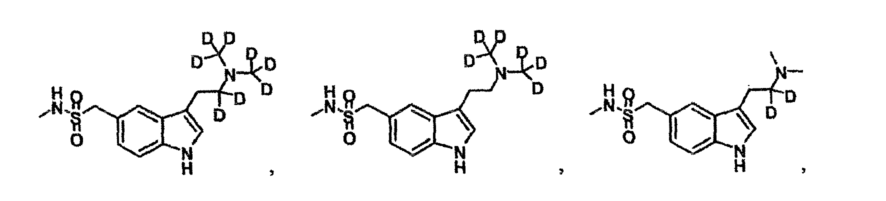 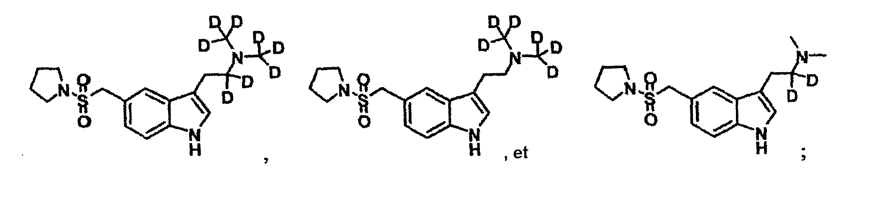 ou son sel ou solvate pharmaceutiquement acceptable.
